# EUROPEAN PATENT SPECIFICATION

(11) **EP 2 184 607 B1**
(45) Date of publication and mention of the grant of the patent: **11.07.2012**
(21) Application number: 09179139.2
(22) Date of filing: 24.05.2005
(51) Int. Cl.: G01N 33/53, G01N 33/566, G01N 33/564, G01N 33/531, G01N 33/00, A61K 39/00, A61K 38/00, G01N 33/68, G01N 33/50

(54) **Immune response assessment method**
Verfahren zur Beurteilung von Immunreaktionen
Procédé d'évaluation de réponse immunitaire

(30) Priority: 24.05.2004 US 573912 P
(43) Date of publication of application: 12.05.2010
(62) Divisional of application: 05804838.0
(73) Proprietor: Baylor Research Institute, Dallas, TX 75204 (US)
(72) Inventor: Banchereau, Jacques, Montclair, NJ 07042 (US); Connolly, John E., Dallas, TX 75226 (US); Palucka, Anna Karolina, Dallas, TX 75204 (US); Ueno, Hideki, Plano, TX 75093 (US)
(74) Representative: Sonn & Partner Patentanwälte

(56) References cited:
- WO-A-99/36568
- US-A1- 2002 164 346
- KAKINOKI Y ET AL: "Allergen induced mRNA expression of interleukin-5, but not of interleukin-4 and interferon-gamma, in peripheral blood mononuclear cells obtained before the pollen season predicts the clinical efficacy of immunotherapy for seasonal allergic rhinitis" SCANDINAVIAN JOURNAL OF IMMUNOLOGY, vol. 51, no. 2, February 2000 (2000-02), pages 202-208, XP002572388 ISSN: 0300-9475
- KARLSSON A C ET AL: "Comparison of the ELISPOT and cytokine flow cytometry assays for the enumeration of antigen-specific T cells" JOURNAL OF IMMUNOLOGICAL METHODS, ELSEVIER SCIENCE PUBLISHERS B.V.,AMSTERDAM, NL, vol. 283, no. 1-2, 1 December 2003 (2003-12-01), pages 141-153, XP004476985 ISSN: 0022-1759
- HASELDEN BRIGITTE M ET AL: "Proliferation and release of IL-5 and IFN-gamma by peripheral blood mononuclear cells from cat-allergic asthmatics and rhinitics, non-cat-allergic asthmatics, and normal controls to peptides derived from Fel d 1 chain 1" JOURNAL OF ALLERGY AND CLINICAL IMMUNOLOGY, vol. 108, no. 3, September 2001 (2001-09), pages 349-356, XP002479547 ISSN: 0091-6749
- HOLEN E ET AL: "Novel B and T cell epitopes of chicken ovomucoid (Gal d 1) induce T cell secretion of IL-6, IL-13, and IFN-gamma" CLINICAL AND EXPERIMENTAL ALLERGY, vol. 31, no. 6, June 2001 (2001-06), pages 952-964, XP002479548 ISSN: 0954-7894
- LI ET AL: "Persistent protective effect of heat-killed Escherichia coli producing ''engineered,'' recombinant peanut proteins in a murine model of peanut allergy" JOURNAL OF ALLERGY AND CLINICAL IMMUNOLOGY, MOSBY, INC, US, vol. 112, no. 1, 1 July 2003 (2003-07-01), pages 159-167, XP005687264 ISSN: 0091-6749
- EARLEY MARIE C ET AL: "Report from a workshop on multianalyte microsphere assays." CYTOMETRY, vol. 50, no. 5, 15 October 2002 (2002-10-15), pages 239-242, XP002479549 ISSN: 0196-4763
- OKANO ET AL.: J. ALLERGY CLIN IMMUNOL, vol. 108, no. 1, July 2001 (2001-07), pages 101-108,

## Description

### TECHNICAL FIELD OF INVENTION

This application relates to methods for predicting an immune response in a subject to an immune therapy.

### BACKGROUND OF THE INVENTION

The immune system provides protection against microbial insult. It is by its very essence a complex system as it must be able to recognize and adapt to ever-changing threats while limiting collateral damage to self tissue. It is a consequence of this complexity that immune dysfunction, both hyper- and hypo-activity, are at the root of many human diseases. The adaptive immune system (composed of B cells and T cells) recognizes antigens in the form of molecular patterns, such as peptides, glycopeptides, phosphopeptides and lipids, bound to and presented in the context of specialized classes of antigen-presenting molecules. The antigen-derived fragments, for example, peptides that are recognized by the T-cells, are referred to as epitopes. It is widely accepted that the recognition by a T-cell of its specific epitope is the central event in adaptive immune function.

These specialized antigen-presenting molecules include: i) the classic human leukocyte antigen (HLA) proteins which are highly polymorphic and can be separated into two major classes, Class I and Class II; ii) the non-classic group that is encoded by the HLA E, F, and G subloci (Braud VM, Curr Opin Immunol. 1999 Feb;11(1):100-8), and iii) the CD1 family of antigen-presenting molecules (Bendelac A, Science. 1995 Jul 14;269(5221):185-6). HLA Class I molecules are encoded by HLA A, B and C loci and present 8-10 amino acid (AA) peptides to CD8+ cytotoxic T cells (Adams EJ, Immunol Rev. 2001 Oct;183:41-64). HLA Class II molecules present 12-16 AA peptides to CD4+ helper T cells and are encoded by the HLA DR, DP and DQ loci (Korman AJ, Immunol Rev. 1985 Jul;85:45-86). T-cells recognize antigens only in the context of antigen-presenting molecules, and only after appropriate antigen processing and presentation. For HLA dependent antigen presentation, the antigen processing usually consists of proteolytic fragmentation of the protein, resulting in polypeptides that fit into the groove of the HLA molecule and this peptide/HLA complex is subsequently presented on the cell surface of the antigen-presenting cell to the T-cell (Rock, K, Adv Immunol. 2002;80:1-70, Cresswell P, Curr Biol. 1994 Jun 1;4(6):541-3). The non-classic group is encoded by the HLA E, F, and G subloci and is characterized by a low degree of polymorphism and an ability to bind and present a restricted set of peptides to immune effector cells. Recognition of peptides presented by non-classic HLA molecules has been demonstrated to be important in inducing immune tolerance in areas such as the maternal fetal interface and/or tumor immunity. The other important group among these specialized molecules is the CD1 family, which is involved in the presentation of lipids such as bacterial glycolipids to specialized effector cells in the immune system.

Three major types of T cell immunity may follow antigen recognition: Type-1 (Th1), Type-2 (Th2), and Regulatory (T-Regulatory). Type-1 immunity is important for the clearance of intracellular pathogens and anti-tumor immunity, and is primarily characterized by the expression of IFN gamma (IFN-γ). Type-2 immunity is critical for the clearance of extracellular pathogens and is primarily characterized by the expression of cytokines such as IL-4, IL-5, IL-9 and IL-13. T-Regulatory type of immunity, mediated by cytokines such as IL-10 or TGF-beta, is essential for the generation and maintenance of self-tolerance. These three types of response are not restricted to the classical alpha/beta TCR+ CD8+ T cells and can also be mediated by gamma/delta TCR+ CD8+ T cells as well as natural killer (NK) cells and NK T cells. Thus, recognition of specific antigenic epitopes by any of these cell types leads to their activation and distinctive, specialized response. The quality of an immune response may therefore be described by observing the coordinated expression of cytokine patterns (Type-1, Type-2, and T-Regulatory).

Activation of appropriate T-cell response during the course of an immune reaction often involves dominance of one specialized response over the others. This selective activation of an appropriate specialized T-cell response is a critical determinant for disease outcome. A striking example of this is demonstrated in patient response in leprosy, wherein a Type-1 response to infection is characterized by protective immunity and a Type-2 response often leads to fatal disease. In another dramatic example regarding tumor immunity, tolerogenic epitopes leading to an inappropriate shift toward a T-Regulatory response have been shown to result in subsequent tumor progression in humans, while activation of Type-1 responses by tumor antigen epitopes are associated with the inhibition of tumor growth. Furthermore, an inappropriate shift away from a T-Regulatory response may lead to the development of autoimmune disease if self antigen is presented. The specific clinical picture of autoimmune disease would depend on the dominant response: either Type-1, as in the case of Type-1 diabetes, or Type-2, as in the case of allergy.

As the activation of these specialized T-cell responses is dependent on presentation of specific epitopes to them, it would be advantageous therefore to identify these peptide epitopes. By identifying these specialized T-cell epitopes early on in the disease state, the opportunity arises to enhance appropriate T-cell responses or eliminate inappropriate T-cell responses, and thereby shift the balance of the immune response to improve disease outcome. A methodology for quantitative identification and qualitative characterization of these antigenic T-cell epitopes is an important basis for vaccine design as well as diagnostic and therapeutic approaches to infectious, autoimmune and neoplastic diseases.

Kakinoki et al. (Journal of Immunology 51 (2), 2000: 202-208) relates to immunotherapy for seasonal llergic rhinitis comprising culturing PBMCs from a patient with pollen allergens and analyzing the cytokine profile.

### SUMMARY OF THE INVENTION

The present invention provides a method for predicting an immune response in a subject to an immune therapy with an agent comprising an antigenic protein of interest comprising the steps of:
culturing isolated immune cells from the subject with a cluster of several overlapping peptides from an overlapping peptide library of the antigenic protein of interest;
analyzing simultaneously a supernatant of the subject's culture medium for multiple parameters of immune reactivity comprising the specificity and cytokine response of cytokines and chemokines, which allow identification of Type-1, Type-2 and T-Regulatory cell phenotypes to the cluster of several overlapping peptides;
determining the type of immune response based on the coordinated cytokine expression in response to the individual peptides in the culture medium as an indicator of treatment outcome.

The therapy can be a immunotherapy such as vaccination, passive immunotherapy and adoptive cell transfer.

This method is based on also disclosed methods for identifying one or more immunomodulatory peptides from a subject by culturing the immune cells of the subject in the presence of one or more peptides from an overlapping peptide library taken from an antigenic protein of interest and analyzing simultaneously the culture for multiple parameters of immune reactivity such as specificity and cytokine response to epitopes on the peptides, wherein an immunomodulatory peptide is identified by the presence of immune reactivity toward the peptide that is independent of the HLA type of the subject. For example, the reactive epitopes may be selected from the immunomodulatory peptides in a pool of peptides. The method also includes the isolation of peptide specific effector cells from that specific individual.

Disclosed is a method for identifying one or more immunomodulatory fragments by: isolating immune cells from the subject; culturing the immune cells in the presence of one or more fragments from the processed peptide library; and simultaneously analyzing the culture for multiple parameters of immune specificity and cytokine response to the fragments, wherein an immunomodulatory fragment is identified by the presence of immune reactivity toward the fragment. Using this method, multiple peptide fragment-specific effector cells may be isolated and even purified, e.g., into a mix or clonal population. Also disclosed is a method for identifying the type of immune reactivity expressed by a subject towards an antigenic material by isolating and culturing one or more immune cells from a subject in the presence of one or more antigenic fragments; identifying the cytokines produced by the immune cells; and determining the type of immune reactivity based on the coordinated cytokine expression in response to the antigenic material. Antigenic material may include an antigen, a peptide, a microbe, a cell, and mixtures of combinations thereof. The antigenic protein may include peptides with known or unknown amino acid sequences, from known or unknown proteins and/or protein fragments, or even an overlapping peptide library taken from a known protein or peptides fractionated by size prior to use in the method and subsequent characterization. In some embodiment, the antigenic material may includes multiple antigenic materials and/or peptides that are provided in different ratios to drive the type of immune response, e.g., Th1 v. Th2 or Tc1 v. Tc2 or mixtures and combinations thereof.

Disclosed is a method for predicting a subject's response to therapy in which immune cells are cultured from the subject in the presence of an overlapping peptide library taken from an antigenic protein of interest, the culture is analyzed for multiple parameters of immune reactivity to identify at least one immunomodulatory molecule for the subject's T-cell and HLA repertoire and the culture is analyzed for cytokine production to determine the type of immune reactivity to the immunomodulatory molecule based on the identification of the cytokines produced to determine the epitope-specific immune status of the subject prior to treatment as an indicator of treatment outcome. The therapy may be an immunotherapy such as vaccination, passive immunotherapy and adoptive cell transfer. Another disclosed example is a method for predicting a subject's disease course and clinical outcome by culturing one or more immune cells from the subject in the presence of a series of peptides from an overlapping peptide library taken from an antigenic protein of interest and analyzing the culture for cell proliferation and cytokine production profile to determine the type of immune reactivity to the immunomodulatory molecule, wherein the cytokine production profile is a marker of disease severity and status of disease progression or regression.

In operation, a disclosed method for treatment of a subject in need of immunotherapy includes: exposing immune cells from the subject to one or more immunomodulatory peptides to determine the type of immune response triggered by the one or more immunomodulatory peptides; treating the subject with immunomodulatory peptides that affect the type of immune response; and evaluating the type of immune response after treating the subject with one or more immunomodulatory peptides and if necessary modifying the one or more immunomodulatory peptides to change the type of immune response. The assessment of therapeutic efficacy may be used to monitor vaccine therapy, anti-cancer therapy, anti-tumor therapy, organ transplantation, allergy, therapy of autoimmune disease, and therapy of infectious disease. Another method of the present invention includes identifying and characterizing new therapeutic agents for immunotherapy by culturing isolated immune cells in the presence and absence of an immune modulator and one or more peptides from an overlapping peptide library taken from an antigenic protein of interest; simultaneously analyzing the cultures for multiple parameters of immune reactivity such as specificity and cytokine response to epitopes on the peptides; and comparing the immune reactivity when the immune modulator is present to the immune reactivity when the immune modulator is absent, wherein the inhibition of the immune reactivity is indicative of an immunosuppressive therapeutic agent and wherein the enhancement of the immune reactivity is indicative of an adjuvant and may even drive the type of immune response to a Th1, a Th2, a Tc1, a Tc2, and combinations thereof

### BRIEF DESCRIPTION OF THE DRAWINGS

For a more complete understanding of the features and advantages of the present invention, reference is now made to the detailed description of the invention along with the accompanying figures and in which:

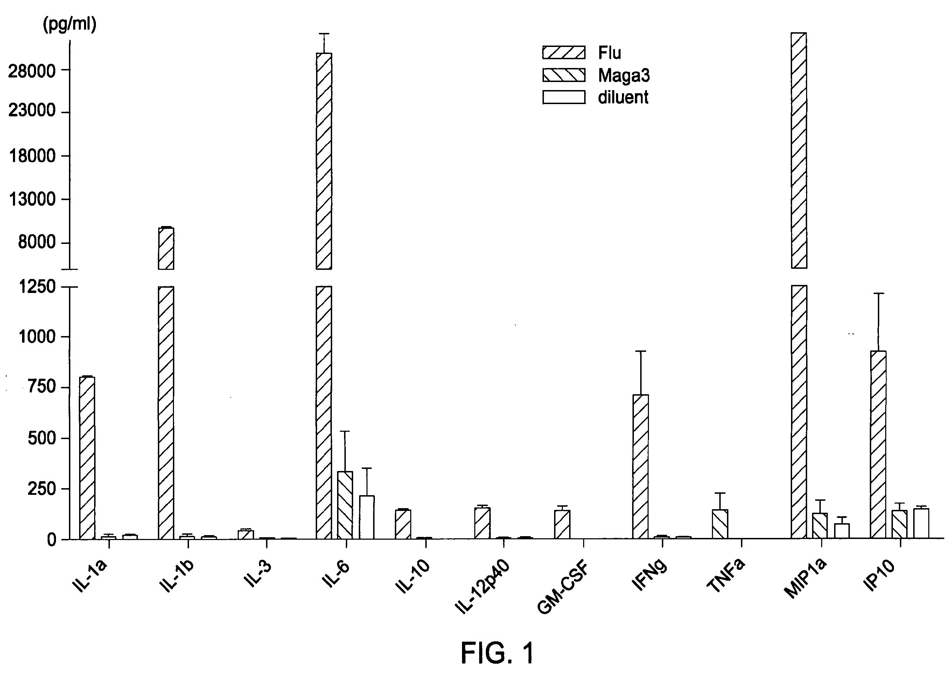

Figure 1 is a graph that depicts the immune response of a normal donor evaluated according the present invention, wherein peripheral blood mononuclear cells (PBMC) were isolated from freshly drawn blood from an HLA-A0201⁺ healthy volunteer, seeded at 2 x 10⁵ cells/well in triplicates in round-bottom 96-well plates, and incubated with 1 µl of either of HLA-A0201 restricted peptides Flu-MP₅₈₋₆₆ (GILGFVFTL) or MAGE-3₂₇₁₋₂₇₉ (FLWGPRALV), or peptide diluent (5% DMSO in H₂O); and culture supernatants were harvested 48 h later and evaluated quantitatively for the presence of cytokines and chemokines using Luminex technology;

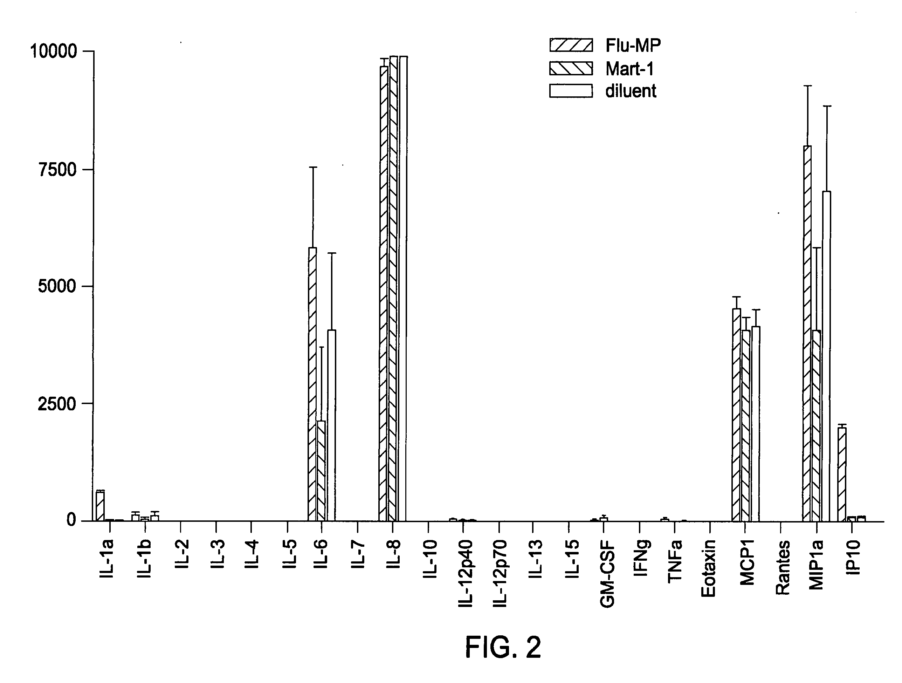

Figure 2 is a graph that depicts another example of immune response to HLA-A0201 restricted peptides Flu-MP₅₈₋₆₆ (GILGFVFTL) in a normal donor according the present invention. PBMCs were isolated from freshly drawn blood from another HLA-A0201⁺ healthy volunteer, seeded at 5 x 10⁵ cells/well in triplicates in round-bottom 96-well plates, and incubated with 10 µg/ml of either of HLA-A0201 restricted peptides Flu-MP₅₈₋₆₆ (GILGFVFTL) or MART-1₂₇₋₃₅ (AAGIGILTV), or peptide diluent (5% DMSO in H₂O); and culture supernatants were harvested 48 h later and evaluated quantitatively for the presence of cytokines and chemokines using Luminex technology;
Figure 3A-3D are graphs that depict the immune response of 2 melanoma patients as evaluated according the present invention;
Figure 4A-4C are graphs that depict the immune response of a melanoma patient as evaluated according the present invention;
Figure 5 is a graph that depicts the kinetics of the immune response of a normal donors evaluated according the present invention;
Figure 6 depicts the scheme of epitope focusing according to the present invention in which overlapping peptide libraries were split into clusters of peptides (5-10 peptides/cluster) a cluster analysis conducted, followed by epitope focusing;
Figure 7 is a graph that depicts the immune response of a melanoma patient as evaluated according the present invention;
Figure 8 is a graph that depicts the immune response of a melanoma patient as evaluated according the present invention;
Figure 9 is a graph that depicts the immune response of melanoma patients as evaluated according the present invention;
Figure 10 is a graph that depicts the immune response of a melanoma patient as evaluated according the present invention;
Figure 11 is a graph that depicts the immune response of a melanoma patient as evaluated according the present invention;
Figures 12A and 12B are graphs that depict the immune response of a melanoma patient as evaluated according the present invention in which PBMCs obtained before DC vaccination and post 8 DC vaccination from a melanoma patient (same as Figure 10) were stimulated with single 15-mer MART-1 peptide or diluent;
Figure 13 is a graph that depicts the immune response of a melanoma patient as evaluated according the present invention;
Figure 14 is a graph that depicts the immune response of a melanoma patient as evaluated according the present invention;
Figure 15 is a graph that depicts the immune response of a melanoma patient as evaluated according the present invention;
Figures 16A-D are graphs that depicts the immune response of a melanoma patient as evaluated according the present invention;
Figure 17 is a graph that depicts the immune response of a melanoma patient as evaluated according the present invention;
Figure 18 is a graph that depicts the immune response of melanoma patients as evaluated according the present invention;
Figure 19 is a graph that depicts the immune response of a melanoma patient as evaluated according the present invention;
Figure 20 is a graph that depicts IL-2 production from CD4+ T cells upon stimulation with the identified 15-mer peptide as shown in Figure 19;
Figures 21A-B are graphs that depict the immune response of a melanoma patient as evaluated according the present invention with PBMCs obtained from the same melanoma patient shown in Figure 19;
Figures 22 A-C are graphs that depict the immune response of a melanoma patient as evaluated according the present invention;
Figure 23 is a graph that depicts the immune response of a melanoma patient as evaluated according the present invention;
Figure 24 is a graph that depicts IL-5 production from CD4+ T cells upon stimulation with the identified 15-mer peptide as shown in Figure 23;
Figure 25 is a graph that depicts the immune response of melanoma patients as evaluated according the present invention;
Figure 26 is a graph that depicts the immune response of three melanoma patients as evaluated according the present invention;
Figure 27 depicts a summary of new epitopes for CD4+ and CD8+ T cells within 4 melanoma antigens, i.e.
gp100, MART-1, NY-ESO1, and TRP-1, which are identified with EPIMAX methodology;
Figure 28 depicts the immune response of three melanoma patients as evaluated according the present invention;
Figures 29A-D are graphs that depict the immune response of a melanoma patients as evaluated according the present invention;
Figure 30 includes graphs that depict the immune response of a melanoma patient as evaluated according the present invention;
Figure 31 is a graph that depicts the immune response of melanoma patients as evaluated according the present invention;
Figure 32 is a graph that depicts the immune response of melanoma patients as evaluated according the present invention;
Figure 33 includes graphs that depict the immune response of three normal, healthy volunteers as evaluated according the present invention;
Figure 34 includes graphs that depict the immune response of three normal, healthy volunteers as evaluated according the present invention;
Figure 35 includes graphs that depict the immune response of normal healthy volunteers as evaluated according the present invention;
Figure 36 are graphs that depicts the immune response of normal healthy volunteers as evaluated according the present invention from frozen and thawed PBMCs;
Figure 37 are graphs that depicts the immune response of normal healthy volunteers as evaluated according the present invention from frozen and thawed PBMCs;
Figure 38A and 38B are graphs that depict the immune response of normal healthy volunteers as evaluated according the present invention;
Figure 39A and 39B are graphs that depict the immune response of normal healthy volunteers against the peptides identified in the EPIMAX assay and demonstrate that EPIMAX allows for the identification of epitope for functional Flu-MP specific CD4+ T cells;
Figure 40 are graphs that depict the immune response of a normal healthy volunteer as evaluated according the present invention and shows the identification of novel epitopes for specific CD4+ T cells;
Figure 41 A and B are graphs that depict the immune response of normal healthy volunteers against the peptides identified in the EPIMAX assay;
Figure 42 are graphs that depict the immune response of a melanoma patient as evaluated according the present invention and the antigenic epitopes for the patient, as both the peptides contain amino acid mismatches and show the ability to identify allogeneic-antigen specific T cells in a setting of organ transplantation;
Figure 43 is a graph that depicts the immune response of a type-1 diabetic patient as evaluated according to the present invention;
Figures 44A-44B depict the immune response of a melanoma patient as evaluated according the present invention, which allows the identification of various types of immune responses;
Figure 45 is a graph that depicts the immune response of five normal, healthy volunteers as evaluated according the present invention, which allows the identification of various types of immune responses by non-T cells;
Figure 46 includes two graphs that depict the immune response in a melanoma patient as evaluated according to the present invention, which allows the identification of various types of immune responses by non-T cells;
Figure 47 is a graph that depicts the immune response in a melanoma patient as evaluated according to the present invention, which allows the identification of various types of immune responses by non-T cells;
Figure 48 includes four graphs that depict the immune response in a melanoma patient as evaluated according to the present invention, which allows the identification of various types of immune responses by non-T cells; and
Figure 49 includes two graphs that depict the immune response in a melanoma patient as evaluated according to the present invention and demonstrates the identification of various types of immune responses by non-T cells.

### DETAILED DESCRIPTION OF THE INVENTION

To facilitate the understanding of this invention, a number of terms are defined below. Terms defined herein have meanings as commonly understood by a person of ordinary skill in the areas relevant to the present invention. Terms such as "a", "an" and "the" are not intended to refer to only a singular entity, but include the general class of which a specific example may be used for illustration. The terminology herein is used to describe specific embodiments of the invention, but their usage does not delimit the invention, except as outlined in the claims.

The present invention includes compositions and methods that are capable of assessing the antigen specific immune responses, without respect to the type of response induced. As used herein, the term "type of immune response induced" or "type of immune response," is used to describe the activation or regulation of an immune cell response via Th1, Th2, Tc1, Tc2 and combinations thereof or other T cell-based adaptive immunity regardless of the Major Histocompatibility Complex (MHC) presenting the peptide or antigen, whereby the T cell that recognizes and responds to the specific peptide or antigen in the context of MHC and its effect on the same cell, adjacent cells or cell that interact with that particular T cell or its effect on the cell presenting the peptide or antigen. The skilled immunologist will recognize that terminology may be overlapping, that is, it is common to refer to a protein that has been processed and loaded by an antigen presenting cell into a peptide, antigen or epitope, which is presented in the context of a particular MHC glycoprotein, that is, Class I or Class II.

It will be clear as used in context herein that the terms, "peptide," "antigen" or "epitope," are used in reference to the presentation of immune modulation by T cells after presentation by an antigen presenting cell. As used herein, the term "peptide" is used to describe a string of amino acids.

As used herein, the terms "antigen," "immunogen," "epitope" and "antigenic determinant," are used interchangeably to describe those peptides that modulate an immune response. As used herein the term, "modulate" is used to describe the activation, regulation, anergization or even suppression of an immune response as measured by, e.g., the proliferation of cell, cytokine or immunokine secretion, interactions between cells, apoptosis and the like. For example, a T cell response may be "modulated" in which certain cytokine or immunokines are released by the T cell that are capable of activating a certain type of response, e.g., antibody production; and concurrently reducing another type of response, e.g., the activation of cytotoxic T cells or NK cells.

As used herein, the term "immunomodulatory" is used to describe the effect that a particular antigen, immunogen, epitope or antigenic determinant has on effect cells, that is, cell that are activated in situ or even downstream in an effector cascade.

The present inventors recognized that many tools for prediction and characterization of antigenic epitopes have been previously developed. For example, computer modeling of peptide-major histocompatibility complex (MHC) interactions, based largely on X-ray crystal structures data of MHC peptide complexes, is often used to predict potential epitopes within proteins. Though this methodology has benefited greatly from the introduction of new prediction algorithms, it is, however, dependent on the size of the structure database and may therefore be less efficient at predicting MHC complexes not represented in that data set. This method provides a means of identifying antigenic epitopes but cannot be used to characterize the specific response elicited by those epitopes. (Flower DR, Novartis Found Symp. 2003;254:102-20; discussion 120-5, 216-22, 250-2).

Elution of peptides from MHC followed by mass spectrometry sequencing has been used successfully to identify antigenic epitopes (Hunt DF, Science. 1992 Mar 6;255(5049):1261-3). Although a powerful methodology for identification, peptide elution is expensive, slow, and requires a large sample size, making it impractical for use in a clinical setting. This method also provides a means of identifying antigenic epitopes but cannot be used to characterize the specific response elicited by those epitopes.

Overlapping peptide libraries have been used to identify antigenic epitopes in pathogen associated proteins (Martin R, Methods. 2003 Mar;29(3):236-47). Peptide library screening uses biological assays as readouts for reactivity and can, therefore, be used to assess the magnitude of response to the epitope. By using overlapping libraries, all potential Class I and Class II epitopes can be identified. To date, the biological assays used to evaluate potential antigenic epitopes using peptide libraries include the following methodologies: intracytoplasmic cytokines, ELISA, and cell proliferation (Martin R, Methods. 2003 Mar;29(3):236-47). Reactivity to a given peptide within the library in any of these assays is an indication that an antigenic epitope is present; however, none of these methods give a clear indication as to the quality of the response to that epitope, e.g., antigenic, allergenic, tolerogenic or any other specific response.

Combinations of known technologies useful for the prediction and characterization of antigenic epitopes have also been reported. Examples of combination assays are given below.

Peptide libraries and ELISPOT. The ELISPOT technique uses a surface bound capture antibody to bind cytokines secreted from cells cultured on the plate. A second labeled antibody is used to quantitate the number of cytokine secreting cells. By incubating cells with overlapping peptide libraries and assessing cytokine production by ELISPOT, antigenic epitopes have been isolated (Geginat G, J Immunol. 2001 Feb 1;166(3):1877-84). This methodology is limited, however, in that for any given sample, the production of only a few cytokines can be measured. This inability to measure the full range of cytokines and chemokines produced in response to epitope recognition makes any characterization of the immune response (i.e., Type-1, Type-2, or T-Regulatory) extremely difficult.

Peptide libraries and ELISA. The ELISA technique uses a surface bound capture antibody to bind soluble cytokines secreted from cells. A second labeled antibody is used to quantitate the concentration of secreted cytokine when compared to a standard curve. By incubating cells with overlapping peptide libraries and assessing cytokine production by ELISA, antigenic epitopes have been isolated. This methodology is limited, however, in that for any given sample the production of only a few cytokines can be measured. This inability to measure the full range of cytokines and chemokines produced in response to epitope recognition makes any characterization of the response (i.e., Type-1, Type-2, or T-Regulatory) extremely difficult. Furthermore, assessment of a single cytokine by ELISA requires a significant volume of at least 100 microliters of biological fluid and/or culture supernatant.

Peptide libraries and cellular proliferation assays. The cellular proliferation assay uses radionucleotide incorporation or fluorescent dye dilution to monitor cell division in response to stimulus. By incubating cells with overlapping peptide libraries and assessing cell division by cellular proliferation assay, antigenic epitopes have been isolated (Mutch D, J Acquir Immune Defic Syndr. 1994 Sep;7(9):879-90). This methodology is limited, however, in that although cell division may be an indication of the magnitude of antigenic response, this assay gives no information as to the quality of the response (i.e., Type-1, Type-2, or T-Regulatory). It should also be noted that some antigen-specific responses are not associated with rapid cellular proliferation such as T-Regulatory responses.

Peptide libraries and cytotoxic lymphocyte assays (CTL). The cytotoxic lymphocyte assay monitors the release of radionucleotide or fluorescent dye from antigen loaded (including but not limited to cDNA, virus and phage expression libraries, whole protein, protein fragments, peptide, modified peptide and lipids) target cells as a measure of epitope recognition and function of cytolytic cells. By incubating cells with overlapping peptide libraries and assessing cellular cytotoxicity by cytotoxic lymphocyte assay, antigenic epitopes have been isolated. Although cellular cytotoxicity may be an indication of the magnitude of a Type-1 antigenic response, this methodology is of limited use when assessing responses other than Type-1 (i.e., Type-2 or T-Regulatory). Characterization of antigen-specific cytotoxicity using CTL assays is often plagued with low sensitivity due to reporter release from target cells. It should also be noted that this methodology requires extensive cell culture manipulation and therefore, cannot be considered high throughput.

Peptide libraries and intracytoplasmic cytokine staining. The intracytoplasmic cytokine staining technique uses standard fluorescent staining procedures and anti-cytokine antibodies to measure the level of intracellular cytokines produced in response to an antigenic stimulus. Measurements of cytokine production and quantitation of cytokine-producing cells can then be accomplished by standard cytometric, microscopic or spectrophotometric techniques. By incubating cells with overlapping peptide libraries and assessing cytokine production by intracytoplasmic staining, antigenic epitopes have been isolated (Karlsson AC, J Immunol Methods. 2003 Dec;283(1-2):141-53). This methodology is limited, however, in that for any given sample, the production of only a few cytokines can be measured. This inability to measure the full range of cytokines and chemokines produced in response to epitope recognition makes any characterization of the response (i.e., Type-1, Type-2, T-Regulatory) extremely difficult. This methodology is further limited in that it requires a large numbers of cells for the analysis and is destructive to analyzed cells.

Peptide Elution and mass spectrometric sequencing. Elution of peptides from MHC followed by mass spectrometry sequencing has been used successfully to identify antigenic epitopes (Hunt DF, Science. 1992 Mar 6;255(5049):1261-3.). Although a powerful methodology for identification, peptide elution is expensive, slow, and requires a large sample size, making it impractical for use in a clinical setting. This method provides a means of identifying antigenic epitopes but cannot be used to characterize the specific response (i.e., Type-1, Type-2, T-Regulatory) elicited by those epitopes.

Computer modeling of peptide MHC interactions. Computer modeling of peptide-major histocompatibility complex (MHC) interactions, based largely on X-ray crystal structures data of MHC peptide complexes, is often used to predict potential epitopes within proteins. Though this methodology has benefited greatly from the introduction of new prediction algorithms, it is, however, dependent on the size of the structure database and may therefore be less efficient at predicting MHC complexes not represented in that data set (Kuhara S. Pac Symp Biocomput. 1999;:182-9.). This method provides a means of identifying antigenic epitopes but cannot be used to characterize the specific response elicited by those epitopes.

Serological identification of antigens by recombinant expression cloning (SEREX). SEREX technology involves identification of epitopes recognized by serum antibodies through combinatorial expression library screening. Although SEREX technology may give an indication as to the specificity of the humoral immune response, no such indication is given as to either the magnitude or nature of the cellular immune response (Tureci O, Mol Med Today. 1997 Aug;3(8):342-9.). This technology is both time and labor intensive, and therefore impractical in a clinical setting.

Real-time PCR for multiple cytokine analysis. Real-time PCR can be used to measure in a quantitative manner the expression of RNA encoding multiple cytokines (Giulietti A., Methods. 2001 Dec;25(4):386-401). This technology is, however, both time and labor intensive, requires large numbers of cells to isolate sufficient quantities of RNA for analysis, and is destructive to cells. Finally, the expression of RNA does not indicate the secretion of a biologically active cytokine.

As indicated above, technologies capable of predicting and characterizing antigenic epitopes have been reported, and in some cases, various technologies have been combined in order to maximize the amount of useful information, such as florescent based proliferation assays and intracellular cytokine assays. These combined assay approaches often suffer from the limitations of its individual component assays, such as the limitations associated with intracellular cytokine staining described above.

There is an urgent and thus far unfulfilled need for high throughput multiparameter technology, amenable to automation, non-destructive and requiring a small sample size making it applicable to a clinical diagnostic setting, allowing assessment of immune responses in humans. This need is particularly important in the view of emerging and re-emerging pathogens as well as biothreat agents where rapid assays providing the picture of immune response and/or identifying the nature of pathogens are needed. None of the methodologies described above provide a clear characterization of the qualitative and quantitative immune response to an antigenic epitope. With the exception of computer modeling, these methods do not provide the high throughput, limiting their utility from a diagnostic or immune-monitoring standpoint.

The present invention is defined in the claims. The present invention relates to a method to assess antigen-specific immune responses, irrespective of the type of response induced, thus permitting the evaluation of the type of immune response induced in both healthy and diseased individuals. This methodology gives both a qualitative and quantitative measure of the immune response, while simultaneously identifying the epitope to which that response is generated.

The methods of the present invention are a combination of three technologies: cell culture of immune cells from either humans or animals, presentation of antigenic material that is non-MHC limited, and analysis for the presence of cytokines and/or chemokines which provides for the ability to observe all varieties of immune responses. In one embodiment, the three technologies involve cell culture of immune cells from either humans or animals, overlapping peptide libraries that are non-MHC limited, and multiplex analysis for the presence of cytokines and/or chemokines. Preferably, the present invention is a multiphasic method to assess antigen-specific immune responses, wherein the first phase is a high-throughput screening (Phase I), and the second phase provides for identification of specific antigenic epitopes (Phase II).

Methods of the present invention use cell culture analysis with multiple cell types present that are capable of diverse antigen processing, presentation and recognition for both peptide and non-peptide epitopes (e.g., glycolipid). Suitable cells for the methods of the present invention include but are not limited to T cells, B cells, dendritic cells, monocytes, neutrophils, mast cells and red blood cells. Isolated peripheral blood mononuclear cells (PBMCs) including T cells, B cells, NK cells, and NK-T cells provide an easily obtainable source of multiple cell types for use in the present invention. Moreover, any standardized procedure known in the art for culturing human or animal cells which also supports the production of cytokines is suitable for use in the present invention.

In the methods of the present invention, the antigenic material is a protein.

In one embodiment, overlapping peptide libraries are used in the present invention to identify antigenic epitopes on a protein of interest and to stimulate immune responses. This technique provides for the identification of all possible epitopes Class I and Class II for any given antigen and is suitable for both CD4 and CD8 T-cell responses. And identification of the epitopes is not limited by the HLA-haplotype.

The amino acid sequence of the protein of interest is divided into small overlapping peptides, for example, a series of 8- to 15-mer overlapping peptides with, e.g., 3 to 4 amino acid offsets. According to the present invention, an overlapping peptide library can either be prepared or purchased from commercially available sources. For example, from the flu matrix protein containing 296 amino acids, an overlapping peptide library of 60 overlapping 15-mer peptides (with 4 amino acid offset) can be prepared.

From the overlapping peptide library, a cell culture container is prepared with one or more peptides adhered to the culture container. In one multiphasic method, multi-well Phase-I culture plates are used for screening purposes, wherein each well contains a cluster of several overlapping peptides. The number of overlapping peptides per cluster and the number of clusters required for screening depends upon the size of the protein itself. Preferably, each cluster contains about 5-10 overlapping peptides; for a smaller protein, about 3-7 overlapping peptides. In one multiphase method, the overlapping peptide library for a protein of interest is distributed by clusters into coded, preconditioned 96-well Phase-I culture plates, with 5-10 peptides per cluster per well. For Phase-II, the overlapping peptides in a reactive cluster are distributed as a single peptide per well. The Phase-I culture plates can be prepared in advance to maximize throughput and minimize assay-to-assay variability. Plates prepared in advance and stored at -80oC have a shelf life of a minimum one year. And to the extent that specific Phase-I clusters are found to be repeatedly antigenic, Phase-II culture plates can also be prepared in advance.

To begin the immune response assay of the present invention, immune cells of interest are added to the culture container described above and incubated for a time and under conditions that support the production of cytokines in response to the antigenic peptide(s) adhered to the culture container. The immune cells that can be assessed using this methodology include but are not limited to PBMCs, lymphocytes, T cells, CD4+ T cells, CD8+ T cells, NK cells, NKT cells, TCR □̃□ T cells, B cells, monocytes, dendritic cells and granulocytes. In one method, peripheral blood mononuclear cells (PBMCs) are used. In the multiphase method, for example, PBMCs are seeded at 2 x 105 per well into one or more coded, preconditioned 96-well Phase-I culture plates described above that contain an overlapping peptide library of the antigen of interest with 5-10 peptides per cluster per well. The immune cells are then incubated under conditions that support the production of cytokines, with the culture medium, temperature and incubation conditions selected for the specific cell type. For example, PBMCs are preferably incubated for 18-48 hours at 37° C in complete medium as described in Example 1. Following incubation, cells are pelleted and the supernatants are isolated from each well and analyzed for the presence of a wide variety of cytokines and chemokines. With respect to the present invention as described herein, the term "cytokines" is used to represent both cytokines and chemokines.

According to the present invention, any method known in the art that is capable of simultaneously analyzing the supernatants for the presence of several cytokines, preferably 20 or more cytokines, in the same sample can be used in the present invention. Such methods include mass spectroscopy, antibody-based array, and multiplex analysis bead technology. Cytokine multiplex analysis is used because it: i) allows for simultaneous measuring of multiple immune parameters, ii) requires small volume samples, iii) provides for a sensitive assay that does not destroy the sample; iv) supports coordinated expression of specific cytokines characteristic of specialized immune responses, and v) provides for a direct measure of immune effectors. A suitable commercially available cytokine multiplex analyzer is the Luminex 100 Cytokine Multiplex work station (Luminex Corp., Austin, Texas), which is capable of simultaneously measuring up to 100 analytes, requires a small volume sample, and rapidly detects the presence of cytokines within a dynamic range of 1-32,000 picograms per milliliter. Cytokine multiplex array technology (Luminex) permits the simultaneous quantitation of multiple cytokines and chemokines in a small volume (Earley MC, Cytometry. 2002 Oct 15;50(5):239-42). The assay is high throughput in nature and can be applied to the analysis of a wide variety of biological samples. By analyzing the coordinated cytokine expression in response to a stimulus or pathogenic insult, state specific biosignatures can be described corresponding to specific immune responses.

In one method, 50 microliters of the supernatants are transferred in 96 well format to a Luminex 100 Cytokine Multiplex work station (Luminex Corp., Austin, Texas) and analyzed according to manufacturer's instructions. Supernatants are rapidly screened in duplicate for the presence of a wide variety of cytokines using a master mix of pre-prepared and validated multiplex beads and reagents.

In the multiphase method, antigen clusters which prove to be positive for reactivity by Phase-I cytokine analysis are broken down into their constituent peptide components for antigenic peptide identification on an epitope focusing Phase-II culture plates as described above. In one method, Phase-II culture plates consist of coded, preconditioned well-strips containing the individual component peptides for each cluster in duplicate. Immune cells of interest are added to the well-strips and incubated for a time and under conditions that support the production of cytokines in response to the antigenic peptide(s) adhered to the well-strip. For example, thawed PBMCs are added at 2 x 10⁵ cells per well in the well-strips and cultured for 18-48 hours at 37° C. Following incubation the cells are pelleted, supernatants isolated and analyzed for cytokine production. In one method, 50 microliters of the supernatants are transferred to the Luminex workstation for analysis. Multiplex screening in Phase-II for a given peptide strip consists of the cytokine or cytokines produced in response to its parent cluster during Phase-I analysis. Peptides identified in a Phase II screen represent antigenic epitopes recognized by the individual. The characteristic pattern of cytokines and chemokines produced in response to the identified peptide are an indication of the type of specialized response elicited by that epitope.

The methods are a high throughput procedure for the rapid identification and characterization of peptide specific T-cell epitopes. As shown herein, development and validation of the assay has lead to the identification of a select group of cytokines and chemokines, which allow identification of Type-1, Type-2 and T-Regulatory cell phenotypes.

This methods of the present invention have several advantages over existing art including: a) high sensitivity: at picogram per milliliter detection for all cytokines tested; b) high throughput: assay can be completed within 48hrs; c) analysis of multiple analytes in a small volume of biological fluid, for example, assessment of at least 30 cytokines and chemokines in a 50 microliter volume; d) non-destructive: permitting the addition of cell based assays for multiparameter analysis; and e) identification of antigenic epitopes in a manner independent of HLA haplotype. The methods of the present invention use cytokine multiplexing to monitor multiple immune parameters and is therefore capable of characterizing the quality of the immune response including but not limited to antigenic, allergenic, and tolerogenic responses. By monitoring the coordinated response of multiple cytokines, identification and characterization of antigenic epitopes is also more sensitive than current state of the art technologies. None of the current art technologies either alone or in combination are able to provide the multiparameter assessment of cytokines and the identification and characterization of antigenic epitopes that the methods of the present invention can provide. Furthermore, the methods of the present invention encompass the advantages of a high-throughput technology. It should also be noted that as opposed to current state of the art technologies, the methods of the present invention are not destructive to the cells being analyzed; it can, therefore, be combined with any number of standard or custom immunoassays, including but not limited to proliferation, intracellular cytokine and surface receptor staining, CTL activity, and ELISPOT. Finally, the methods of the present invention require much smaller volumes of biological fluids and/or culture supernatant and much lower numbers of cells for analysis than any other currently available technology. Thus, the methods described are a high throughput, low volume, non destructive technology, amenable to automation and, therefore, practical for clinical diagnostic, prognostic and therapeutic use. The invention is specifically directed to predicting an immune response in a subject to an immune therapy.

In one aspect, a method that permits identification of immunomodulatory peptides and their derivatives, with diagnostic, prognostic and therapeutic applications is described. (a) Diagnostic: this technology can be used to assess the preexisting immune state of patients. By identifying and characterizing epitopes in a wide variety of antigens, a clear picture of the complete T-cell repertoire for that individual can be determined. This technology is high throughput, amenable to automation, non-destructive and requires a small sample size, making it applicable to a clinical diagnostic setting. It can be used, therefore, as a tool in the diagnosis of a number of auto-reactive diseases and hyperimmune responses including but not limited to allergy, diabetes, arthritis, lupus and multiple sclerosis. (b) Prognostic: the epitope-specific immune status of an individual prior to treatment as assessed by this technology can serve as a prognostic indicator of treatment outcome. Therefore, the course of therapeutic intervention can be tailored to enhance or eliminate this immune state. This technology can be used for prognostic assessment of anti-tumor immunotherapy, organ transplant, allergy and autoimmune diseases treatments. (c) Therapeutic: methods of the present invention can be used during the course of treatment as an immunomonitoring tool. A clear picture of the specificity, magnitude and quality of the epitope-specific immune response during therapy can serve to assess the efficacy of ongoing treatment. Based on data from this approach, treatments can be adapted to enhance, modulate or inhibit specific immune responses. This technology can, therefore, be used to guide ongoing therapeutic treatment in the areas of vaccine development, anti-tumor immunotherapy, organ transplant, allergy and autoimmune diseases.

In another aspect, the methods can be useful in the design of new drugs including but not limited to vaccines, bioactive peptides and targeting reagents. (a) Vaccines: the methods of the present invention can greatly facilitate the development of effective vaccines. Antigenic epitopes identified using this technology can themselves serve as effective vaccines to enhance the immune response against infectious disease or cancer, or to tolerize the immune response against proteins for the treatment of autoimmune disease, allergy, or organ transplant. (b) Bioactive peptides: bioactive peptides generated by proteolysis of larger proteins have been described with profound neuromodulatory and immunomodulatory functions. With this technology, peptides with these properties can be rapidly identified. (c) Targeting reagents: bioactive peptides identified using this technology which interact with specific receptor on cells can be used as a targeting reagent to deliver drugs to specific cellular populations.

The methods of the present invention are suitable for use in several medical applications including but not limited to chronic infections, allergy, neoplastic diseases, autoimmune diseases, Alzheimer's disease, acute infectious diseases, organ transplant, and atherosclerosis. It is to be understood that these applications are meant to be representative, and that other applications such as known in the art are contemplated as part of the present invention.

Chronic infection. A staggering number and variety of human diseases are caused by chronic pathogenic infection. Although the pathogenesis of these diseases may differ, they can all be characterized by an inability to mount and effective response, either Type-1 or Type-2, to the pathogen. Once a response is mounted, self epitope mimicry in pathogen associated proteins may also induce autoimmune responses which persist long after the infection is cleared, such as Lyme arthritis and Herpes associated demyelinating disease. The appearance of autoimmune reactions after pathogen immune recognition makes the selection of specific epitopes critical to a safe, effective therapy. Studies have implicated an inappropriate activation of regulatory response in some chronic infections. Methods of the present invention are suitable for diagnostic, prognostic and therapeutic applications regarding chronic infections. (a) Diagnostic: non-invasive diagnostic tests for some chronically infectious agents, such as PCR-based tests for HBV, demonstrate extreme variability in the clinical setting. The methods of the present invention can be applied to the diagnosis of chronic infection through the rapid detection and characterization of specific responses to pathogen-associated peptides. Diagnosis of pathogen-associated autoimmune complications can also be facilitated through the use of this technology. (b) Prognostic: the methods of the present invention can be used to detect and characterize reactivity, either Type-1, Type-2, or T-Regulatory, to specific pathogen-associated epitopes, which, in turn, can serve as a prognostic indicator of disease outcome, thereby guiding antibiotic therapy. The methods of the present invention are suitable for detecting and characterizing reactivity to specific pathogen-associated epitopes which can also serve as a prognostic indicator as to the onset of infection-associated autoimmune disease. (c) Therapeutic: the identification and characterization of antigenic epitopes to pathogen-associated proteins by means of the present invention can facilitate the development of safe, effective prophylactic and therapeutic vaccines. These would include the implementation of vaccines which avoid self epitope mimicry. Vaccines would also include the identification of pan-variant epitopes, targeting invariant epitopes within the pathogen proteome, thus providing protection against multiple strains of the same pathogen as well as common mutant variants of each pathogen as in the case of HIV and malaria. Identification and elimination of T-Regulatory epitopes and responding cells through application of this technology can play a role in initiating a protective anti-pathogen response.

Allergy. An allergy is a hyperimmune response to substances that are generally not harmful. It has been reported that an inappropriate shift to a Type-2 response plays a critical role in triggering an allergic immune response. Many allergens have been identified including inhalation allergens such as house dust mite, pollen, and mold; food allergens such as egg, wheat, soy, and nuts; or some drugs such as penicillin. A severe, often life-threatening reaction, called anaphylaxis, can result. Activation of a T-Regulatory response in the context of allergy, would, therefore, be advantageous. (a) Diagnostic: a skin test and blood test such as an allergen-specific IgE level is commonly used to identify allergens. However, a skin test can be dangerous to some patients who may experience anaphylaxis. Methods of the present invention can be applied as a safe diagnostic tool to identify patient-specific allergenic epitopes. (b) Prognostic: methods of the present invention allow identification of allergen epitopes for an individual allergy patient. This is useful to monitor immune response during treatment or for following-up. Tolerogenic T-Regulatory stimulatory epitopes can also be identified after treatment and can serve as a prognostic indicator of successful therapy. (c) Therapeutic: identification of an antigen epitope can lead to a new peptide-based vaccine which can be used to switch immune response to T-Regulatory or to selectively eliminate or suppress inappropriate Type-2 cells.

Neoplastic diseases. Neoplastic disease is defined as uncontrolled growth of abnormal cells that have mutated from normal tissues. These include cancer, solid tumors and malignancies of lympho/hematopoietic system, all of which are generally referred to as tumors. It has been reported that tumor environment induces immune tolerance against tumor antigens through several different mechanisms. While the development of Type-1 T cells against tumor cells is related to regression or suppression of tumor growth, the shifted immune response to tolerance inhibits the development of cancer-specific Type-1 T cells. However, in some cancers, tumor-antigen specific Type-1 T responses induce autoimmune disease by antigenic mimicry. For example, it has been reported that cytotoxic T cells specific to CDR2, a breast and ovarian tumor antigen, induce paraneoplastic cerebellar degeneration (PCD) in some patients.

The appearance of autoimmune reactions in the course of anti-tumor immunity makes the selection of specific epitopes critical to a safe, effective therapy. (a) Diagnostic: diagnosis of tumor-associated autoimmune complications can be facilitated through the use of the methods of the present invention. (b) Prognostic: detecting and characterization of reactivity, either Type-1, Type-2, or T-Regulatory, to specific tumor-associated epitopes using this technology can serve as a prognostic indicator of disease outcome. Methods of the present invention allow evaluation of any kind of vaccine treatment by comparing the quality and magnitude of tumor-specific immune responses between pre-vaccine and post-vaccine. Detection and characterization of reactivity to specific tumor-associated epitopes using this technology can serve as a prognostic indicator as to the onset of tumor-associated autoimmune disease. (c) Therapeutic: the identification and characterization of antigenic epitopes to tumor-associated proteins by means of the present invention can facilitate the development of safe effective therapeutic vaccines. This would include the implementation of vaccines, which avoid self epitope mimicry.

Autoimmune diseases. Autoimmune diseases can be defined as disorders caused by an immune response against the body's own tissues. An inappropriate shift away from T-Regulatory function in the context of self antigen presentation is thought to be a critical factor in the onset and progression of autoimmune disease. The specific nature of the resulting disease is dependent on the dominant response. For example, in the case of Type-1 diabetes, Type-1 cells recognizing pancreatic islets have been isolated in patients. (Arif S, J Clin Invest. 2004 113:451). Furthermore, in multiple sclerosis patients, there is evidence indicating that myelin basic protein (MBP)-reactive Type-1 polarized T-cells undergo in vivo activation and clonal expansion, which has been reported to play a major role in the pathogenesis. A restoration of T-Regulatory function would be critical to reconstitution of normal immune homeostasis. (a) Diagnostic: methods of the present invention allow identification of dominant self epitopes responsible for expansion of a given type of autoreactive immune cells. (b) Prognostic: methods of the present invention allow identification of self antigen epitopes for an individual autoimmune patient. This is useful to monitor immune response during treatment or for following-up. Tolerogenic T-Regulatory stimulatory epitopes can also be identified after treatment and can serve as a prognostic indicator of successful therapy. (c) Therapeutic: identification of an antigen epitope can lead to a new peptide-based vaccine which can switch immune response to T-Regulatory or to selectively eliminate or suppress inappropriate Type-1 or Type-2 cells.

Alzheimer's disease. Alzheimer's disease (AD) is a slowly progressive form of dementia, which is a progressive, acquired impairment of intellectual functions. AD is characterized by the progressive deposition of the 42-residue amyloid beta protein (Abeta) in brain regions. Reports indicate that a significantly higher proportion of healthy elderly subjects and patients with AD had strong Abeta-reactive T-cells responses of both Type-1 and Type-2 character when compared to age-matched adults. (a) Diagnostic: in many cases, differential diagnosis between Alzheimer's disease and senile dementia can only be conclusively determined post-mortem. Methods of the present invention can be used to identify A beta reactive T cell responses, which can, therefore, serve as a diagnostic indicator of AD. (b) Prognostic: identification of specific immune responses by means of the present invention can allow prediction of the clinical course of disease.

Acute infection. The induction of Type-1 response against pathogens is critical for recovery from infection of intracellular microbes such as bacteria and viruses. In contrast, extracellular pathogens such as helminthes induce the development of Type-2 cells, whose cytokines direct IgE and eosinophils-mediated destruction of pathogens. Some viruses, such as Ebola virus or Dengue virus, cause severe illness with high mortality rate, for both of which licensed vaccine is not established. In a mouse model, vaccines with virus-like particles (VLPs) derived from Ebola virus glycoprotein (GP) and matrix protein (VP40) has been proved to activate both T cells and B cells, and protects from viral challenge. (a) Diagnostic: methods of the present invention are suitable for the identification of novel epitopes that diagnose the presence of emerging and re-emerging pathogens and biothreat agents. (b) Therapeutic: methods of the present invention can identify T cell epitopes for viral antigens by screening recovered patients from these diseases. Thus, new peptide-based vaccines can be made against acute fatal viral infections.

Organ Transplant. Rejection of an organ transplant is a hyperimmune response generally driven by a dominant Type-1 response against the engrafted organ or, as in the case of graft versus host disease (GVHD), against the recipient. Regulatory function may play a critical role in developing tolerance and long term graft acceptance. Activation of T-Regulatory, or selective elimination of dominant Type-1 responses in the context of organ transplant, would therefore be advantageous. (a) Diagnostic: methods of the present invention are suitable for determining a pattern of immune reactivity which can be predictive of the development of acute GVHD and most particularly chronic GVHD. (b) Prognostic: prior to transplant or emergence after, the existence dominant Type-1 epitopes in either donor or recipient samples can be a prognostic indicator of graft rejection and the present invention is suitable for determining the presence of dominant Type-1 epitopes. Tolerogenic T-Regulatory stimulatory epitopes can also be identified by means of the present invention after treatment and can serve as a prognostic indicator of graft tolerance. (c) Therapeutic: identification of antigen epitopes by means of the present invention can lead to a new peptide-based vaccine which can switch immune response to T-Regulatory, or to selectively eliminate or suppress inappropriate Type-1 cells.

Atherosclerosis. Atherosclerosis is a common disorder of the arteries. Fat, cholesterol and other substances accumulate in the walls of arteries and form "atheromas" or plaques. It is currently appreciated that chronic inflammatory cell-mediated immune responses are involved in the pathogenesis of atherosclerosis. T-cell activation in atherosclerotic plaques is reported to be initiated by plaque-derived antigens, such as oxidized LDL (oxLDL). Others reported detection of Chlamydia pneumoniae-reactive T cells in human atherosclerotic plaques of carotid artery. (a) Diagnostic: methods of the present invention are suitable for identification of early patterns of immune reactivity. (b) Prognostic: methods of the present invention allow for the quantitative measurement of epitopes of antigens related to pathogenesis of atherosclerosis such as oxLDL or cross-reactive Chlamydia antigen, which are recognized by T cells. (c) Therapeutic: methods of the present invention are suitable for determining specific epitopes that can be used to develop new peptide-based vaccines to prevent progression of atherosclerosis.

It is to be understood that the examples given below are representative of the invention and are intended to be illustrative of the invention, but are not to be construed to limit the scope of the invention in any way. In particular, the method for measuring cytokine production presented in the examples is merely representative and any method known in the art for measuring cytokine concentrations can be used in the present invention.

Example 1: Antigen specific immune responses detected as early as 48 hours by simple incubation of normal donor PBMCs with a viral peptide. To determine if an antigen specific immune response can be detected by the methods of the present invention using PBMCs and single peptide, PBMCs freshly prepared from an HLA-A0201+ normal volunteer were incubated with HLA-A0201 restricted peptides. The donor was known to have Flu-MP-specific CD8+ T cells but not Mage 3-specific CD8+ T cells by other methods (data not shown).

Peripheral blood mononuclear cells (PBMC) were isolated from freshly drawn blood from a HLA-A0201+ healthy volunteer by Ficoll-Paque density gradient centrifugation. PBMC were resuspended at a concentration of 1 x 10⁶ cells/ml in RPMI medium supplemented with 10% heat-inactivated human AB serum (Gemini Bio-Products), L-glutamine (2 mM), penicillin (200 UI/ml), streptomycin (200 µg/ml), sodium pyruvate (1 mM), 1% non-essential amino acid, 2-β-mercaptoethanol (50 µM, Sigma), and HEPES pH 7.4 (25 mM, Gibco) (complete medium: CM). 2 x 10⁵ cells/well were seeded in triplicates in round-bottom 96-well plates, and incubated with 1 µl of either of HLA-A0201 restricted peptides (stock concentration 1 mg/ml, culture concentration 10 µg/ml); Flu-MP58-66 (GILGFVFTL), or MAGE-3271-279 (FLWGPRALV, MultiPeptide Systems, San Diego, CA), or peptide diluent (5% DMSO in H₂O). Culture supernatants were harvested 48 hours later, and cytokines and chemokines were measured with Luminex.

PBMCs stimulated with Flu-MP peptide induced a large array of cytokines including IFN-γ within 48 hours (data not shown). The results demonstrated that fresh PBMC from a normal donor respond to Flu-MP peptide and produce a large array of cytokines and that antigen specific immune responses can be detected as early as 48 hours by simple incubation of PBMCs with a peptide.

Example 2: Antigen specific immune responses detected as early as 48 hours for a broad repertoire of peptide reactive cells in PBMC including tumor antigens. Viral antigens are often able to induce stronger recall responses than tumor antigens. To determine if tumor-antigen specific immune responses can be detected by the methods of the present invention, the cryopreserved PBMCs in liquid nitrogen from 2 melanoma patients who received at least 8 injections of DC vaccine (autologous CD34+ hematopoietic progenitor cell-derived DCs loaded with 4 HLA-A2 peptides of melanoma associated antigens) were thawed with cold PBS. After a second washing with PBS, PBMCs were incubated in CM for 15 min at 37° C, and cell debris was removed with a nylon cell filter. Cells were washed again with CM, and resuspended in CM at 1 x 10⁶/ml. Then, 2 x 10⁵ cells/well were seeded in triplicates in round-bottom 96-well plates, and incubated with 1 µl of each of HLA-A0201 restricted peptides: (stock concentration 1 mg/ml, culture concentration 10 µg/ml); Flu-MP 58-66, CMV PP65 (NLVPMVATV, Biosynthesis, Lewisville, TX), MAGE-3 271-279 , MART-1 27-35 (AAGIGILTV), gp100g209-2M (IMDQVPFSV), Tyrosinase 368-376 (YMDGTMSQV, NCI), or peptide diluent (5% DMSO in H2O). Culture supernatants were harvested 48 hours later, and produced cytokines were measured with Luminex technology. Additionally, ELISPOT assay was performed for the detection of antigen-specific IFN-γ-producing T cells according to the manufacturer's protocol (Mabtech). Briefly, PBMCs (2 x 10⁵ cells/well) were added to plates precoated with 10 µg/ml of a primary anti-IFN-γ monoclonal antibody (Mabtech, Stockholm, Sweden) in the presence or absence of 10 µg/ml peptides.

Patient 1, who was known to have CD8+ T cells for Flu-MP and CMV by direct IFN-γ ELISPOT (data not shown), demonstrated induction of IL-1 α, IFN- y, TNF- α, and IP-10 in response to Flu-MP or CMV peptide, but not with 4 melanoma peptides (data not shown). This shows melanoma peptides do not modulate immune response nonspecifically. Patient 2, who was demonstrated to have MART-1 specific CD8+ T cells as well as Flu-MP or CMV (data not shown), displayed slight increase of IL-1 a and marked increase of IP-10 in response to MART-1 peptide, but not with other melanoma peptides (data not shown). Results indicate that fresh PBMC from the normal donor respond to Flu-MP peptide and produce IL-1 α and IP-10. The cytokine/chemokine production profiles are different between two different normal donors, suggesting that different types of CD8+ T cells recognizing the same Flu-MP peptide can be identified with EPIMAX assay. Given the fact that IP-10 can be upregulated in response to IFNs, DC-T interactions may induce IFN- γ production from T cells, which leads to IP-10 production from DCs. Thus, IP-10 could be a useful marker for Type-1 T cell response.

Example 3: The induction of IP-10 in response to Mart-1 peptide in a melanoma patient vaccinated with peptide-loaded CD34-DCs is dependent on IFN-γ. To examine whether IP-10 production is dependent on IFN-γ, PBMCs obtained from a melanoma patient, who was vaccinated with autologous CD34-DCs loaded with HLA-A2 melanoma peptides, were stimulated with either MART-1 peptide#6 (10 µM) or Flu-MP HLA-A2 peptide (10 µg/ml) in the presence of blocking anti-IFN-γR1 mAb. IP-10 production was completely abrogated by blocking of IFN-γR1 (data not shown). Thus, IP-10 production was dependent on IFN-γ production, indicating IP-10 can be a surrogate marker for IFN-γ production.

Thawed cryopreserved PBMCs in liquid nitrogen from 2 melanoma patients who received at least 8 injections of DC vaccine (autologous CD34⁺ hematopoietic progenitor cell-derived DCs loaded with four (4) HLA-A2 peptides of melanoma associated antigens) were seeded in triplicates in round-bottom 96-well plates, and incubated with 10 µg/ml of each of HLA-A0201 restricted peptides: Flu-MP₅₈₋₆₆, CMV PP65 (NLVPMVATV), MAGE-3₂₇₁₋₂₇₉(FLWGPRALV), MART-1₂₇₋₃₅ (AAGIGILTV), gp100_{g209-2} M (IMDQVPFSV), Tyrosinase₃₆₈₋₃₇₆ (YMDGTMSQV), or peptide diluent (5% DMSO in H₂O); and culture supernatants were harvested 48 h later and evaluated quantitatively for the presence of cytokines and chemokines using Luminex technology. ELISPOT assay was performed for the detection of antigen-specific IFN-γ-producing T cells, wherein PBMCs (2 x 10⁵ cells/well) were added to plates precoated with 10 µg/ml of a primary anti-IFN-γ monoclonal antibody in the presence or absence of 10 µg/ml peptides. The results demonstrated a broad repertoire of peptide reactive cells in PBMC were identified by the method of the present invention.

Example 4: Methods of present invention allow identification of specific peptide sequence recognized by T cells. To determine if antigen epitope(s) recognized by T cells and an immune response induced by that epitope can be identified with the methods of the present invention, 5 clusters of peptides consisting of 4-7 peptides from the 15-mer MART-1 peptide library, were incubated with PBMCs obtained from an HLA-A0201+ melanoma patient who received CD34-DC vaccine. 15-mer overlapping peptides with four (4) amino acid offsets were designed to cover whole amino acid sequence of MART-1 (Mimotopes, San Diego, CA). Each peptide was reconstituted with 50% Acetonitrile at 2 mM, and kept -80° C until use. PBMCs were obtained from a melanoma patient who received DC vaccines. 4-6 clustered peptides (1 µl per peptide), or single peptide (1 µl) were seeded in triplicates in 96-well plates, and dried at room temperature. Then, 2 x 10⁵ PBMCs in 200 µl CM were added to each well and incubated for 48 hours at 37° C in a humidified 5% CO₂ incubator. The cytokine or chemokine levels in culture supernatant were measured with Luminex.

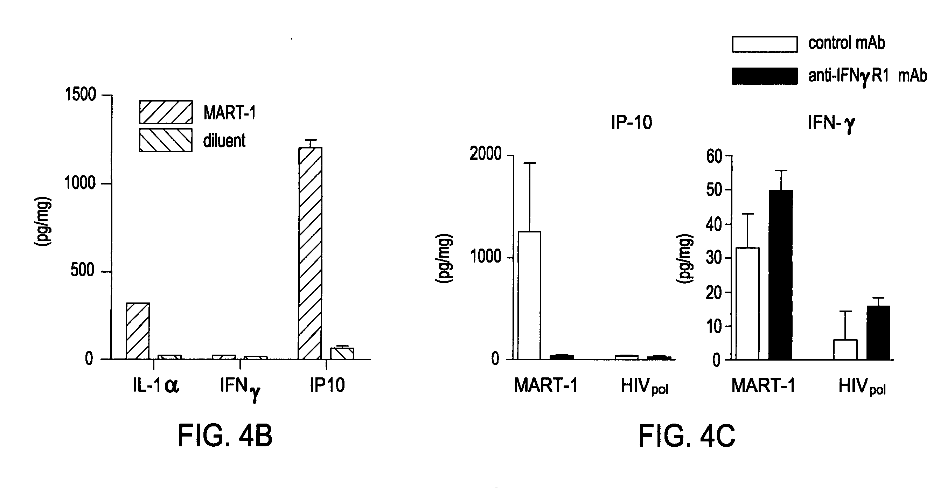

It was found that a significantly higher amount of IP-10 was detected in the cluster #1 culture. Next, a second culture of PBMCs with a single peptide from MART-1 library was made to identify the responsible peptide within cluster #1. Peptide #6 induced marked IP-10, indicating that this peptide is the epitope for the melanoma patient's T cells. Interestingly, this peptide contains HLA-A0201 dominant epitope sequence (MART-127-35; AAGIGILTV). PBMCs were obtained from a melanoma patient who received DC vaccines. This patient is HLA-A*0201^{pos}, and was shown to have MART-1 specific CD8⁺ T cells recognizing HLA-A*0201-restricted dominant epitope (MART-1₂₆₋₃₅) with tetramer binding assay (data not shown). When PBMCs were cultured with this HLA-A*0201 dominant MART-1 peptide, two cytokines, IL-1 α and IP-10, were specifically induced, but no upregulation of IFN-γ was noted (data not shown). Given that IP-10 is secreted by many cell types in response to type I or type II interferons, we surmised that IP-10 was produced in response to IFN-γ secreted from MART-1 specific T cells. Accordingly, IP-10 production was completely abrogated by blocking the function of IFN-γ (Figure 4C). The results indicate that the induction of IP-10 in response to MART-1 peptide in the melanoma patient vaccinated with peptide-loaded CD34-DCs is dependent on IFN-γ.

To check if this patient's PBMCs contain CD8+ T cells recognizing this dominant epitope, PBMCs were cultured with autologous monocyte-derived DCs loaded with MART-127-35 or other HLA-0201 peptides (e.g., Mage-3, Tyrosinase, and Flu-MP), and examined for the induction of antigen-specific CD8+ T cell population with specific tetramers. As shown in Figure 4B-4E, MART-1 specific CD8+ T cells were easily expanded within 7 days, indicating that this patient has memory or effector CD8+ T cells for MART-1 and that this CD8+ T cell population was recognized according to methods of the present invention with the MART-1 library.

Example 5: Methods of present invention allow identification of various types of immune responses. Methods of the present invention allow simultaneous identification of epitopes for immune response as well as the type of response induced. According to general methods given in Example 4, PBMCs from melanoma patients either pre-vaccine or post-vaccine were incubated with 5-6 peptide clusters from MART-1 or NY-ESO1 peptide libraries for 48 hours (MART-1 with 5 clusters; NY-ESO1 with 8 clusters). Typical results were shown to represent signatures of cytokine profiles corresponding to different immune responses (data not shown). The peptide clusters displayed the pattern of each immune response were shown in gray bars. In a Type-1 response, IL-10 production is suppressed and IP-10 is induced. On the contrary, in a T-Regulatory response, more IL-10 is induced and IP-10 production is abrogated because of the inhibition of Type-1 responses. IL-13 and eotaxin are detected in Type-2 responses without affecting the level of IP-10. Thus, the present invention identifies various types of immune responses induced by any kind of antigens in as fast as 48 hours. 2 x 10⁵ fresh PBMCs from an HLA-A0201+ donor were stimulated with HLA-A0201 restricted Flu-MP58-66, Mage3271-279, or diluent in triplicates for the indicated periods. Cytokines in the culture supernatant were measured with multiplex bead-based cytokine assay. Mean±SD are shown from triplicate data. This result shows that the peptide-specific production of IL-1a and IP-10 reached plateau at 48 h of peptide stimulation.

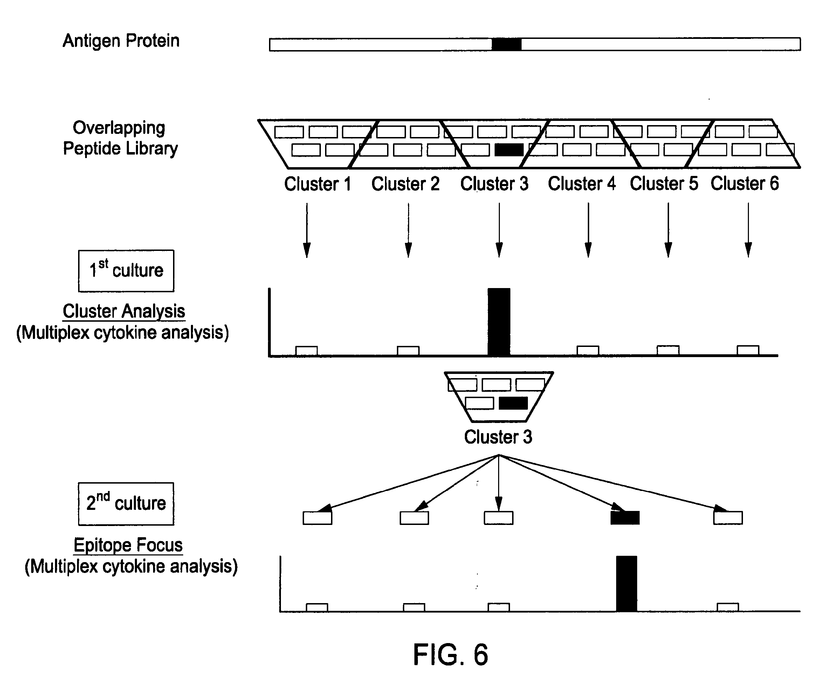

Example 6: Methods of present invention allow identification of other types of immune responses induced by non-T cells. It is known that 15-mers peptides are recognized by other immune cells, such as B cells, NK cells, or NK-T cells, and that some peptides bind to an unidentified receptor, leading to modulation of immune responses. Another example of the present invention was used to identify an immune response induced by non-T cells with a peptide that exhibited a regulatory signature in an MHC-non restricted. PBMCs from a melanoma patient were incubated with peptide clusters from survivin library. As shown in Figure 6A-6H, peptide #15 from the survivin cluster #3 induced a strong regulatory pattern of cytokine production. Namely, IL-10 and IL-1β were strongly induced, and IL-1α and IP-10, which are markers for Type-1 responses, were severely abrogated.

To determine if this response is restricted to a certain type of MHC-molecule, PBMCs derived fresh blood taken from 5 normal healthy volunteers were incubated with survivin peptide #15 for 48 hours, and cytokine concentrations were measured according to the methods of the present invention. Peptide #15 inhibited IP-10 production in all five normal volunteers (data not shown), indicating that this peptide is not restricted to a certain HLA subtype. Survivin Peptide #15 was shown to have a strong capacity to modify immune response, because PBMC from a melanoma patient stimulated with live flu virus in the presence of Peptide#15 exhibited strong abrogation of IP-10 induction, apparently due to IL-10 production (data not shown). Furthermore, Peptide #15 partially inhibited TSST-1 induced T cell proliferation (data not shown). Depletion studies showed that CD56+ cells were responsible for IL-10 production and IP-10 blockade (data not shown), indicating that survivin Peptide #15 acts on the NK, NK-T cell or gamma/delta T cell population. Peptide#15 was also shown to maintain the survival of CD3-CD56+ NK cells (data not shown). Thus, the methods of the present invention are useful for identification of any kind of immune modulation induced by certain types of peptides.

Example 7: Methods of present invention allow for immunomonitoring of melanoma patient during vaccination. Cells were seeded at 2 x 10⁵ per well in coded, preconditioned 96 well Phase-I culture plates. Phase-I culture plates contain 5 peptide clusters each, of the tumor antigens GP100, MAGE3, NY-ESO, and MART-1, in quadruplicate with 12 overlapping peptides, each 15 amino acids in length, per cluster, as well as KLH and killed Colo cell controls. Plates may be prepared in advance to maximize throughput and minimize assay to assay variability. Cells are incubated for 5 days at 37° C. In parallel, cells designated for restimulation are cultured for 7 days in the presence of autologous mature DCs loaded with the full spectrum of antigenic peptide clusters at a ratio of 30:1 PBMC to DC. On the seventh day, these cells are extensively washed and transferred to a Phase-I culture plate for 24 hours. Following incubation, these restimulated cells will are pelleted, and 150 microliters of each supernatant is transferred in 96-well format to a fully automated Luminex 100 Cytokine Multiplex work station equipped with a TECAN Genesis RPS robotic sample processor (TECAN).

A workstation capable of operator independent sample processing may be used with barcode identification and random microplate analysis. Supernatants are rapidly screened in duplicate for the presence of IFN-γ, TNF-α, IL-13, IL-10 and Granzyme-B, using a master mix of pre-prepared and validated multiplex beads and reagents. Remaining supernatant samples are frozen for later analysis. It is anticipated that DCs from the co-culture may contribute to background cytokine production during the 24-hour incubation in the Phase-I plate. Supernatants from mature DCs serve, therefore, as a control in the cytokine multiplex analysis. Cells from the primary stimulation assay are spun down and resuspended in media containing H3 thymidine and incubated 37° C. After 5 days, antigen specific proliferation is assessed in quadruplicate by a thymidine incorporation assay. Supernatants from the primary stimulation assay are frozen at -80° C for analysis at a later date. Those peptide clusters, which are positive for cytokine production after restimulation, are screened for cytokine production during primary stimulation.

Tumor antigen clusters which prove to be positive for reactivity by Phase-I cytokine multiplex array, are broken down into their constituent components for antigenic peptide identification on Phase-II culture plates. Phase-II culture plates includes well-strips containing the 12 component peptides for each cluster in duplicate. Phase-II peptide well-strips are prepared in advance and set into bar code labeled frames. Thawed PBMCs are cocultured for 7 days at 37° C with autologous mature DCs loaded with the specific peptide cluster identified in Phase-I, washed and transferred to a Phase-II culture plate for 24 hours at 2 x 10⁵ per well. Following incubation, cells are pelleted, and 150 microliters of supernatants are transferred to the Luminex workstation for analysis. Multiplex screening in Phase-II for a given peptide strip consists of the cytokine or cytokines produced in response to its parent cluster during Phase-I analysis. Mature DCs alone serve as a background control for Phase-II analysis. Once the antigenic peptide or peptides of a given cluster are identified, they are used to isolate the antigen specific effector populations.

Phase-III monitoring consists of identification and characterization of peptide specific effector cells. In this procedure, 2 x 10⁶ PBMCs are thawed and incubated for 7 days at 37° C with autologous mature DCs loaded with each antigenic peptide isolated in Phase-II. Cells are then washed and subjected to a 24 hour restimulation. Following restimulation, cells are brefeldin-A treated, fixed, permeabilized and stained with a panel of antibodies to characterize both surface marker and intracellular cytokine expression. Identification of antigen specific effector populations is accomplished by multicolor flowcytometric analysis using a FACSaria flow cytometer/sorter (Becton-Dickinson), capable of 9 color analysis and sorting. The CTL function of stimulated T cells is determined in vitro by a Cr51 release assay using peptide loaded, EBV transformed, autologous B-LCLs as targets. Following identification, antigen specific effector cell populations are subcloned by coculturing for 7 days with autologous mature antigenic peptide loaded DCs in the presence of IL-2 and IL-6, followed by weekly restimulations with peptide loaded mature DCs in the presence of IL-2 and IL-7. Once clonal effector cell populations are established, an extensive characterization of gene expression patterns and cytotoxic function is undertaken.

Example 8: Methods of present invention allow selection of vaccine epitopes in cancer. Detecting and characterization of reactivity, either Type-1, Type-2, or T-Regulatory, to specific tumor-associated epitopes using this technology can serve as a tool for the identification and characterization of antigenic epitopes to tumor-associated proteins that elicit a desired type of immune response when used for vaccination.

To select vaccine epitopes, a patient's PBMCs are exposed to a series of peptide libraries representing antigenic epitopes from tumor associated proteins. After 48 hours culture, supernatants are harvested, and secreted cytokines are analyzed according to the teaching of the present invention. Clusters of peptides that induce a Type-1 T cell response, for example, production of IFN-γ but no production of IL-10 (T-Regulatory response) or Type 2-cytokines are identified. In the next step, peptides corresponding to a given patient HLA type can be further selected and used for vaccination of this particular patient. Thus, agents for vaccination tailored to produce a desired type of immune response in a patient can be determined with the present invention.

Example 9: Methods of present invention allow selection of vaccine epitopes in infectious diseases. It is known that many of currently available vaccines against microbial agents do not confer protective immunity, for example Dengue virus vaccine. Lack of the protective immunity can be due to the induction of an inappropriate immune response, for example, some of the current vaccine epitopes may induce Type-2, or Regulatory T cells rather than Type-1 T cells, thus skewing and/or inhibiting the immune response towards a non-protective one. In this example, the technology of the present invention can serve as a tool for the identification and characterization of antigenic epitopes of microbe associated antigens that elicit a desired type of immune response when used for vaccination.

To select vaccine epitopes, a patient's PBMCs are exposed to a series of peptide libraries representing antigenic epitopes from a microbe, for example Dengue virus. After 48 hours culture, supernatants are harvested, and secreted cytokines are analyzed according to the teaching of the present invention. Clusters of peptides that induce a Type-1 T cell response, for example, production of IFN-γ but no production of IL-10 (T-Regulatory response) or Type-2 cytokines are identified. In the next step, peptides are further selected and used for vaccination. Thus, the methods of the present invention allows for the selection of epitopes that permit the induction of protective antimicrobial immunity upon vaccination.

Figure 1 is a graph that depicts the immune response of a normal donor evaluated according the present invention, wherein peripheral blood mononuclear cells (PBMC) were isolated from freshly drawn blood from an HLA-A0201⁺ healthy volunteer, seeded at 2 x 10⁵ cells/well in triplicates in round-bottom 96-well plates, and incubated with 1 µl of either of HLA-A0201 restricted peptides Flu-MP₅₈₋₆₆ (GILGFVFTL) or MAGE-3₂₇₁₋₂₇₉ (FLWGPRALV), or peptide diluent (5% DMSO in H₂O); and culture supernatants were harvested 48 h later and evaluated quantitatively for the presence of cytokines and chemokines using Luminex technology. Results indicate that fresh PBMC from the normal donor respond to Flu-MP peptide and produce a large array of cytokines.

Figure 2 is a graph that depicts another example of immune response to HLA-A0201 restricted peptides Flu-MP₅₈₋₆₆ (GILGFVFTL) in a normal donor according the present invention. PBMCs were isolated from freshly drawn blood from another HLA-A0201⁺ healthy volunteer, seeded at 5 x 10⁵ cells/well in triplicates in round-bottom 96-well plates, and incubated with 10 µg/ml of either of HLA-A0201 restricted peptides Flu-MP₅₈₋₆₆ (GILGFVFTL) or MART-1₂₇₋₃₅ (AAGIGILTV), or peptide diluent (5% DMSO in H₂O); and culture supernatants were harvested 48 h later and evaluated quantitatively for the presence of cytokines and chemokines using Luminex technology. Results indicate that fresh PBMC from the normal donor respond to Flu-MP peptide and produce IL-1α and IP-10. The cytokine/chemokine production profiles are different between two different normal donors, suggesting that different types of CD8+ T cells recognizing the same Flu-MP peptide can be identified with EPIMAX assay.

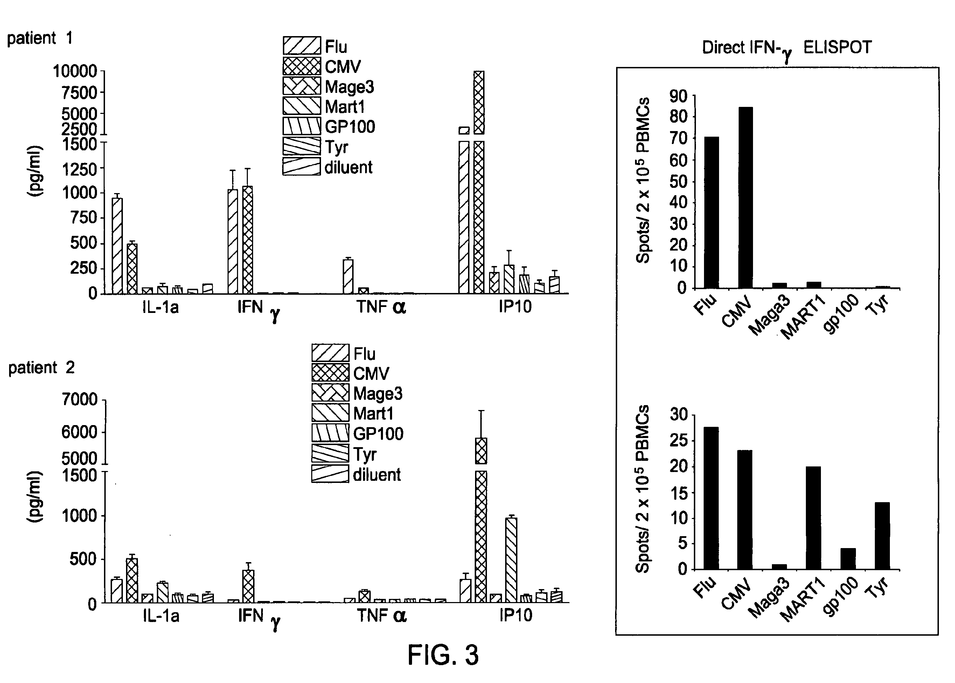

Figures 3A-3D are graphs that depict the immune response of 2 melanoma patients as evaluated according the present invention. In Figures 3A and 3C, for Patient 1 and 2, respectively, thawed cryopreserved PBMCs in liquid nitrogen from 2 melanoma patients who received at least 8 injections of DC vaccine (autologous CD34⁺ hematopoietic progenitor cell-derived DCs loaded with four HLA-A2 peptides of melanoma associated antigens) were seeded in triplicates in round-bottom 96-well plates, and incubated with 10 µg/ml of each of HLA-A0201 restricted peptides: Flu-MP₅₈₋₆₆, CMV PP65 (NLVPMVATV), MAGE-3₂₇₁₋₂₇₉(FLWGPRALV), MART-1₂₇₋₃₅ (AAGIGILTV), gp100_{g209-2} M (IMDQVPFSV), Tyrosinase₃₆₈₋₃₇₆ (YMDGTMSQV), or peptide diluent (5% DMSO in H₂O); and culture supernatants were harvested 48 h later and evaluated quantitatively for the presence of cytokines and chemokines using Luminex technology. In Figures 3B and 3D for Patient 1 and 2, respectively, ELISPOT assay was performed for the detection of antigen-specific IFN-γ-producing T cells, wherein PBMCs (2 x 10⁵ cells/well) were added to plates precoated with 10 µg/ml of a primary anti-IFN-γ monoclonal antibody in the presence or absence of 10 µg/ml peptides. The results indicate that a broad repertoire of peptide reactive cells in PBMC was identified by the method of the present invention.

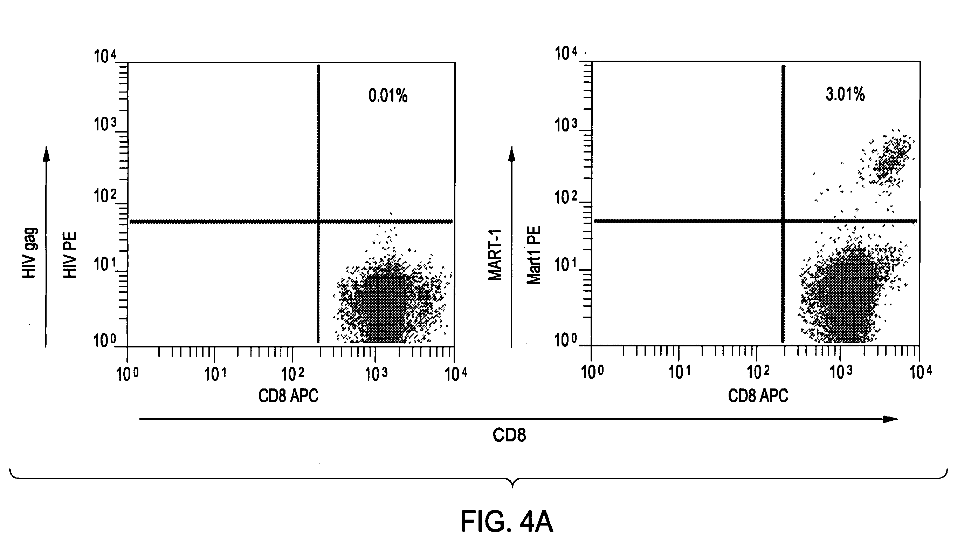

Figures 4A-4C are graphs that depict the immune response of a melanoma patient as evaluated according the present invention. For Figure 4A, PBMCs were obtained from an HLA-A*0201^{pos} melanoma patient who received DC vaccines, and stimulated with autologous DCs loaded with HLA-A*0201-restricted dominant MART-1 peptide (MART-1₂₆₋₃₅). The patient was shown to have MART-1 specific CD8+ T cells recognizing HLA-A*0201-restricted dominant epitope (MART-1₂₆₋₃₅) with tetramer binding assay (Figure 4A). When PBMCs were cultured directly with this HLA-A*0201 dominant MART-1 peptide, two cytokines, IL-1α and IP-10, were specifically induced, but no upregulation of IFN-γ was noted (Figure 4B). Given that IP-10 is secreted by many cell types in response to type I or type II interferons, we surmised that IP-10 was produced in response to IFN-γ secreted from MART-1 specific T cells. Accordingly, IP-10 production was completely abrogated by blocking the function of IFN-γ using IFNγR blocking mAb (Figure 4C). The results indicate that the induction of IP-10 in response to MART-1 peptide in the melanoma patient vaccinated with peptide-loaded CD34-DCs is dependent on IFN-γ.

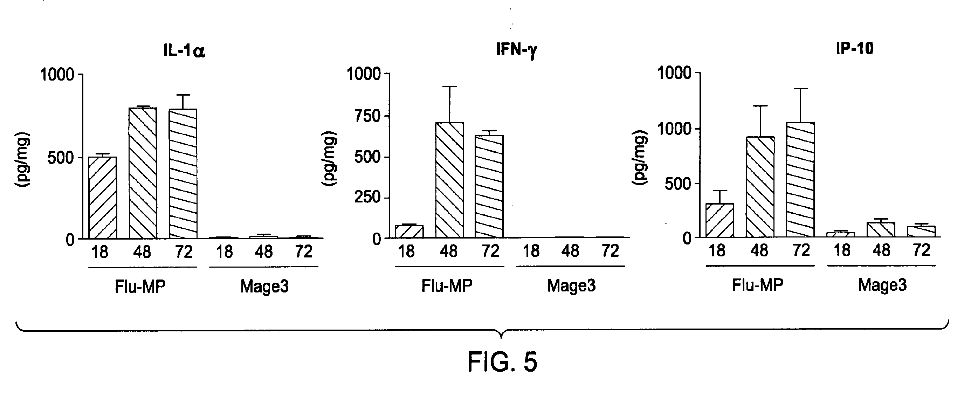

Figure 5 is a graph that depicts the kinetics of the immune response of a normal donor evaluated according the present invention. 2 x 10⁵ fresh PBMCs from an HLA-A0201⁺ donor were stimulated with HLA-A0201 restricted peptides Flu-MP₅₈₋₆₆, or Mage3₂₇₁₋₂₇₉, or diluent in triplicates for the indicated periods. Cytokines in the culture supernatant were measured with multiplex bead-based cytokine assay. Mean±SD are shown from triplicate data. These result shows that the peptide-specific production of IL-1a and IP-10 in EPIMAX assay reached plateau at 48 h of peptide stimulation.

Figure 6 depicts the scheme of epitope focusing according to the present invention. First, 15-mer overlapping peptide libraries encoding antigenic proteins are split into clusters of peptides (5-10 peptides/cluster) in duplicates. Five x 10⁵ PBMCs are first stimulated with each cluster of peptides (Cluster Analysis). Multiple cytokines at culture supernatant are measured with a multiplex bead-based cytokine assay, and a "hit" cluster was determined. In the second culture, Epitope Focusing, PBMCs were cultured with individual peptides from the "hit" cluster for 48 h, and subsequently the antigenic peptide was determined with multiple cytokine analysis.

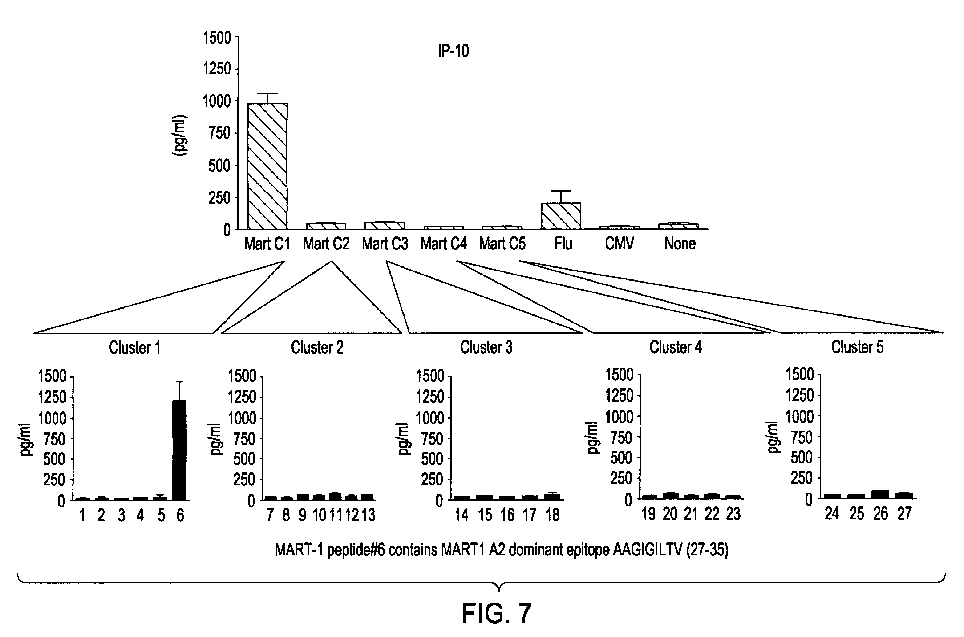

Figure 7 is a graph that depicts the immune response of a melanoma patient as evaluated according the present invention, which allows identification of epitope recognized by specific T cells. PBMCs were obtained from a melanoma patient who received DC vaccines. This patient is HLA-A*0201^{pos}, and was shown to have MART-1 specific CD8+ T cells recognizing HLA-A*0201-restricted dominant epitope (MART-1₂₆₋₃₅) with tetramer binding assay (Figure 4A). 15-mer overlapping peptides with 4 amino acid offsets covering the whole amino acid sequence of MART-1 as either 4-6 clustered peptides (10 µM per peptide) or a single peptide (10 µM) were seeded in triplicates in 96-well plates; and 2 x 10⁵ cells PBMCs in 200 µl CM were added to each well and incubated for 48 hours at 37 degrees C in a humidified 5% CO2 incubator; and the cytokine or chemokine levels in the culture supernatant were measured with Luminex. As shown in Figure 7, MART-1 #6 peptide induces strong production of IP-10 in PBMCs obtained from a vaccinated melanoma patient. MART-1 peptide #6 contains 10-mer HLA-A2 dominant epitope, as depicted in Figure 4A. Thus, the methods of the present invention allow identification of peptide-specific T cell responses.

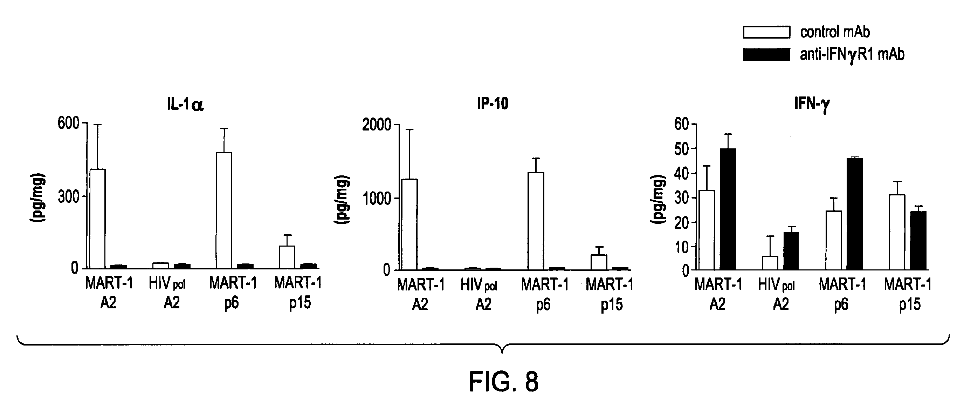

Figure 8 is a graph that depicts the immune response of a melanoma patient as evaluated according the present invention. PBMCs were obtained from a melanoma patient who received DC vaccines. This patient is HLA-A*0201^{pos}, and was shown to have MART-1 specific CD8⁺ T cells recognizing HLA-A*0201-restricted dominant epitope (MART-1₂₆₋₃₅) with tetramer binding assay (Figure 4A). PBMCs were stimulated for 48 h with A2-MART-1 peptide or 15-mer MART-1 peptide#6 in the presence of blocking IFN γR mAb (20 µg/ml) or control mAb. A2-HIVpol peptide and 15-mer MART-1 peptide#15 were used as control peptides. The levels of IL-1a and IP-10 in the culture supernatant are shown. This result indicates that both IL-1a and IP-10 production are dependent on IFN-γ production from peptide-specific T cells, demonstrating that IL-1a and IP-10 are surrogate markers for Type 1 T cells responses, i.e. Th1 and Tc1 cells.

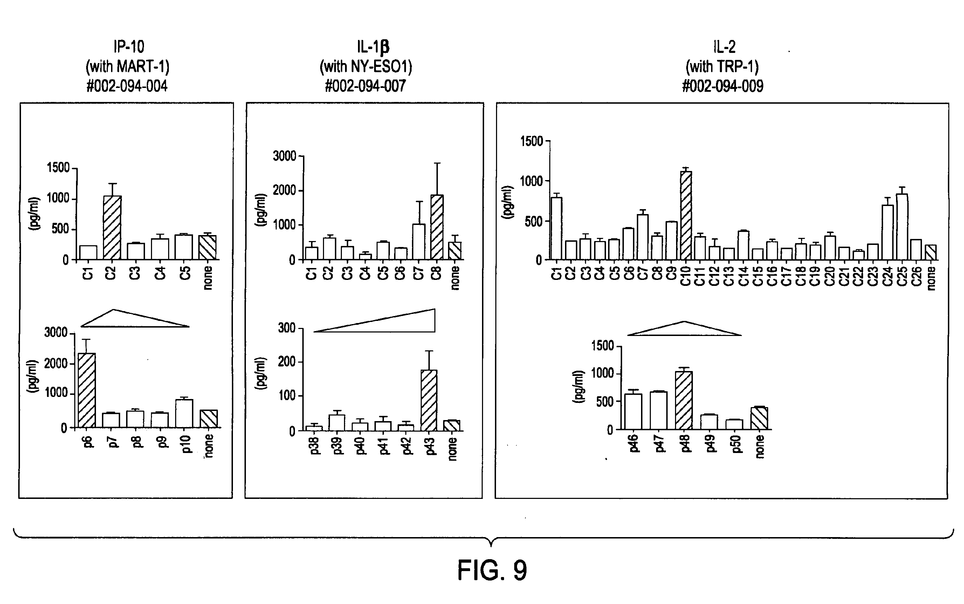

Figure 9 is a graph that depicts the immune response of melanoma patients as evaluated according the present invention. PBMCs obtained from melanoma patients were stimulated for 48 h with either clusters of overlapping peptides or individual peptides encoding melanoma antigens. The levels of cytokines/chemokines in the culture supernatants at 48 hours of cultures were measured with Luminex. This result indicates that the concept of epitope focusing can be applied to any antigens and any cytokines in the method of the present invention.

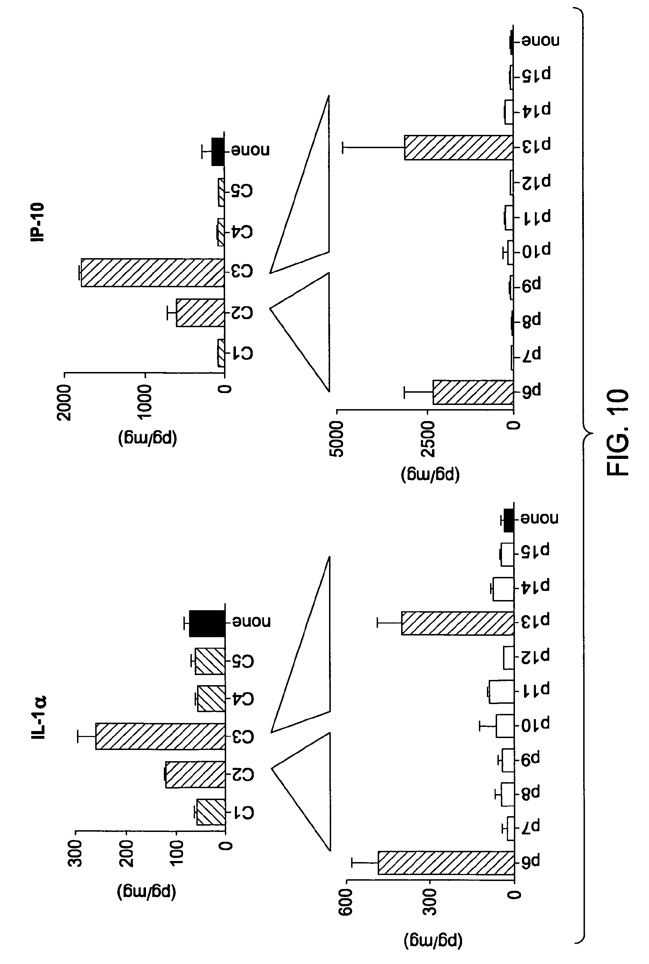

Figure 10 depicts the immune response of a melanoma patient as evaluated according the present invention. PBMCs obtained from a melanoma patient who received DC vaccination series were stimulated with either clusters of overlapping peptides or individual peptides encoding MART-1, a melanoma differentiation antigen. The levels of IL-1a and IP-10 in the culture supernatants at 48 h of culture are shown. With the cluster analysis (Top), cluster #2 and #3 induced upregulation of IL-1a and IP-10. With the epitope focusing (Bottom), peptide#6 from cluster #2 and peptide#13 from cluster #3 were identified as epitopes for specific T cells. This result indicates that the method of the present invention allows identifying epitopes for specific T cells capable of making IFN-γ.

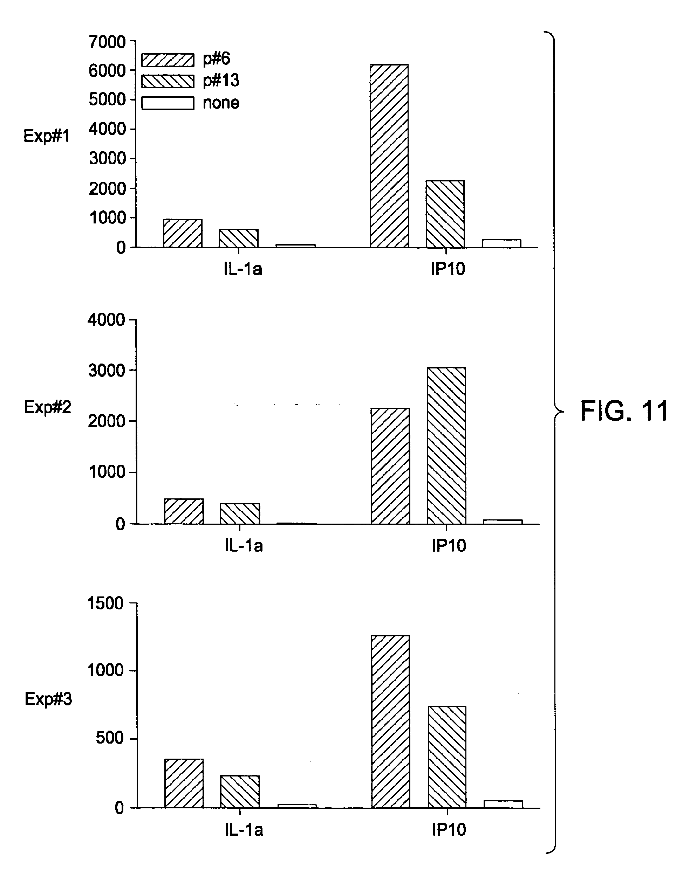

Figure 11 depicts the immune response of a melanoma patient as evaluated according the present invention. PBMCs were obtained from the same patient with Figure 10 with a leukopheresis, made aliquots in cryotubes, and kept in liquid nitrogen until use. Thawed PBMCs were stimulated with MART-1 15-mer peptide#6, peptide#13, or diluent. This experiment was repeated three times separately. The levels of IL-1a a and IP-10 in the culture supernatants at 48 h of culture are shown. Three out of 3 experiments revealed upregulation of IL-1a and IP-10 upon stimulation with peptide#6 or #13, demonstrating a high reproducibility of this EPIMAX assay.

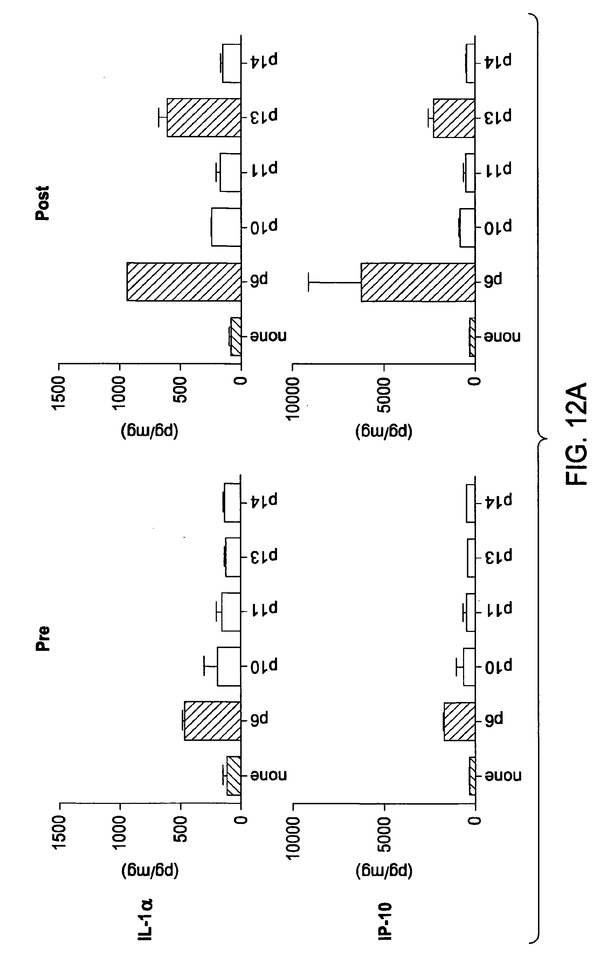

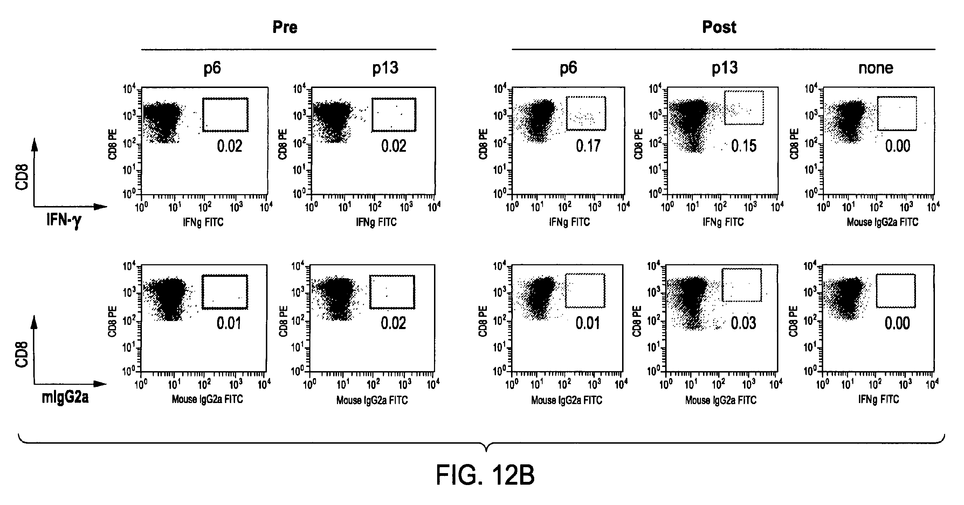

Figures 12A is a graph that depicts the immune response of a melanoma patient as evaluated according the present invention. PBMCs obtained before DC vaccination and post 8 DC vaccinations from a melanoma patient (same as Figure 10) were stimulated with single 15-mer MART-1 peptides or diluent. The levels of IL-la and IP-10 in the culture supernatants at 48 h of culture are shown. While Post-PBMC showed upregulation of IL-1a andIP-10 in response to identified two (2) 15-mer peptides, peptide#6 and #13, Pre-PBMC failed upregulation of IL-1a and IP-10 in response to peptide#13. IL-1a and IP-10 production from Pre-PBMC in response to peptide#6 was less than that from Post-PBMC. These results suggest that DC vaccination induced peptide#13-specific T cell responses and enhanced peptide#6-specific T cell responses. To examine this hypothesis, we used a different well-established methodology, intracellular cytokine detection assay. The same PBMCs were stimulated for 8 h with MART-1 15-mer single peptides in the presence of anti-CD28/CD49d mAb and monensin. After permeabilization of the cell membrane, the cells were stained with anti-IFN-γ mAb and the IFN-γ+ populations were analyzed with a flowcytometer (Figure 12B). While about 0.15% of CD8+ T cells produced IFN-g in response to either peptide#6 or #13 in Post-PBMC, no IFN-g production was detected in Pre-PBMC. These results demonstrate that EPIMAX can be used to monitor antigen-specific T cell responses in cancer patients, and suggest that the magnitude of each specific T cell response is predictable by looking at the magnitude of cytokine/chemokines modulation in EPIMAX.

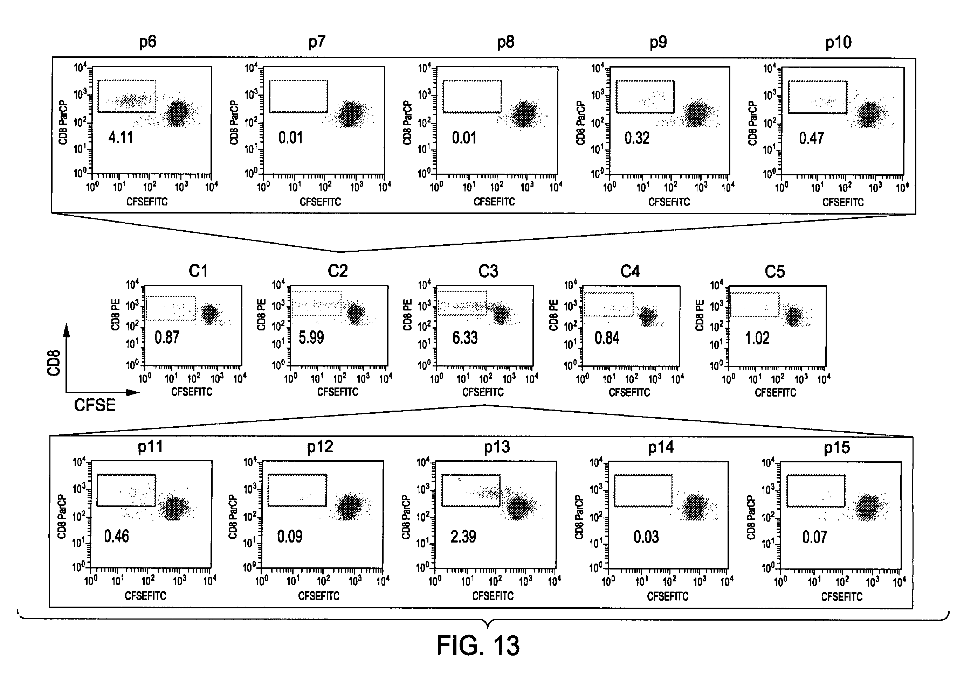

Figure 13 depicts the immune response of a melanoma patient as evaluated according the present invention. CFSE (5,6-carboxyfluorescein diacetate succinimidyl ester) is a tracer dye that permits to identify the number of cell division with a flowcytometer. As EPIMAX is a non-destructive assay, we combined with EPIMAX and CFSE technology to determine the proliferative capacity of antigen-specific T cells. It was confirmed that CFSE staining does not alter the cytokine production in EPIMAX. PBMCs obtained from the same melanoma patient (same as Figure 10) were first stained with 1 uM of CFSE, and stimulated with either clusters of overlapping peptides or individual peptides encoding MART-1. On day 8 of culture, the cells were stained with CD8-PE, CD3-PerCP, and CD4-APC, and analyzed T cell proliferation in response to peptide stimulation. The figure shows the analysis of CD8+ T cell proliferation. MART-1 cluster #2 and #3, and peptide#6 and #13 induced dilution of CFSE intensity in some CD8+ T cell populations, demonstrating that peptide stimulation induced proliferation of antigen-specific CD8+ T cells. This result indicates that combination with other methodology permits to obtain more phenotypical and/or functional characterization of antigen-specific T cells.

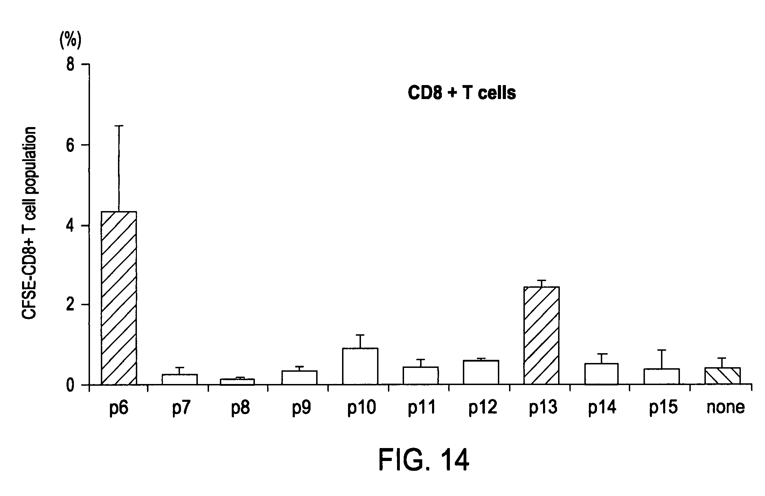

Figure 14 depicts the immune response of a melanoma patient as evaluated according the present invention. PBMCs obtained from the same melanoma patient (same as Figure 10) were first stained with 1 µM of CFSE, and stimulated with individual peptides encoding MART-1 in triplicates. On day 8 of culture, the cells were stained with CD8-PE, CD3-PerCP, and CD4-APC, and analyzed T cell proliferation in response to peptide stimulation. The figure shows the % of CFSE-diluted CD8+ T cell populations within the CD8+ T cell populations. MART-1 peptide#6 and #13 induced dilution of CFSE intensity in some CD8+ T cell populations, demonstrating that peptide stimulation induced proliferation of antigen-specific CD8+ T cells.

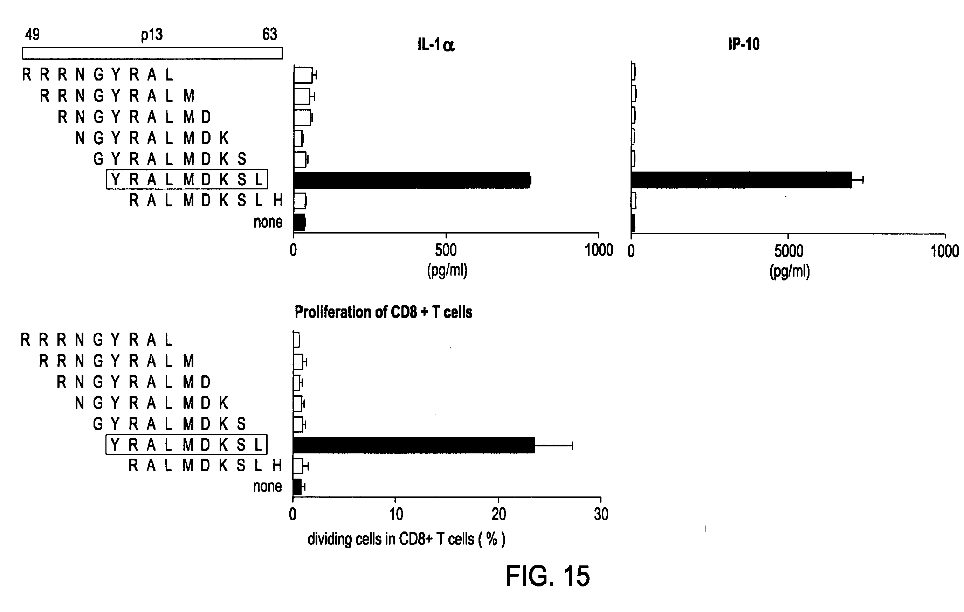

Figure 15 depicts the immune response of a melanoma patient as evaluated according the present invention. CD8⁺ T cells recognize 8-10mer peptide in the context of MHC-class I molecules expressed on antigen-presenting cells. To determine the precise epitope for identified CD8⁺ T cells, overlapping 10mer peptides were generated with 1 amino acid lagging within 15mer MART-1 peptide#13, and used to stimulate the same patient's PBMCs. The levels of IL-1a and IP-10 in the culture supernatant at 48 h, and CFSE dilution assay on day 8 of culture are shown. These results demonstrate that MART-1₅₄₋₆₂ is the epitope for the CD8+ T cells, which allows production of IFN-γ and proliferation of specific CD8+ T cells. Thus EPIMAX permits to identify precise epitopes for CD8+ T cells.

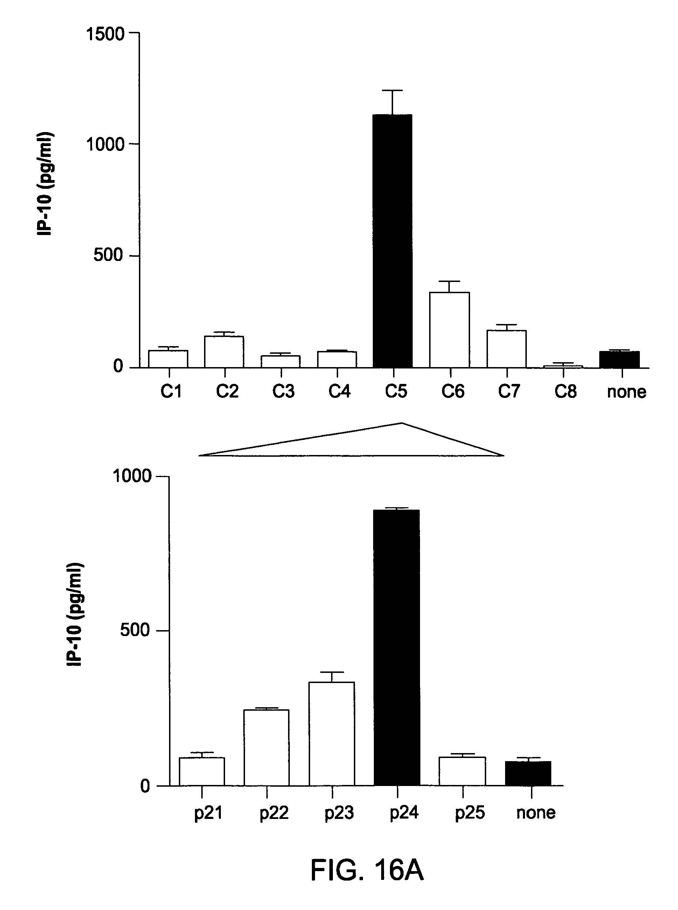

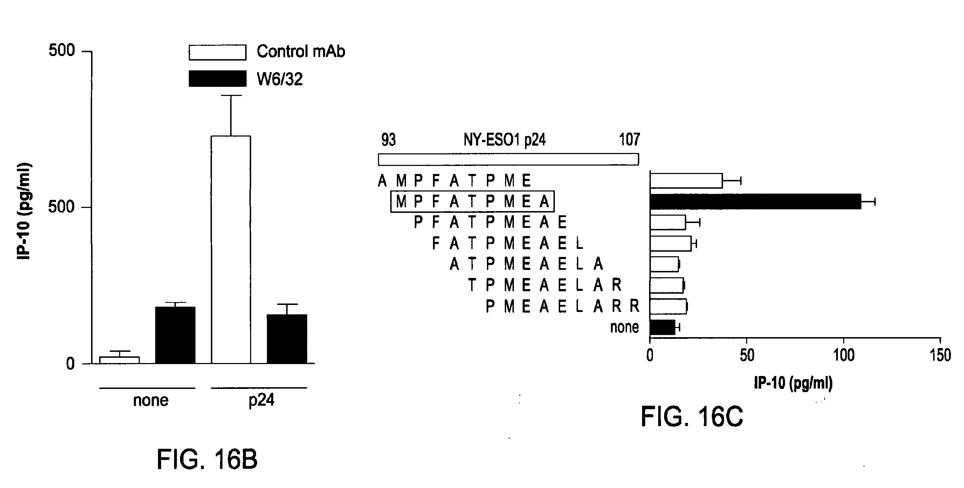

Figures 16A-D depicts the immune response of a melanoma patient as evaluated according the present invention. PBMCs obtained from a HLA-A2^{neg} melanoma patient were stimulated with either clusters of overlapping peptides or individual peptides encoding NY-ESO1, a cancer-testis antigen broadly expressed in most of melanoma cells. NY-ESOl peptide#24 within cluster#5 induced upregulation of IP-10 at 48 h (Figure 16A). These results indicate that NY-ESOl₉₃₋₁₀₇ is recognized as an epitope by specific T cells. To examine which T cell subsets recognize this epitope, the patient's PBMCs were stimulated with NY-ESOl peptide#24 in the presence of anti-MHC class I blocking mAb (W6/32) or isotype control mAb. As shown in Figure 16B, the IP-10 production in response to peptide#24 was completely abrogated by W6/32, indicating that the production of IP-10 is dependent on MHC-class I molecule, thus that CD8⁺ T cells recognize peptide#24.

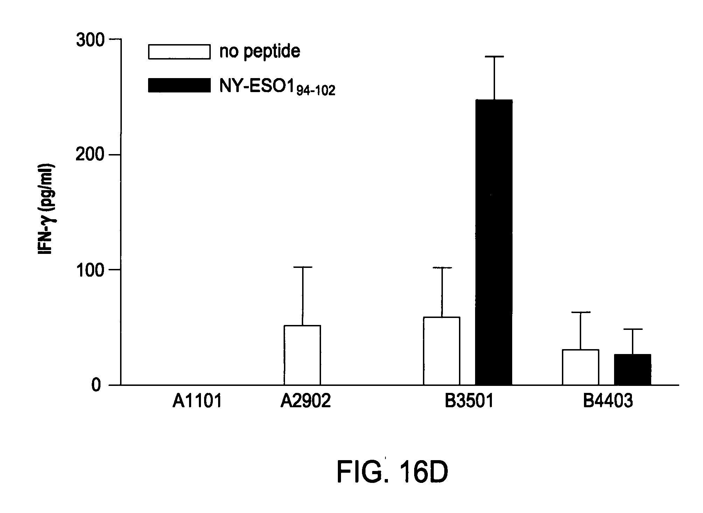

To identify precise epitope for CD8+ T cells, 9mer overlapping peptide within peptide#24 were generated, and used to stimulate the patient's PBMCs. As shown in Figure 16C, NY-ESOl₉₄₋₁₀₂ induced strong upregulation of IP-10, indicating that this is the epitope for CD8+ T cells. To identify HLA-restriction element, the PBMCs were stimulated with autologous monocyte-derived DCs pulsed with NY-ESOl peptide#24 in the presence of IL-2 (10 IU/ml). At day 9 of culture, the cells were re-stimulated with LCLs lacking MHC-class I molecules transfected with expression plasmids encoding each HLA, pulsed or unpulsed with NY-ESOl₉₄₋₁₀₂ peptide. IFN-γ secretion during 8 h stimulation was measured with ELISA. As shown in Figure 16D, HLA-B*3501 transfectant pulsed with NY-ESOl₉₄₋₁₀₂ peptide induced IFN-γ production from T cells, indicating this novel peptide is presented by HLA-B*3501 molecule, These results indicate that EPIMAX allows to identify novel epitopes for CD8+ T cells in HLA-A2^{neg} patients, and combination with other methodologies allows us to identify their HLA restriction elements.

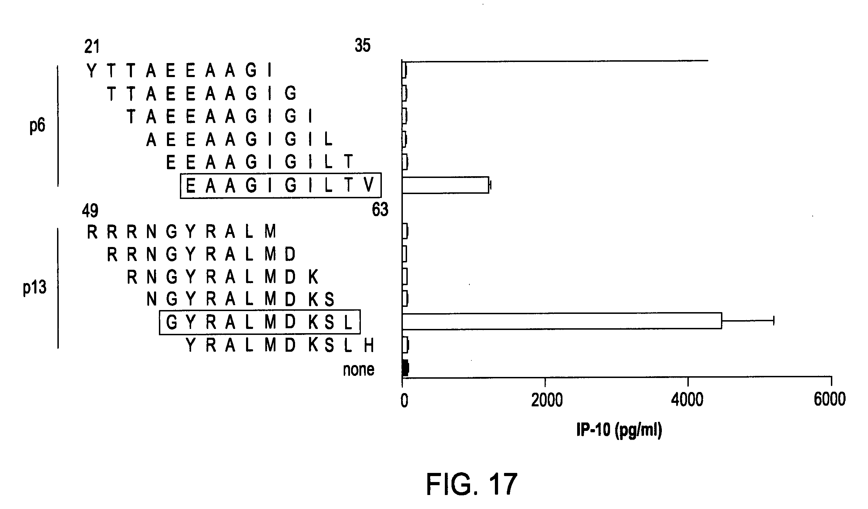

Figure 17 depicts the immune response of a melanoma patient as evaluated according the present invention. The PBMCs obtained from a melanoma patient (same as Figure 10) were stimulated with 1Omer overlapping peptides within MART-1 15-mer peptides #6 and #13 for 48 h. Figure 17 shows the levels of IP-10 in the culture supernatant at 48 hours is shown. MART-1₂₆₋₃₅ (known epitope) and MART-1₅₃₋₆₂ (novel epitope) are the epitopes.

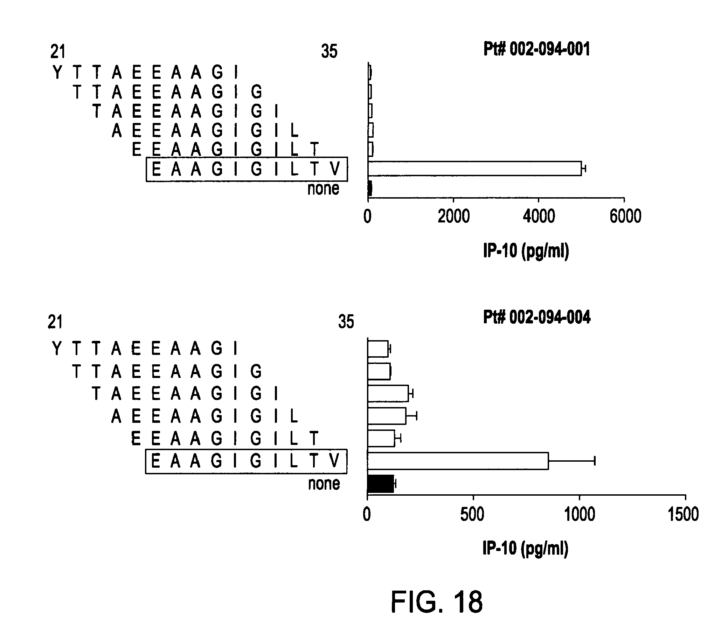

Figure 18 depicts the immune response of melanoma patients as evaluated according the present invention. The PBMCs obtained from two HLA-A2^{neg} melanoma patients were stimulated with 10mer overlapping peptides within MART-1 15-mer peptides #6 for 48 hours. The levels of IP-10 in the culture supernatant at 48 hours are shown. MART-1₂₆₋₃₅ (known epitope) is the epitopes for the both patients. This epitope is known as restricted to HLA-A2. As these patients are HLA-A2^{neg}, and the HLA-class I subtypes are completely different between these two patients, this epitope is presented by at least three different MHC class I molecules. This is another example of identification of epitopes for CD8+ T cells with EPIMAX.

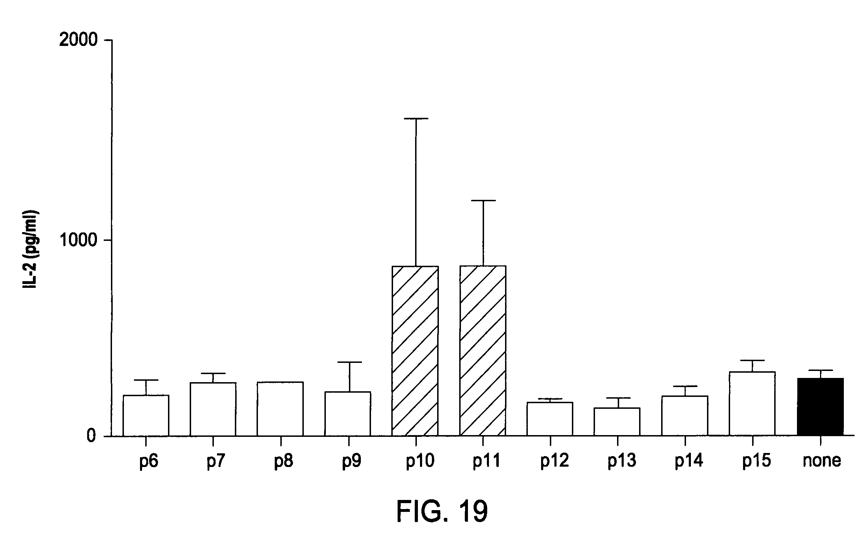

Figure 19 depicts the immune response of a melanoma patient as evaluated according the present invention. The PBMCs obtained from a melanoma patient (same as Figure 10) were stimulated in triplicates with individual MART-1 15-mer peptides from #6 to #15 for 48 h. The levels of IL-2 in the culture supernatant at 48 hours are shown. MART-1 peptides#10 and #11 induced upregulation of IL-2. This result demonstrates another example of epitope identification with EPIMAX.

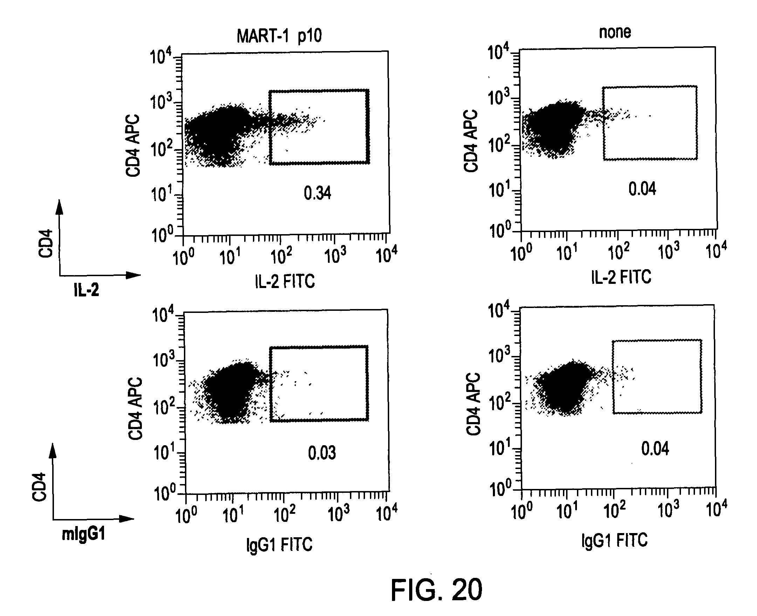

Figure 20 depicts IL-2 production from CD4⁺ T cells upon stimulation with the identified 15-mer peptide as shown in Figure 19. The same patient's PBMCs were stimulated for 8 hours with MART-1 15-mer peptide#10 in the presence of antiCD28/CD49d mAb and monensin. The production of IL-2 were analyzed by staining with specific mAbs. The figure demonstrates the CD4+ T cell populations producing IL-2 in response to MART-1 peptide#10. These results indicate that EPIMAX can also identify epitopes for CD4⁺ T cells.

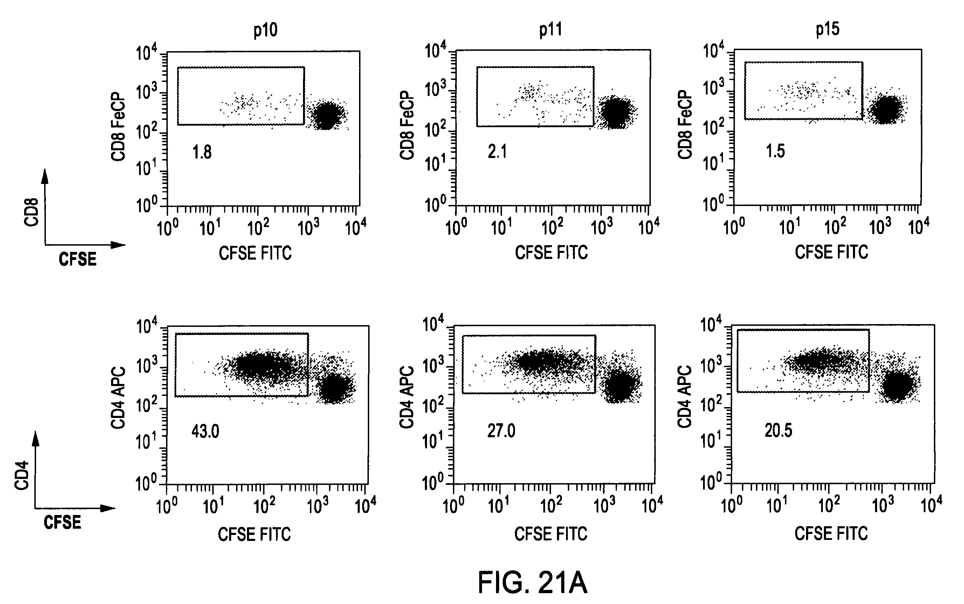

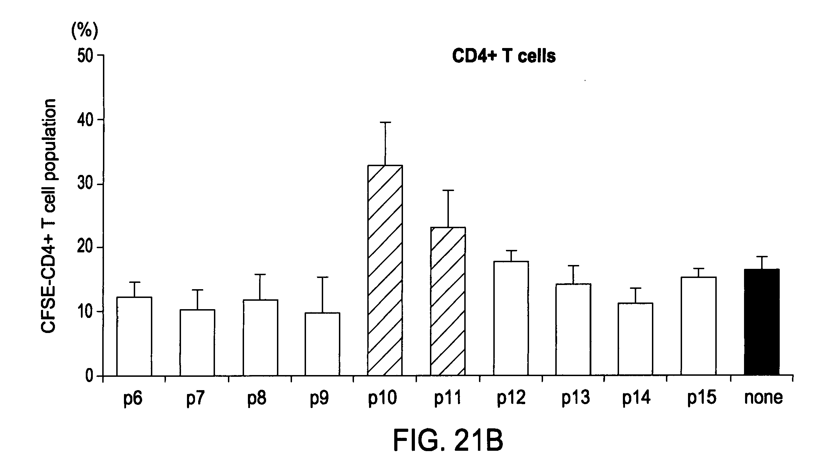

Figures 21A-B depicts the immune response of a melanoma patient as evaluated according the present invention. PBMCs obtained from the same melanoma patient (same as Figure 19) were first stained with 1 µM of CFSE, and stimulated with individual peptides encoding MART-1 in triplicates. On day 8 of culture, the cells were stained with CD8-PE, CD3-PerCP, and CD4-APC, and analyzed T cell proliferation in response to peptide stimulation. The figure 21A shows the % of CFSE-diluted CD4+ T cell populations within the CD4⁺ T cell populations. MART-1 peptide#10 and #11 induced more CFSE-diluted CD4⁺ T cell populations than no peptide, demonstrating that these peptides induced proliferation of antigen-specific CD4⁺ T cells. Figure 21B depicts the CFSE-diluted CD4+ T cell populations in response to each MART-1 15-mer peptide within the CD4⁺ T cell population. MART-1 peptide#10 and #11 induced significantly more proliferation of CD4⁺ T cells than the other 15-mer peptides. These results strongly indicates that EPIMAX allows identification of epitopes for CD4⁺ T cells, and permits to determine the proliferative capacity of specific CD4⁺ T cells by combining with the CFSE technology.

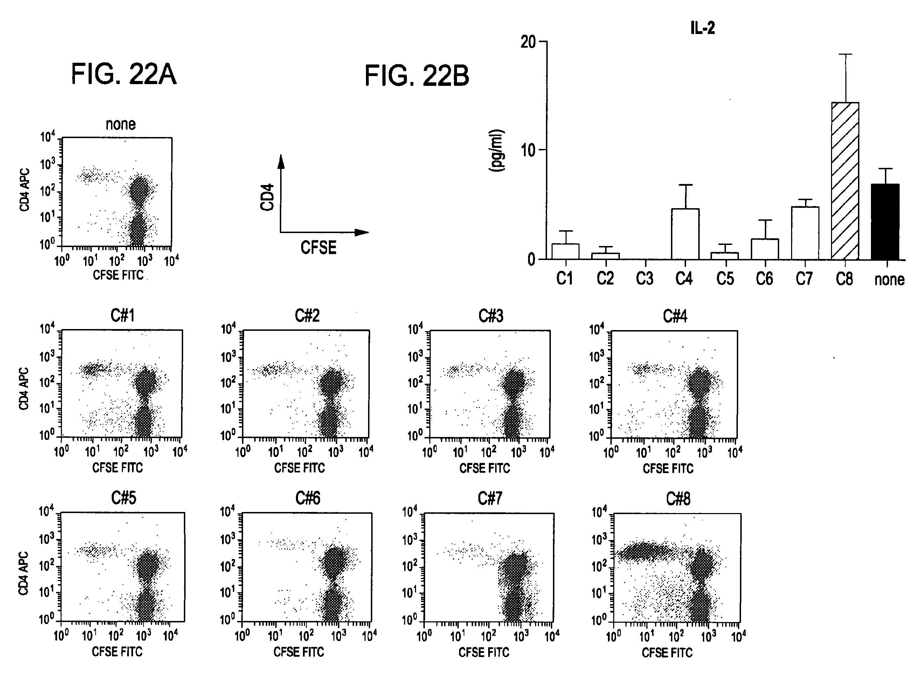

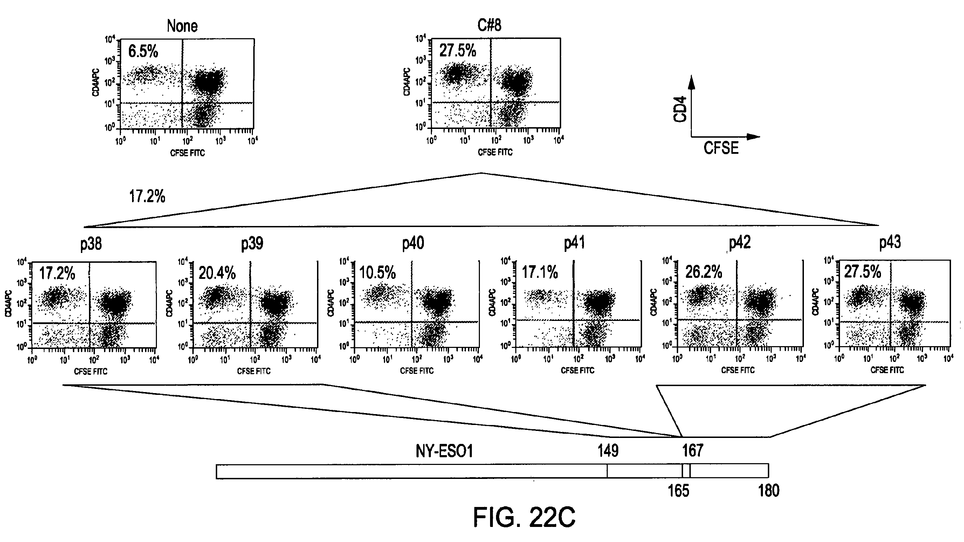

Figures 22 A-C depicts the immune response of a melanoma patient as evaluated according the present invention. PBMCs obtained from a HLA-A2^{neg} melanoma patient who received DC vaccinations were first stained with 1 µM of CFSE, and stimulated with clusters of peptides encoding NY-ESOl. On day 8 of culture, the cells were stained with CD8-PE, CD3-PerCP, and CD4-APC, and analyzed T cell proliferation in response to peptide stimulation was analyzed based on CFSE dilution with a flowcytometer. Figure 22A shows the percent (%) of CFSE-diluted CD4⁺ T cell populations within the CD4+ T cell populations. NY-ESOl cluster#8 induced more CFSE-diluted CD4⁺ T cell populations than the other clusters, demonstrating that these peptides induced proliferation of antigen-specific CD4+ T cells. Figure 22B depicts the IL-2 levels in the culture supernatant at 48 hours of culture. IL-2 was produced in the culture with NY-ESO1 cluster#8. To identify the epitope for CD4+ T cells, CFSE-stained PBMCs were stimulated with individual peptides within NY-ESOl cluster#8. The proliferating CD4+ T cells were analyzed based on CFSE dilution with a flowcytometer on day 8. As shown in Figure 22C, NY-ESOl₁₄₉₋₁₆₇ and NY-ESOl₁₆₅₋₁₈₀ induced more proliferation of CD4⁺ T cells, demonstrating that these are the epitopes. This result indicates another example of identification of CD4⁺ T cell epitopes in melanoma antigens.

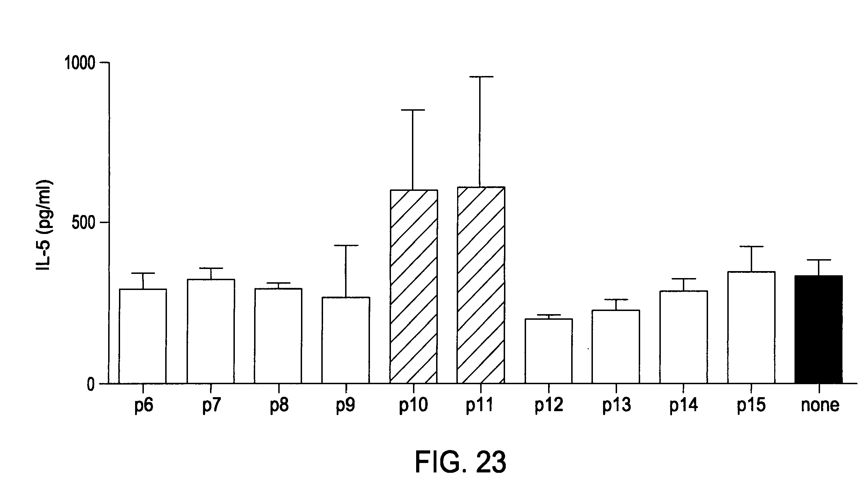

Figure 23 depicts the immune response of a melanoma patient as evaluated according the present invention. The PBMCs obtained from a melanoma patient (same as Figure 10) were stimulated in triplicates with individual MART-1 15-mer peptides from #6 to #15 for 48 h. The levels of IL-5 in the culture supernatant at 48 hours are shown. MART-1 peptides#10 and #11 induced upregulation of IL-5. This result demonstrates another example of epitope identification with EPIMAX.

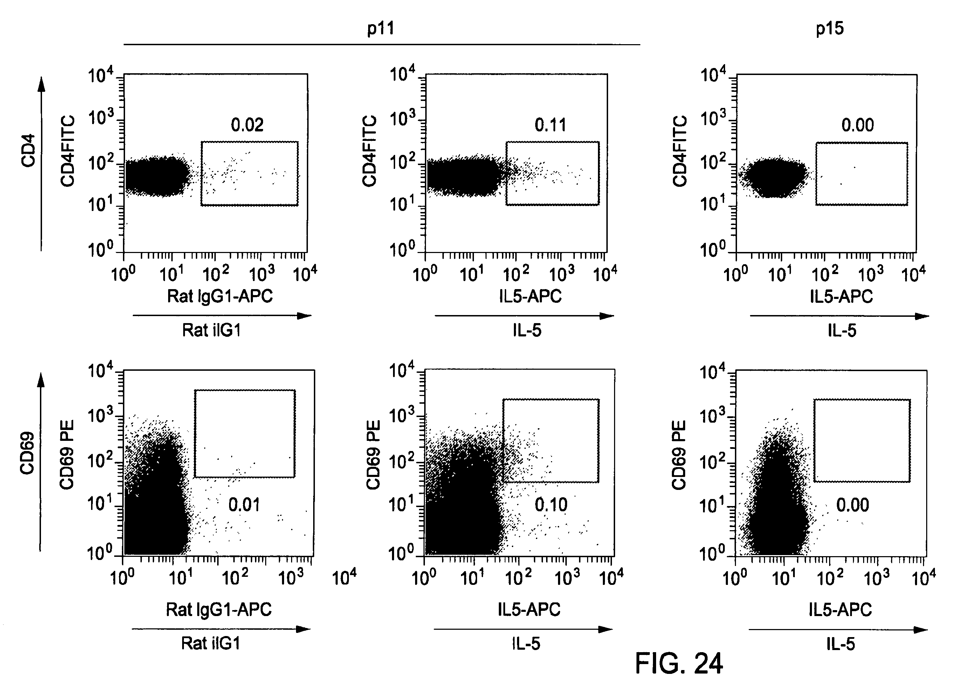

Figure 24 depicts IL-5 production from CD4⁺ T cells upon stimulation with the identified 15-mer peptide as shown in Figure 23. The same patient's PBMCs were stimulated for 8 hours with MART-1 15-mer peptide#11 in the presence of antiCD28/CD49d mAb and monensin. The production of IL-5 were analyzed by staining with specific mAbs. The figure demonstrates the CD4⁺ T cell populations producing IL-5 in response to MART-1 peptide#11. This result indicates EPIMAX can identify epitopes as well as type of cytokines produced by specific CD4⁺ T cells.

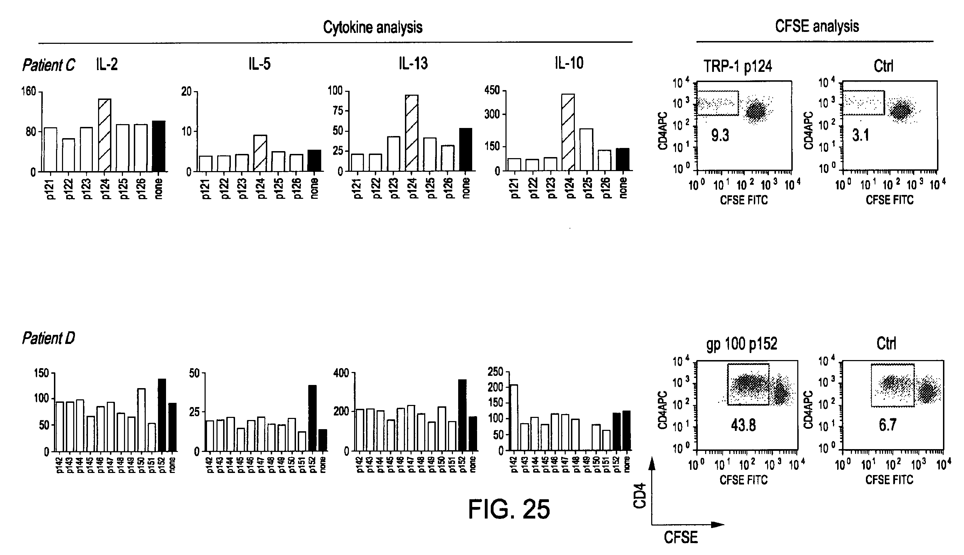

Figure 25 depicts the immune response of melanoma patients as evaluated according the present invention. CFSE-stained PBMCs obtained from two melanoma patients were stimulated with individual 15-mer peptides either from TRP-1 cluster#25 or gp100 cluster#15. The cytokines in the culture supernatants were measured at 48 h, and the proliferation of T cells were analyzed on day 8 of culture based on CFSE dilution assay. The results indicate that TRP-1 peptide#124 and gp100 peptide#152 are epitopes for CD4⁺ T cells, both of which are novel epitopes for CD4⁺ T cells. Peptide stimulation induced various effector cytokine production, such as IL-2, IL-5, IL-13, and IP-10 (IFN-γ), and proliferation of specific CD4⁺ T cells. These results indicate another example of identification of CD4⁺ T cell epitopes in melanoma antigens, and suggest that EPIMAX can be used to identify different type of T cell responses.

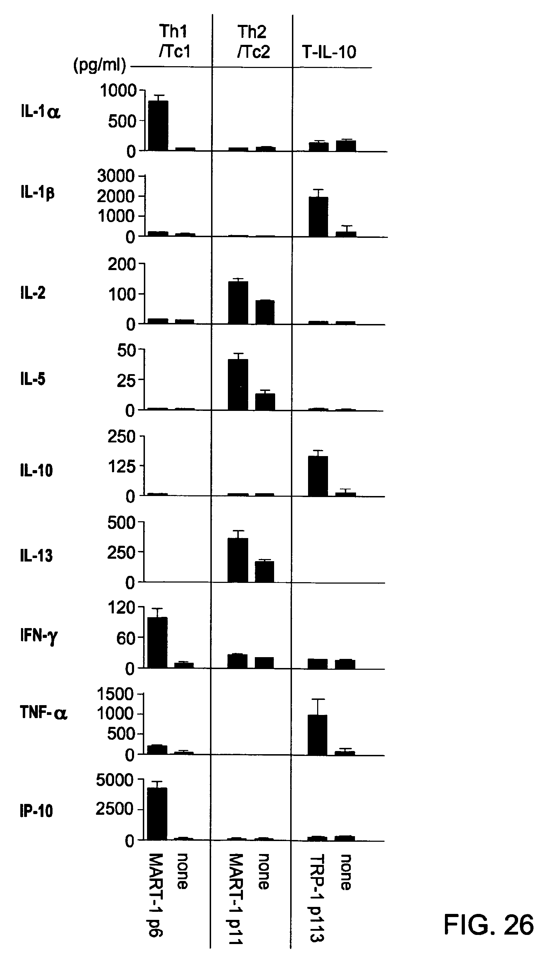

Figure 26 depicts the immune response of three melanoma patients as evaluated according the present invention. EPIMAX permits to identify different T cell subsets, such as Type1 (Th1, and Tc1), Type 2 (Th2 and Tc2) cells. PBMCs obtained from patients with metastatic melanoma were stimulated with newly-identified melanoma peptides (15-mers) for 48 h, and cytokines in the culture supernatants were measured with a multiplex bead-based assay. Colored columns represent the levels of cytokines with indicated peptides (bottom), and open columns represent those with diluent. The representative cytokine production for three different T cell subsets, Type 1, 2, and Type-IL-10 are shown. Type 1 response is identified by upregulation of IL-1a and IP-10, while Type 2 response is identified by upregulation of type 2 cytokines, such as IL-4, IL-5, and IL-13 without upregulation of IP-10. EPIMAX was used to identify a novel T cell response characterized by upregulation of IL-10 (Type IL-10).

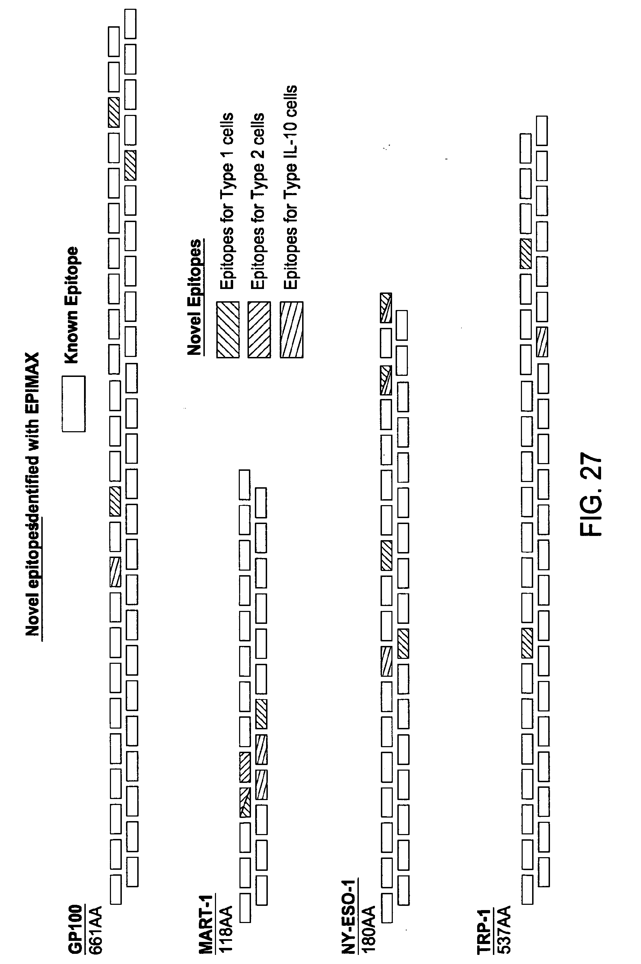

Figure 27 depicts a summary of new epitopes for CD4⁺ and CD8⁺ T cells within 4 melanoma antigens, i.e., gp100, MART-1, NY-ESO1, and TRP-1, which are identified with EPIMAX methodology. These new epitopes were identified by screening 13 melanoma patients PBMCs with EPIMAX.

**Table 1 depicts the summary of epitopes for CD8⁺ T cells identified with EPIMAX. The identified 15-mer peptides and their amino acid sequence number are shown. Some 15-mer peptides were focused either with 9-mer or 10-mer overlapping peptides, and shown in the table.**

| Patient | Pre or Post | peptide library | identified epitopes | epitope sequence | Precise epitope | Type of response |
|---|---|---|---|---|---|---|
| | Pre | MART-1 | p6 | MART-1₂₁₋₃₅ | MART-1₂₆₋₃₅ | 1 |
| | Post | MART-1 | p6 | MART-1₂₁₋₃₅ | MART-1₂₆₋₃₅ | 1 |
| 094-001 | Pre | NY-ESO1 | p24 | NY-ESO1₉₃₋₁₀₇ | NY-ESO1₉₄₋₁₀₂ | 1 |
| | Post | NY-ESO1 | p24 | NY-ESO1₉₃₋₁₀₇ | NY-ESO1₉₄₋₁₀₂ | 1 |
| | Pre | gp100 | p60 | gp100₂₃₇₋₂₅₁ | | 1 |
| 094-004 | Pre | MART-1 | p6 | MART-12₁₋₃₅ | MART-1₂₆₋₃₅ | 1 |
| | Post | MART-1 | p6 | MART-1₂₁₋₃₅ | MART-1₂₆₋₃₅ | 1 |
| 094-007 | Pre | NY-ESO1 | p39 | NY-ESO1₁₅₃₋₁₆₇ | | 1 |
| | Post | NY-ESO1 | p39 | NY-ESO1₁₅₃₋₁₆₇ | | 1 |
| | Post | MART-1 | p6 | MART-1₂₁₋₃₅ | MART-1₂₆₋₃₅ | 1 |
| 094-009 | Post | MART-1 | p13 | MART-1₄₉₋₆₃ | MART-1_{53-62, 54-62} | 1 |
| | Post | gp100 | p52 | gp100₂₀₅₋₂₁₉ | gp100₂₀₈₋₂₁₇ | 1 |
| 094-010 | Post | NY-ESO1 | p21 | NY-ESO1₈₁₋₉₅ | | 10 |
| | Post | gp100 | p140 | gp100₅₅₇₋₅₇₁ | | 1 |
| 094-012 | Post | NY-ESO1 | p31 | NY-ESO1₁₅₃₋₁₆₇ | | 1 |
| | Post | NY-ESO1 | p38 | NY-ESO1₁₄₉₋₁₆₃ | | 0 |
| | Post | NY-ESO1 | p40 | NY-ESO1₁₅₇₋₁₇₁ | NY-ESO1₁₆₀₋₁₆₉ | 0 |
| 094-015 | Post | NY-ESO1 | p41 | NY-ESO1₁₆₁₋₁₇₅ | NY-ESO1₁₆₁₋₁₇₀ | 1/10 |
| | Post | gp100 | p73 | gp100₂₈₉₋₃₀₃ | | 10 |

**Table 2 depicts the summary of epitopes for Th0, Th1, and Th2 cells identified with EPIMAX. The identified 15-mer peptides and their amino acid sequence number are shown.**

| Patient | Pre or Post | peptide library | identified epitopes | epitope sequence | Type of response | proliferation |
|---|---|---|---|---|---|---|
| | | NY-ESO1 | p38/p39 | NY-ESO1₁₄₉₋₁₆₇ | 0 | Yes |
| 094-004 | Post | NY-ESO1 | p42/p43 | NY-ESO1₁₆₅₋₁₈₃ | 0 | Yes |
| | | TRP-1 | p118 | TRP-1₄₆₉₋₄₈₃ | 1 | Yes |
| | | TRP-1 | p124 | TRP-1₄₉₃₋₅₀₇ | 1 | Yes |
| | | MART-1 | p10/p11 | MART-1₃₇₋₅₅ | 2 | Yes |
| 094-009 | Post | gp100 | p152 | gp100₆₀₅₋₆₁₉ | 0 | Yes |
| | | TRP-1 | p48 | TRP-1₁₈₉₋₂₀₃ | 0 | Yes |
| 094-012 | Post | gp100 | p32 | gp100₁₂₅₋₁₃₉ | 2 | Yes |
| | | gp100 | p40 | gyp100₁₅₇₋₁₇₁ | 2 | Yes |
| | | gp100 | p71 | gp100₂₈₁₋₂₉₅ | 2 | Yes |
| | | gp100 | p78 | gp100₃₀₉₋₃₂₃ | 2 | Yes |
| | | gp100 | p152 | gp100₆₀₅₋₆₁₉ | 2 | Yes |
| | | gp100 | p1/p2 | gp100₁₋₁₈ | 0 | Yes |
| 094-015 | Post | gp100 | p152 | gp100₆₀₅₋₆₁₉ | 0 | Yes |
| | | MART-1 | p11 | MART-1₄₁₋₅₅ | 0 | Yes (CD4/CD8) |

**Table 3 depicts the summary of epitopes for Type-IL-10 CD4+ T cells identified with EPIMAX. The identified 15-mer peptides and their amino acid sequence number are shown.**

| Patient | Pre or Post | peptide library | identified epitopes | epitope sequence | Type of response | proliferation |
|---|---|---|---|---|---|---|
| | Pre | MART-1 | p10 | MART-1₃₇₋₅₁ | 10 | No |
| 094-004 | Post | MART-1 | p10 | MART-1₃₇₋₅₁ | 10 | No |
| | Pre | TRP-1 | p113 | TRP-1₄₄₉₋₄₆₃ | 10 | No |
| 094-005 | Pre | gp100 | p93 | gp100₃₆₉₋₃₈₄ | 10 | No |
| 094-006 | Pre | MART-1 | p8 | MART-1₃₇₋₅₁ | 10 | No |
| 094-007 | Pre | NY-ESO1 | p43 | NY-ESO₁₆₉₋₁₈₃ | 10 | No |
| | Pre | NY-ESO1 | p39/p40 | NY-ESO1₁₆₉₋₁₈₃ | 10 | Marginal |
| | Pre | TRP-1 | p50 | TRP-1₁₉₇₋₂₁₁ | 10 | Marginal |
| 094-010 | Post | NY-ESO1 | p23 | NY-ESO1₈₉₋₁₀₃ | 10 | Yes |
| 094-*013 | Post | NY-ESO1 | p23 | NY-ESO1₈₉₋₁₀₃ | 10 | |

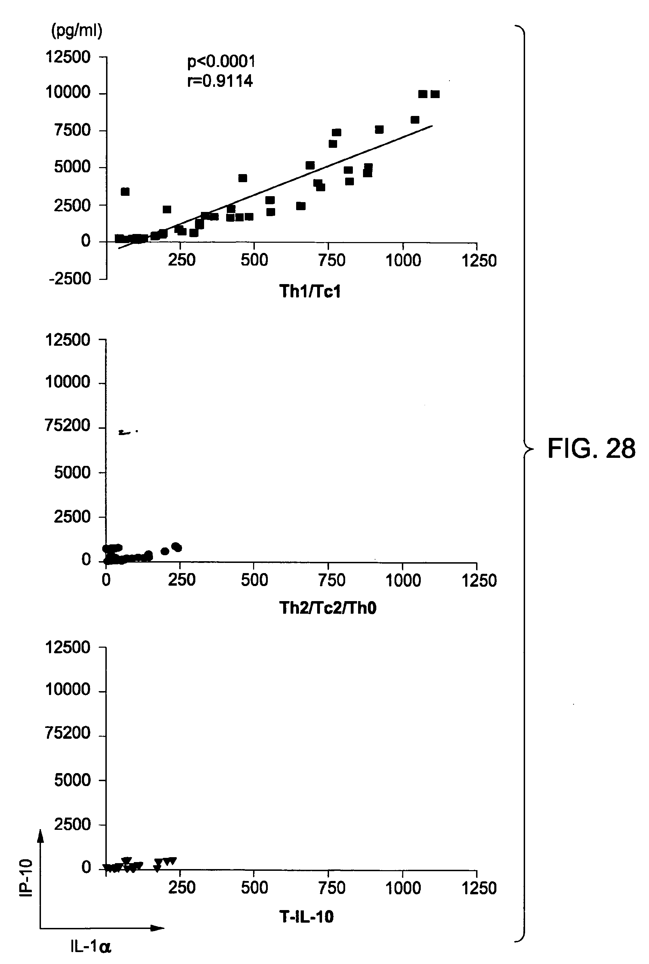

Figure 28 are graphs that depict the immune response of three melanoma patients as evaluated according the present invention. EPIMAX analysis of PBMCs obtained from patients with metastatic melanoma (13 patients) with MART-1, gp100, NY-ESO1, and TRP-1 overlapping peptides yielded 16 peptides inducing Type 1 responses, 15 peptides inducing Type 0/2 responses, and 9 peptides inducing Type-IL-10. The cytokines were measured at day 2 of cultures. IL-1α and IP-10 levels in each T cell subsets are shown respectively. Each dot represents cytokine levels in each separate well from these patients' PBMC culture stimulated with identified single peptides. This result clearly indicates that IL-1α and IP-10 completely correlate in the Type 1 response.

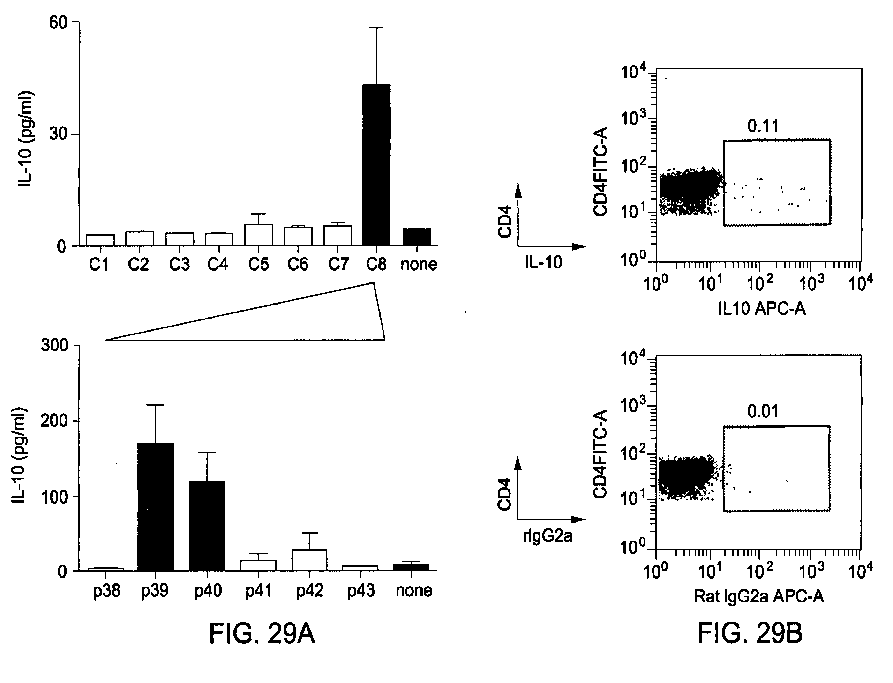

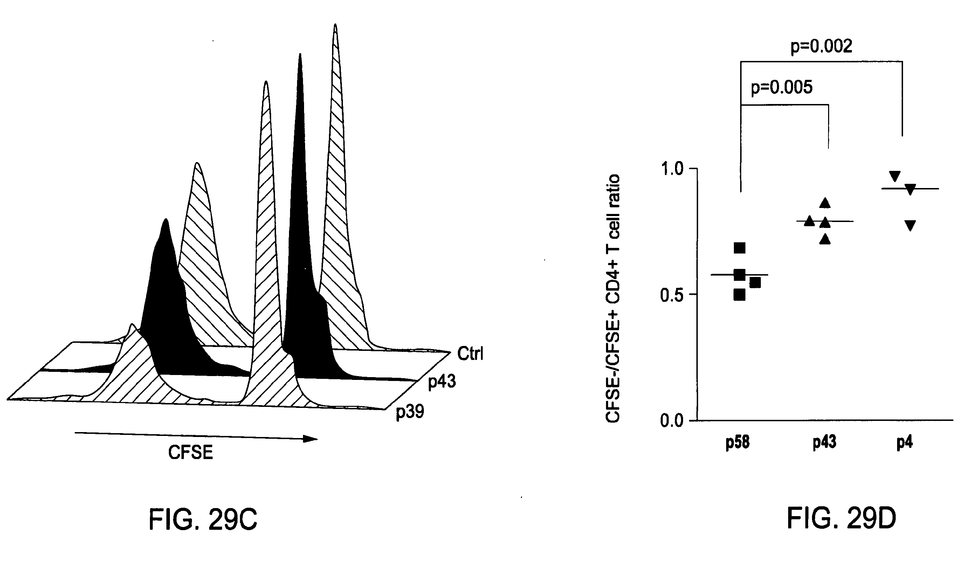

Figures 29A-D are graphs that depict the immune response of a melanoma patients as evaluated according the present invention. PBMCs obtained from a melanoma patient were stimulated with clusters of peptides or individual peptides encoding NY-ESO1. IL-10 levels in culture supernatant at 48 hours of culture are shown (Figure 29A). NY-ESO1 peptide#39 and #40 are the epitopes. To explore the origin of IL-10 production, the PBMCs were stimulated with peptide#39 in the presence of anti CD28/CD49d mAbs and monensin for 8 hours, and stained with IL-10 specific mAb. As shown in Figure 29B, about 0.11% of CD4+ T cells produced IL-10 in response to NY-ESO1 peptide#39. This result indicates that EPIMAX permits IL-10 producing melanoma-antigen specific CD4⁺ T cells in melanoma patients. Next, to evaluate whether this IL-10 producing T cells had regulatory properties, PBMCs stimulated with peptide #39 or irrelevant 15-mer peptide were added in transwells to a "third party" MLR to test suppression of T cell expansion by the release of soluble mediators. The MLR used CFSE-labeled CD4⁺T cells and allogeneic mature DCs, both from healthy volunteers, in order to avoid the effect of peptide-specific T cell response. The patient's PBMC stimulated with NY-ESO1 peptide #39, but not with control peptide, significantly suppressed proliferation in the MLR (Figures 29C, 29D). These results indicate EPIMAX approach can identify tumor-antigen specific regulatory T cells.

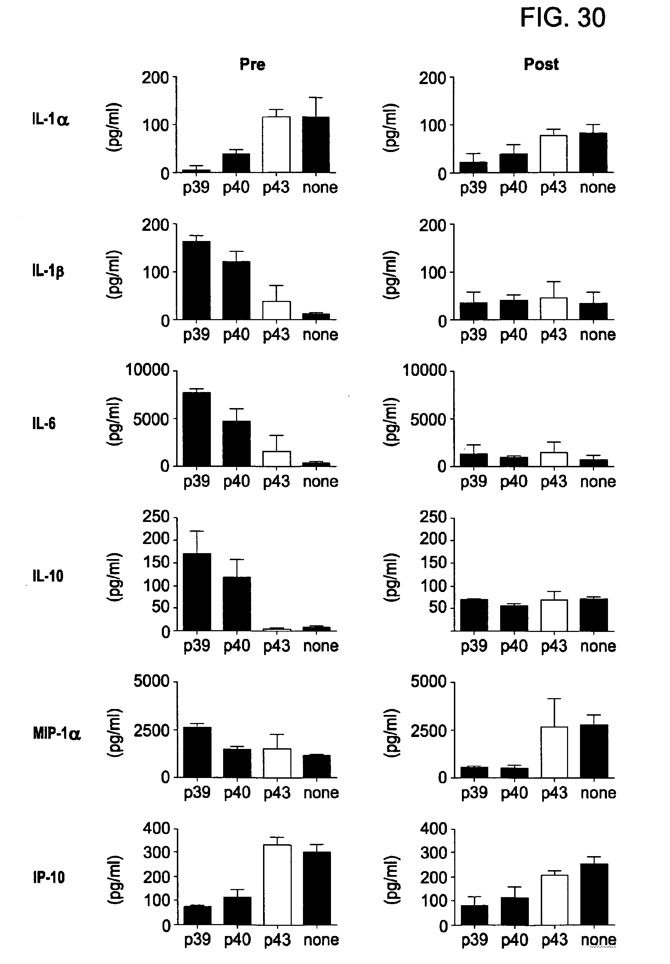

Figure 30 are graphs that depict the immune response of a melanoma patient as evaluated according the present invention. PBMCs obtained from the same melanoma patient as in Figures 29A-D at two different time points, i.e., Pre DC vaccination and Post 8 DC vaccination were stimulated with individual peptides encoding NY-ESO1. The levels of cytokine/chemokines in culture supernatant at 48 h of culture are shown. This result demonstrates that the signature of Type-IL-10 disappeared at Post vaccine, suggesting the function of IL-10 producing CD4⁺ T cells was lost at Post vaccine. These results demonstrate another example of modulation of T cell responses by DC vaccine with EPIMAX assay.

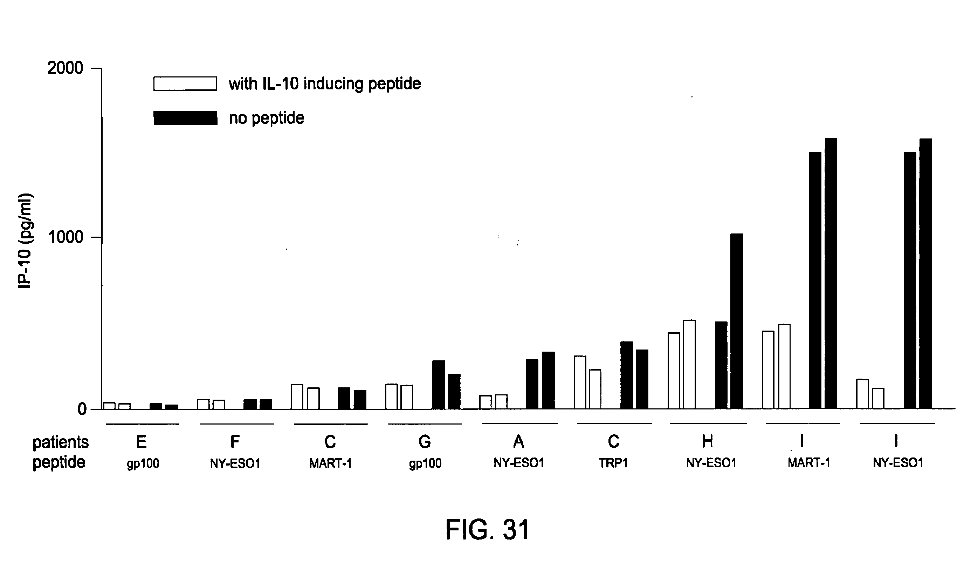

Figure 31 is a graph that depicts the immune response of melanoma patients as evaluated according the present invention. We have identified 9 different epitopes in melanoma antigens inducing Type-IL-10 responses in 7 melanoma patients. PBMCs obtained from patients with melanoma were incubated with each patient-specific identified 15-mer peptide for 48h. The figure shows the inhibition of spontaneous IP-10 production by incubation with identified Type-IL-10 peptides. This is another demonstration of suppressive function of IL-10 producing CD4⁺ T cells identified with EPIMAX.

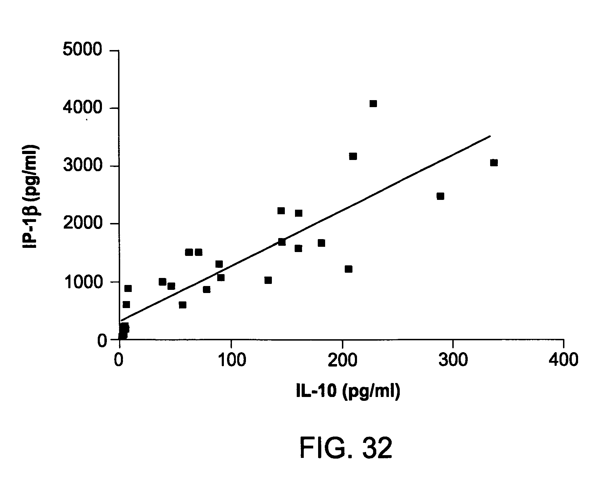

Figure 32 is a graph that depicts the immune response of melanoma patients as evaluated according the present invention. IL-1β and IP-10 levels in each PBMC culture with identified IL-10 inducing 15-mer peptides are shown. Each dot represents cytokine levels in each separate well in PBMC culture with the identified unique single 15-mer peptides. This result shows that IL-1β and IL-10 correlate in identified Type-IL-10 responses, thus both cytokines can be a good marker to identify Type-IL-10 cells in EPIMAX.

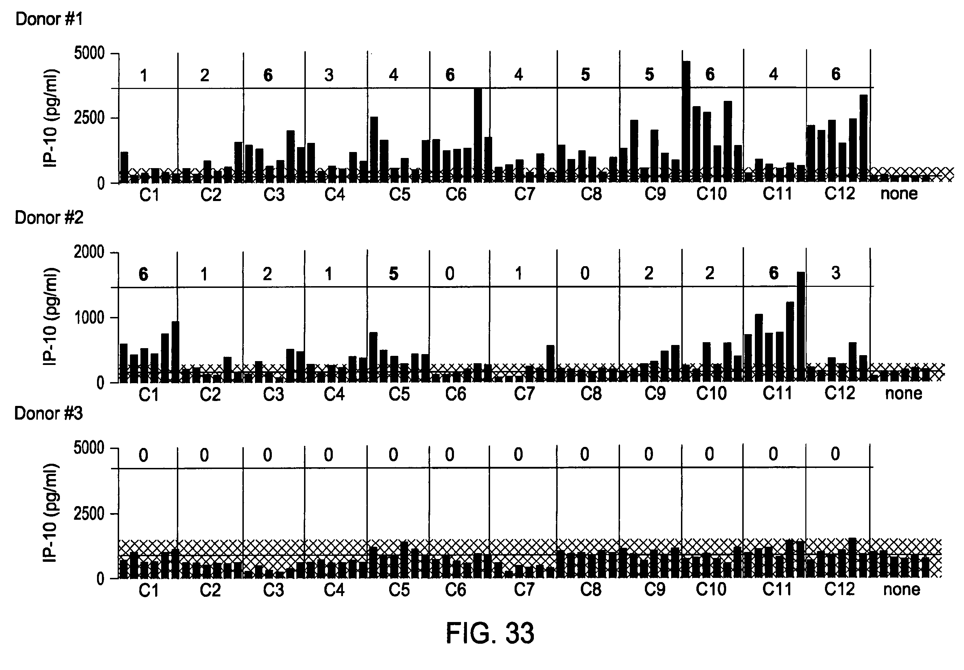

Figure 33 are graphs that depict the immune response of normal healthy volunteers as evaluated according the present invention. The fresh PBMCs obtained from three normal healthy volunteers were split into 3 batches. PBMCs from each batch were stimulated with clusters of 15-mer overlapping peptides encoding influenza virus matrix protein (Flu-MP) in duplicates (2 x 10⁵ cells/well, 10 µM per peptide), thus created 6-plicates of samples. The levels of IP-10 at 48 h of culture are shown. The black bars and shades represent the mean±3SD value of IP-10 in the cultures with no peptides. The levels of IP-10 were determined above mean+3SD of this background are positive induction of IP-10 in response to peptide clusters. In normal donor#1, 9 out of 12 peptide clusters scored positive more than 4 out of 6-plicates of samples, indicating that this donor had very wide repertoire of T cells specific to Flu-MP. In contrast, normal donor#3, no clusters induced upregulation of IP-10. EPIMAX allows identification of the full breadth of T cell responses against a given antigenic protein. Furthermore, this studies shows that EPIMAX is highly reproducible assay.

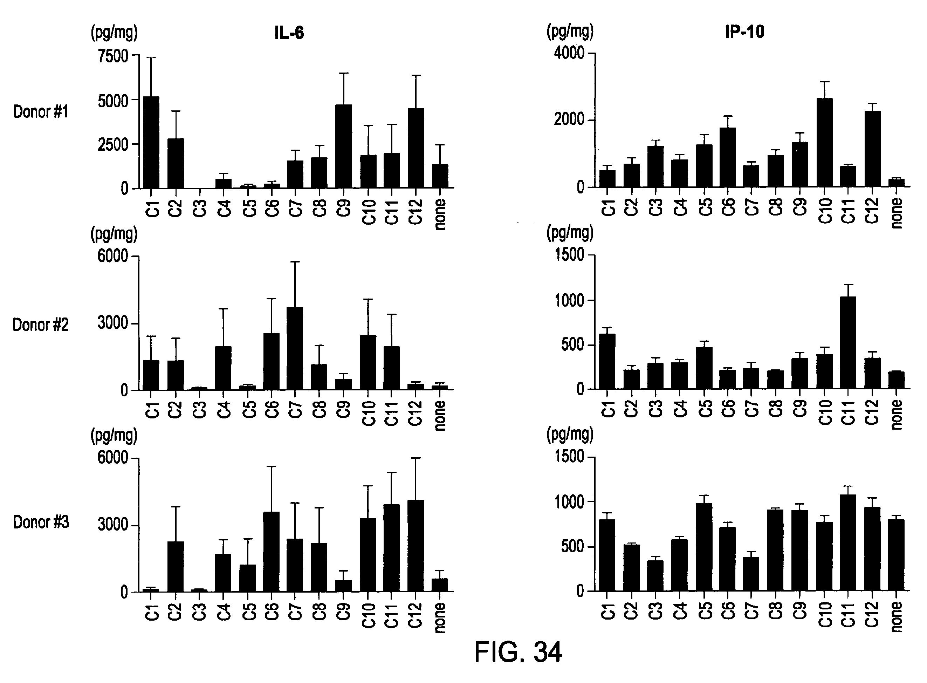

Figure 34 are graphs that depict the immune response of normal healthy volunteers as evaluated according the present invention. The levels of IL-6 and IP-10 in the same studies shown in Figure 33 were shown. Strikingly, in normal donor#3, no hits were identified with IP-10 production, but many clusters of peptides scored positive with IL-6 production. This result demonstrates an example showing that measurement of different sets of cytokines can identify different subsets of T cells in EPIMAX.

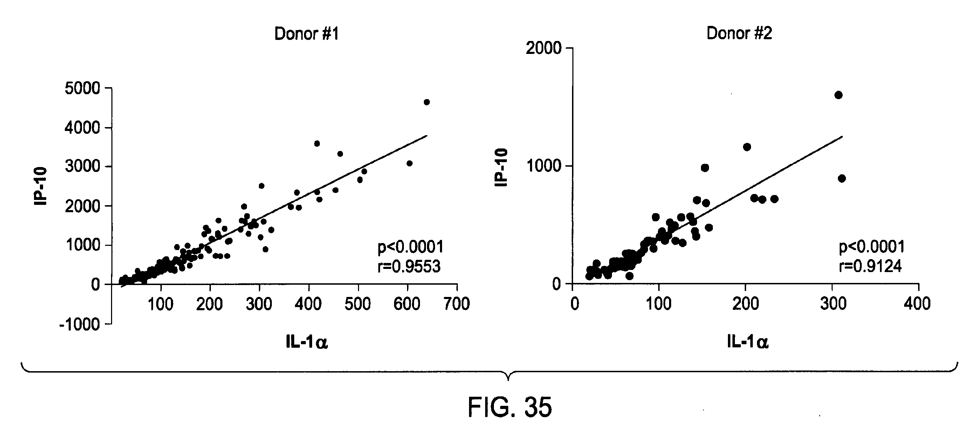

Figure 35 are graphs that depict the immune response of normal healthy volunteers as evaluated according the present invention. The levels of IL-la and IP-10 in the studies shown in Figure 33 were shown. Each dot represents cytokine levels in each separate well in PBMC culture with the peptide clusters. In all the donors, the levels of IL-1a and IP-10 correlate very well in EPIMAX, confirming that these two markers completely correlate in Type 1 responses.

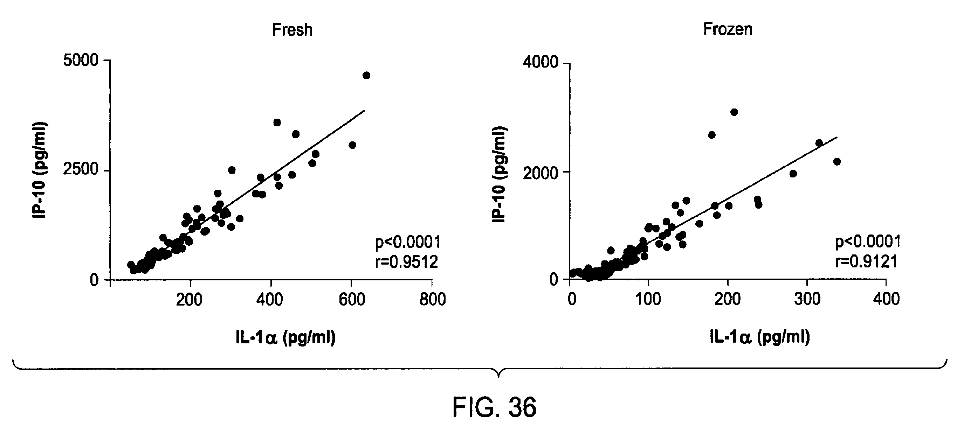

Figure 36 are graphs that depict the immune response of normal healthy volunteers as evaluated according the present invention. The frozen PBMCs obtained from the donor#1 were thawed and stimulated with either clusters of 15-mer peptides or single peptides encoding Flu-MP. The levels of IL-1a and IP-10 are shown. Each dot represents cytokine levels in each separate well in PBMC culture with the peptide clusters or single peptides. Even with the frozen PBMCs, the levels of IL-1a and IP-10 correlate very well (P<0.0001, r=0.9121), indicating that these two markers can be used to identify Type 1 responses in EPIMAX no matter how the PBMCs are obtained freshly or frozen.

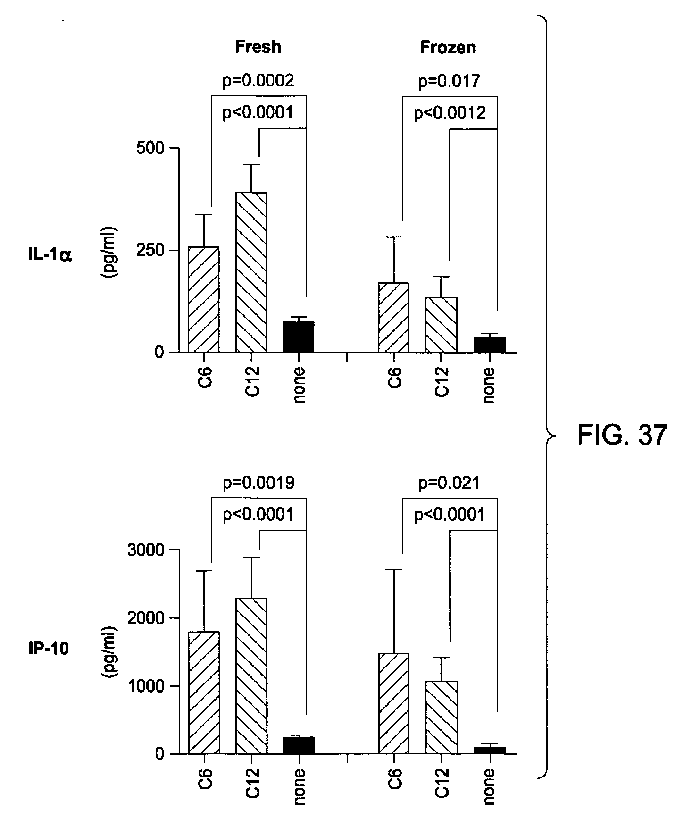

Figure 37 are graphs that depict the immune response of normal healthy volunteers as evaluated according the present invention. The fresh or frozen PBMCs obtained from the donor#1 were stimulated with clusters of 15-mer peptides encoding Flu-MP. The levels of IL-1a and IP-10 are shown. As shown in Figure 37, both clusters#6 and#12 induced the upregulation of IL-1a and IP-10 in fresh and frozen PBMCs. This study demonstrates that frozen PBMCs can be used to monitor antigen-specific T cell responses with EPIMAX.

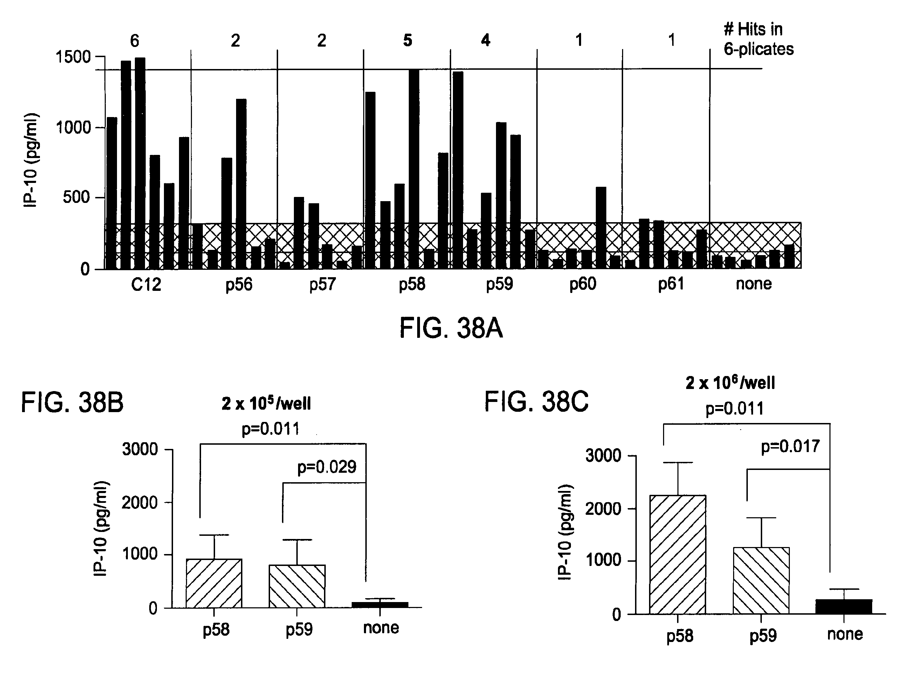

Figure 38A, 38B are graphs that depict the immune response of normal healthy volunteers as evaluated according the present invention. The frozen PBMCs obtained from donor#1 were thawed and stimulated with a cluster of peptides or individual peptides encoding Flu-MP in 6-plicates (2 x 10⁵ cells/well, 10 µM per peptide). The levels of IP-10 at 48 hours of culture are shown (Figure 37A). In another study, the higher number of PBMCs (2 x 10⁶ cells/well) were stimulated with the same concentration of 15-mer peptide in triplicates. As shown in Figure 37B, while peptide#58 and #59 turned on the IP-10 production in both studies, the contrast between samples and control culture is greater where more PBMCs/well were used in EPIMAX. Thus, higher number of PBMCs per well helps to reduce the variability and sensitivity in EPIMAX assay. Considering the low frequency of antigen specific T cells in PBMCs (usually 1 out of 10⁵ PBMCs), it is plausible that T cell responses may not be detected with EPIMAX (or virtually any kind of assays) when few cells responded to the peptide stimulation. Thus, in regular EPIMAX assay, we are using 5 x 10⁵ PBMCs/well.

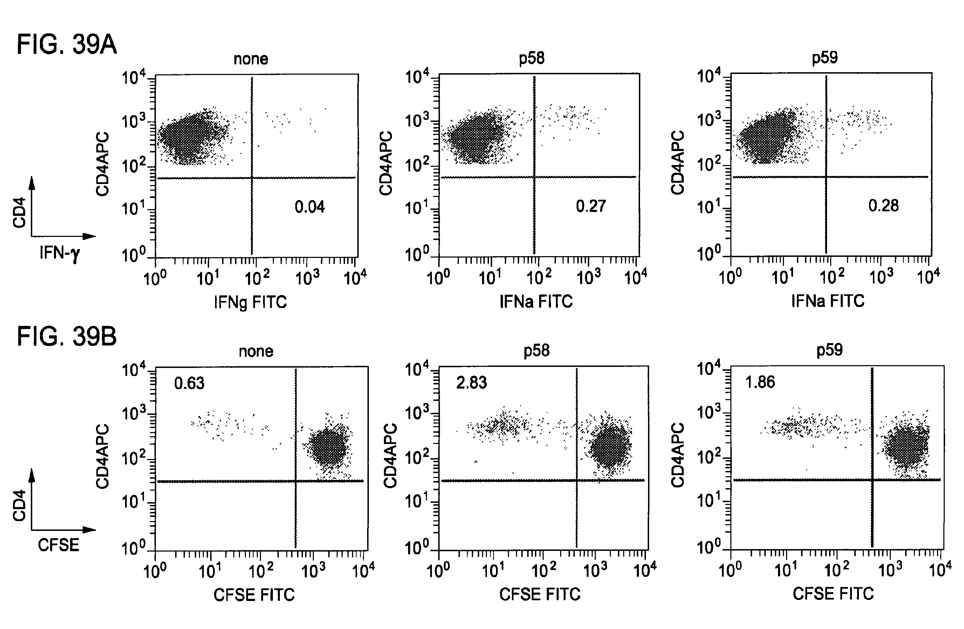

Figure 39A and 39B are graphs that depict the immune response of normal healthy volunteers against the peptides identified in the EPIMAX assay. PBMCs were resuspended in CM at 1 x 10⁶ cells/ml, and 1 ml of cell suspensions were put in culture tubes, and stimulated with indicated single peptides from Flu-MP library for 7 days. Cultured PBMCs were harvested and restimulated with DCs pulsed with indicated peptide for 5 h in the presence of monensin. Cytoplasmic cytokines were examined with specific mAbs after cell permeabilization. The percentages of cytokine producing cells in CD3⁺CD4⁺ T cell population were shown (Figure 39A). Stimulation with dominant peptides induced proliferation of specific CD4⁺ T cells. The donor's PBMCs were first stained with 1 mM of CFSE. PBMCs were resuspended in CM at 1 x 10⁶ cells/ml, and 1 ml of cell suspensions were put in culture tubes, and stimulated with indicated single peptides from Flu-MP library for 6 days. Stimulated cells were stained with anti-CD3 and CD4 mAb, and proliferating CD4⁺ T cells were analyzed with a flowcytometer based on CFSE dilution. The percentages of CFSE- cells in CD3⁺CD4⁺ T cell population were shown (Figure 39B). These results demonstrate the proof that EPIMAX allowed identification of epitope for functional Flu-MP specific CD4⁺ T cells.

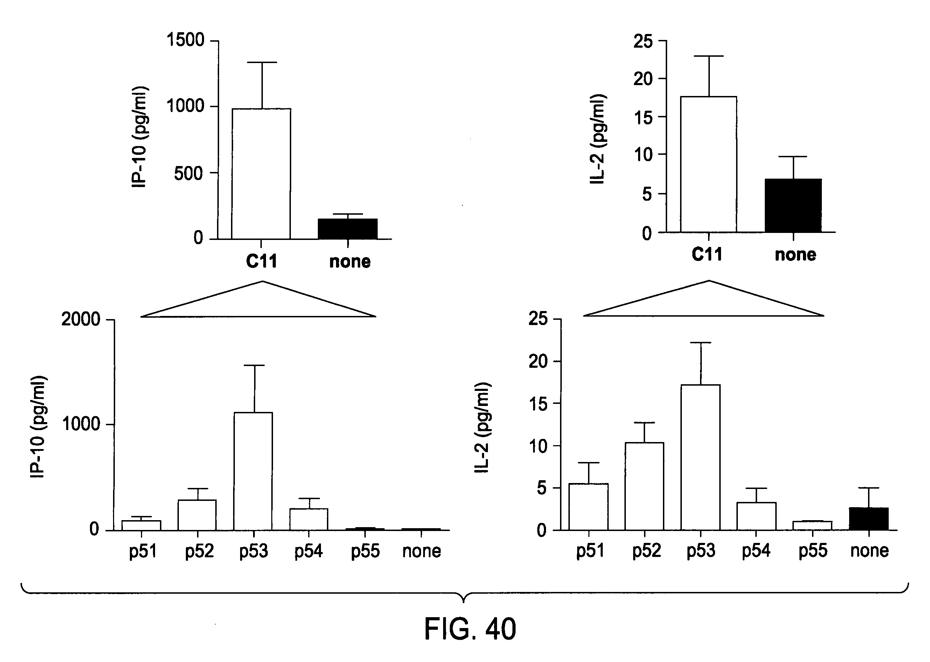

Figure 40 are graphs that depict the immune response of a normal healthy volunteer as evaluated according the present invention. The PBMCs were obtained from donor #2 in Figure 33. The frozen PBMCs were thawed and stimulated with either Flu-MP cluster#11 or single 15-mer peptides within cluster#11 (5 x 10⁵ cells/well, 20 µM per peptide). The levels of cytokines at 48 h of culture are shown. These results indicate that peptide#52 and #53 are epitopes for Flu-MP specific T cells, showing another example of identification of novel epitopes for specific CD4⁺ T cells.

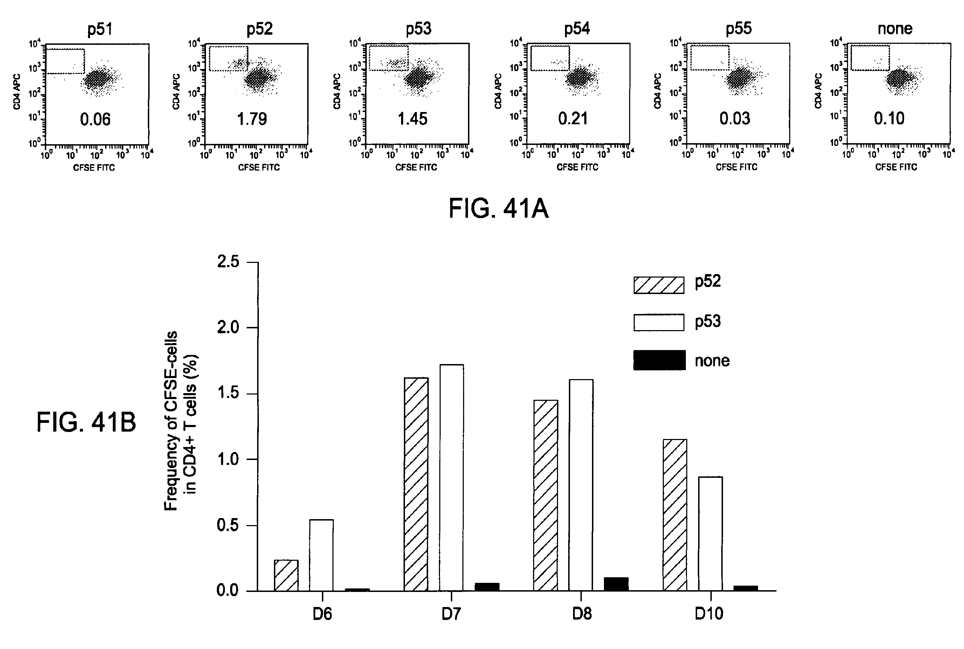

Figure 41A, 41B are graphs that depict the immune response of normal healthy volunteers against the peptides identified in the EPIMAX assay. The PBMCs used in the study in Figure 40 were labeled with CFSE, and stimulated with single peptides within Flu-Mpcluster #11. The CFSE-diluted CD4⁺ T cells were analyzed on day 8 of culture (Figure 41A). In another study, the CFSE-diluted CD4⁺ T cell populations were analyzed on day 6, 7, 8 and 10 of culture (Figure 41B). These studies demonstrate that the identified peptide (Flu-MP peptide#52 and #53) with EPIMAX induced proliferation of peptide-specific CD4⁺ T cells, and the population of proliferating CD4⁺ T cell reached at a peak on day 7 or 8 of peptide stimulation. This study shows another example of identification of novel epitopes for specific CD4⁺ T cells, and justifies performing an analysis of proliferating CD4⁺ T cell population on day 8 of culture.

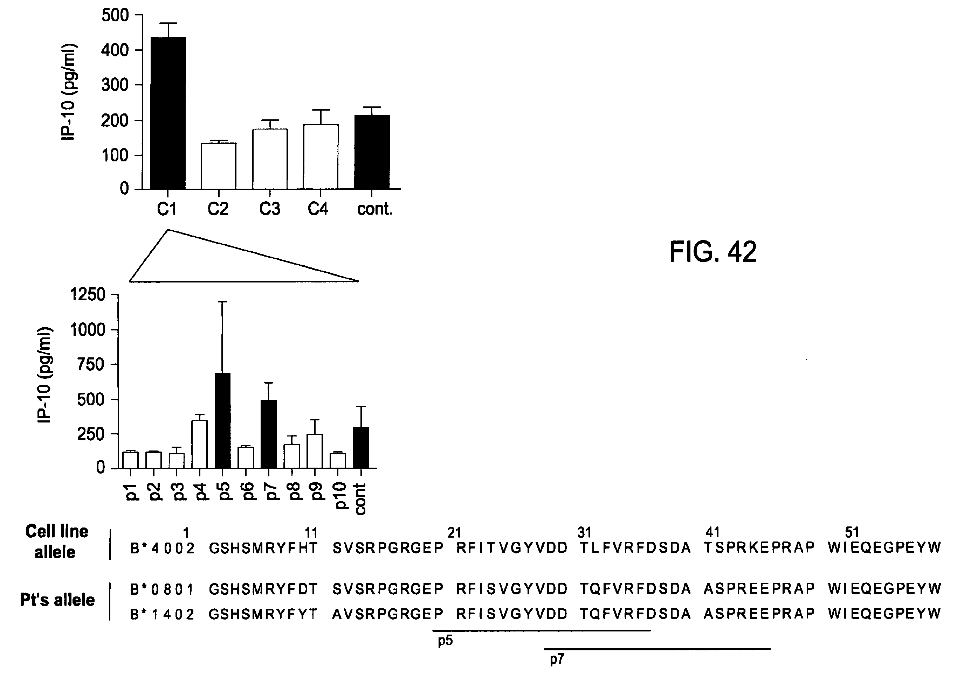

Figure 42 are graphs that depict the immune response of a melanoma patient as evaluated according the present invention. PBMCs were obtained from a melanoma patient who underwent DC vaccinations. The patient received autologous DC vaccine loaded with killed allogeneic melanoma cell lines. It is possible that DC vaccine induced T cells in this patient specific to allogeneic antigens expressed on allogeneic melanoma cell lines used for DC vaccines. To examine this possibility, the patients PBMCs were stimulated with 15-mer overlapping peptides encoding HLA-B*4001, which is expressed on the melanoma cell line, but not on the patient cells. As shown in Figure 42, peptide#5 and peptide#7 within cluster#1 induced IP-10 production in PBMC culture. As expected, these two epitopes can be antigenic epitope for the patient, as both the peptides contain amino acid mismatches. These results demonstrate that EPIMAX can identify allogeneic antigen-specific T cell responses. Thus this methodology can be useful to identify allogeneic-antigen specific T cells in a setting of organ transplantation.

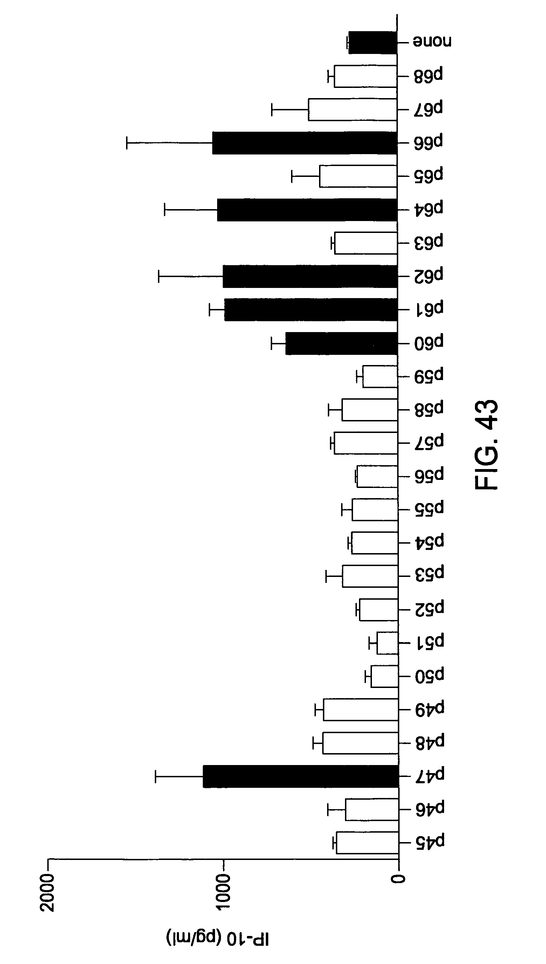

Figure 43 is a graph that depicts the immune response of a type-1 diabetic patient as evaluated according to the present invention. PBMCs were obtained from a patient with Type 1 diabetes taking immunosuppressive drugs, such as Cellcept and Predonisolone. The fresh PBMCs (5 x 10⁵ cells/well) were stimulated with single 15-mer peptides encoding IA-2, one of the auto-antigens specifically expressed on pancreatic β-islet cells. The levels of IP-10 at 48 h of culture are shown. 15-mer peptide#47, 60, 61, 62, 64, and 66 induced upregulation of IP-10 production in PBMC culture.

This study demonstrates that this patient has wide repertoire of IA-2 specific Type-1 cells. Furthermore, 5 out of 6 (peptide#47, 60, 61, 62, and 66, sequences are shown in Table 4) are novel epitopes for T cells. Thus, this study indicates that EPIMAX allows us to identify epitopes and type of T cell response in an autoimmune disease model even under medication of immunosuppressive drugs.

| | |
|---|---|
| peptide#47 | IA-2₇₈₉₋₈₀₃ |
| peptide#60 | IA-2₈₄₁₋₈₅₅ |
| peptide#61 | IA-2₈₄₅₋₈₅₉ |
| peptide#62 | IA-2₈₄₉₋₈₆₃ |
| peptide#64 | IA-2₈₅₇₋₈₇₁ |
| peptide#66 | IA-2₈₆₅₋₈₇₉ |

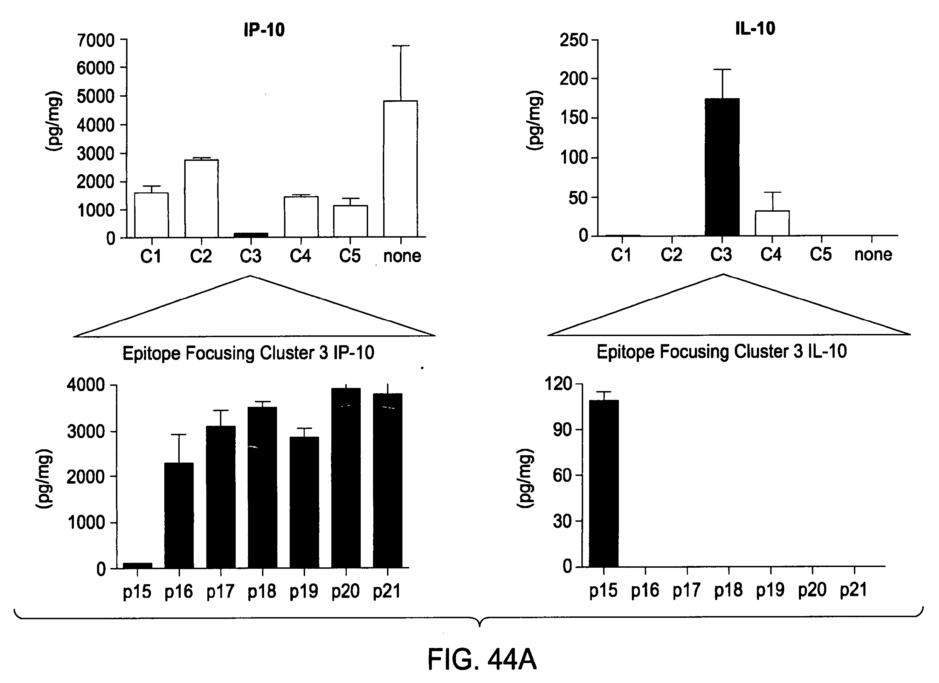

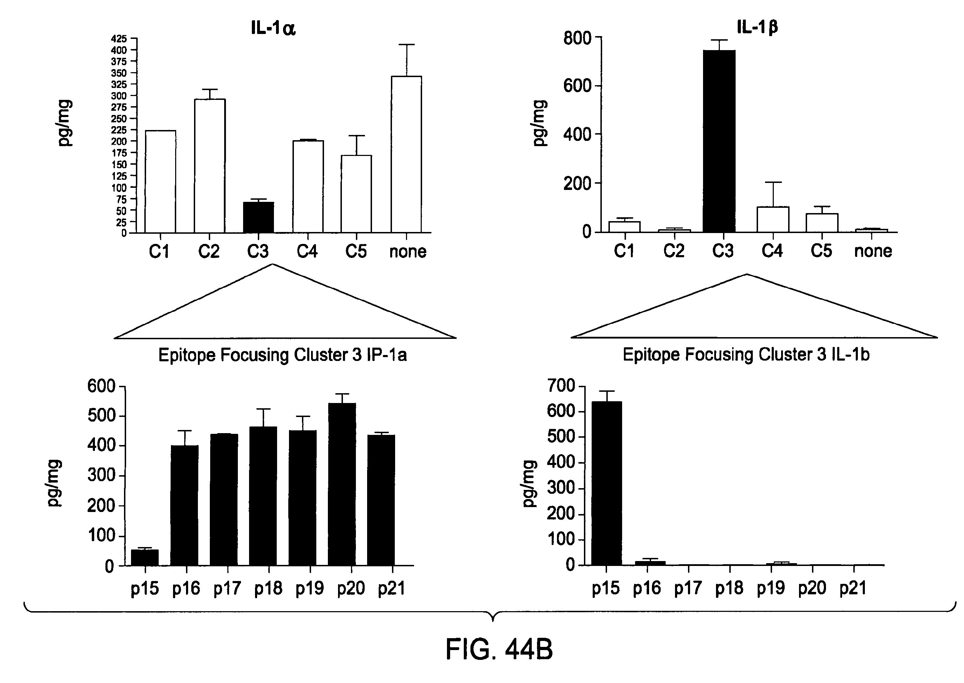

Figure 44A and 44B are graphs that depict the immune response of a melanoma patient as evaluated according the present invention, which allows the identification of various types of immune responses. PBMCs obtained from a pre-vaccine melanoma patient were incubated with 5 peptide clusters (7-8 peptides/cluster) of the survivin peptide library. Survivin cluster#3 induced down-regulation of IP-10 and upregulation of IL-10. PBMCs were then incubated with single peptide from cluster #3 to identify the responsible peptide, peptide#15. The upregulation of IL-10 was correlated with IL-1b. Data are shown in Figure 44A and 44C for IP-10, Figure 44B and 44D for IL-10, Figure 44E and 44G for IL-1α, and Figure 44F and 44H for IL-1β.

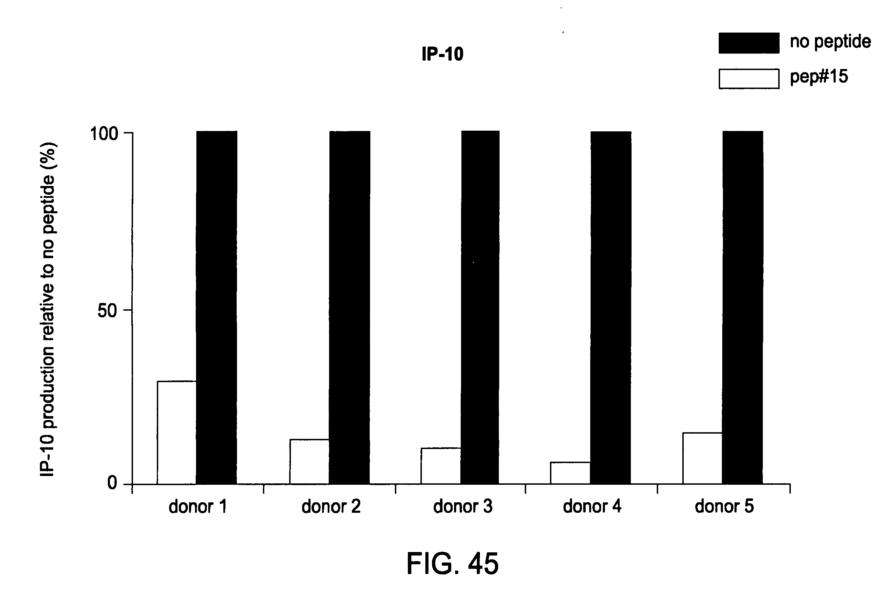

Figure 45 is a graph that depicts the immune response of normal healthy volunteers as evaluated according the present invention, which allows the identification of various types of immune responses by non-T cells. PBMCs were isolated from 5 healthy volunteers' fresh blood, incubated with survivin Peptide#15 for 48 hours and evaluated according to the methods of the present invention. IP-10 produced in the culture supernatant was measured, and the data in Figure 45 are given in relative values (%) to cultures where no peptide was added. Survivin Peptide #15 was shown to inhibit IP-10 production in all five volunteers in a non-MHC restricted fashion.

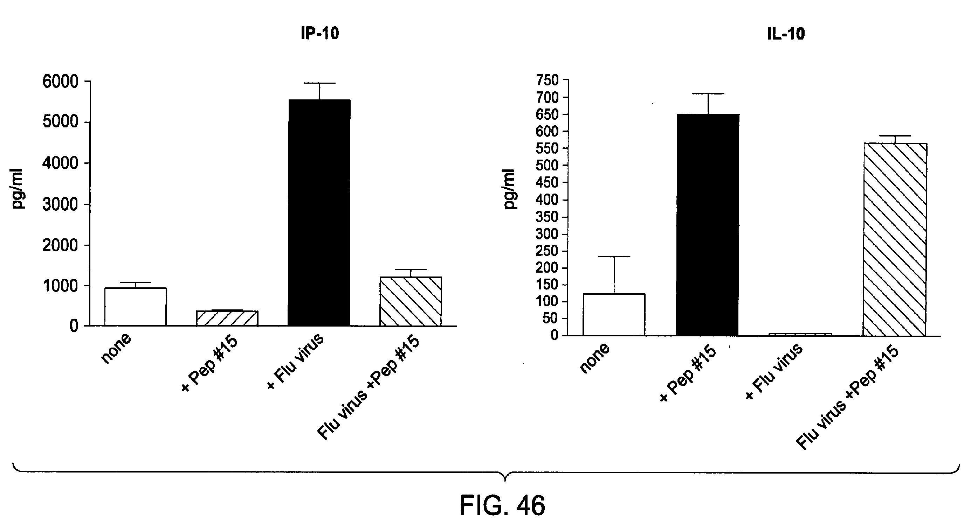

Figure 46A and 46B are graphs that depict the immune response in a melanoma patient as evaluated according to the present invention, which allows the identification of various types of immune responses by non-T cells. PBMCs from a melanoma patient were cultured for 18 hours in the presence or absence of survivin Peptide #15 and/or live flu virus (Charles River Laboratories, CT). IP-10 (Figure 46A) and IL-10 (Figure 46B) concentrations were measured with Luminex. Survivin Peptide #15 was shown to inhibit IP-10 production from PBMCs stimulated with live flu virus.

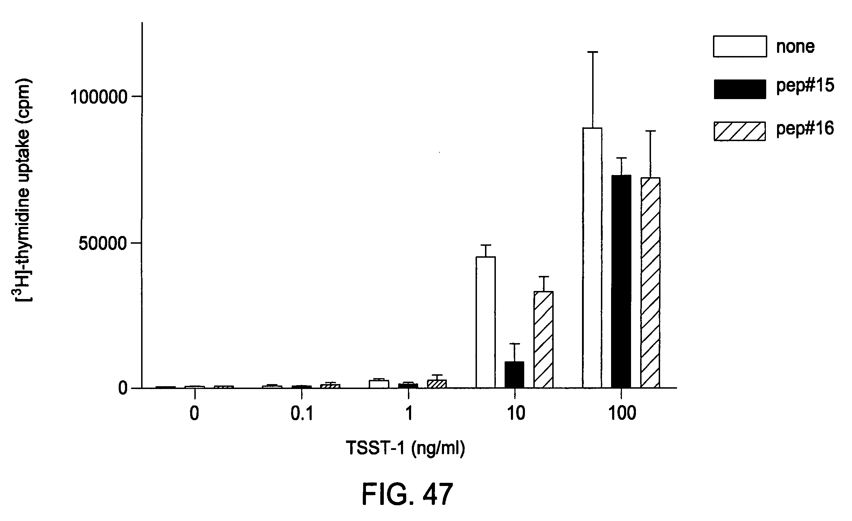

Figure 47 is a graph that depicts the immune response in a melanoma patient as evaluated according to the present invention, which allows the identification of various types of immune responses by non-T cells. PBMCs from a melanoma patient were stimulated for 5 days with TSST-1 at titrated concentrations in the presence of survivin Peptide#15, or Peptide#16 (11 amino acids are identical to Peptide#15). After 5 days, tritiated thymidine (1 µCi/well) was added. The plates were harvested 16 hours later, and incorporated radioactivity was measured by a Wallac scintillation counter. Survivin Peptide #15 was shown to inhibit T cell proliferation stimulated with Test-1.

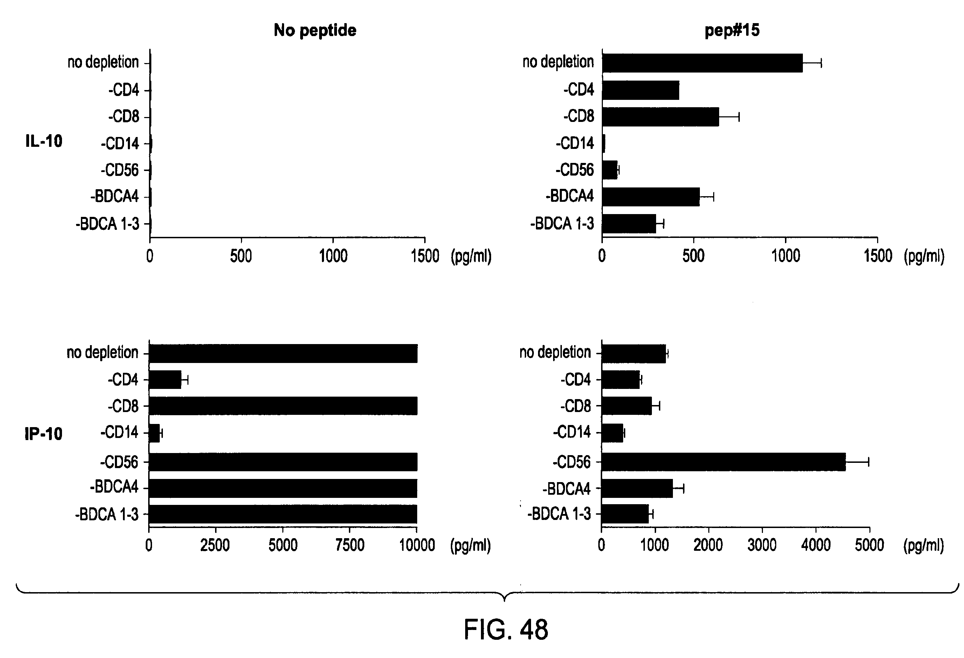

Figure 48A-48D are graphs that depict the immune response in a melanoma patient as evaluated according to the present invention, which allows the identification of various types of immune responses by non-T cells. CD4⁺, CD8⁺, CD14⁺, CD56⁺, BDCA-4⁺, or BDCA-1 or 3⁺ cells were depleted from a melanoma patient's PBMCs by using microbeads conjugated with each mAb (Miltenyi). As a negative control, PBMCs were passed through a column without any beads (no depletion). Depleted cells were resuspended in CM at 1 M/ml, and stimulated with live flu virus in the presence or absence of survivin Peptide # 15. Supernatants were harvested at 48 hours, and IL-10 and IP-10 levels were analyzed with Luminex. The data show that CD14⁺ or CD56⁺ cells are necessary for Peptide #15 to induce IL-10 secretion.

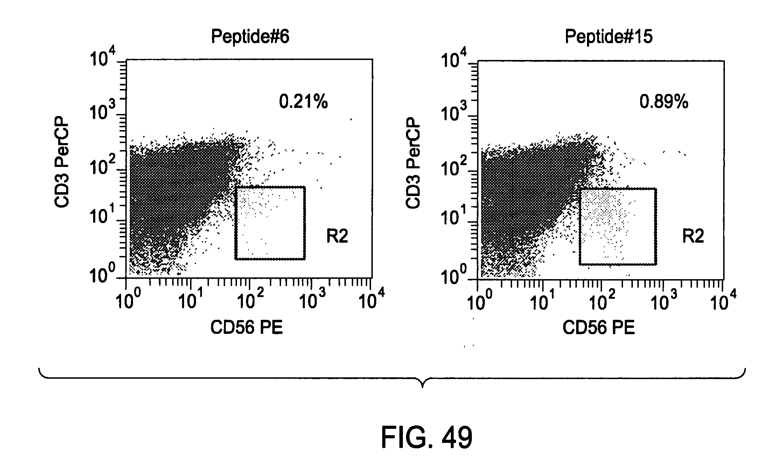

Figure 49A and 49B are graphs that depict the immune response in a melanoma patient as evaluated according to the present invention, which allows the identification of various types of immune responses by non-T cells. PBMCs from melanoma patients were stained with 5 µM CFSE, and cultured for 4 days with 10 µM of survivin Peptide#15 (Figure 49B) or control Peptide#6 (Figure 49A). The NK population was analyzed with FACS. Those CD56⁺ cells did not decrease the intensity of CFSE, showing that they are not proliferating (not shown). Thus, Peptide# 15 was shown to maintain the survival of CD3-CD56⁺ NK cells.

It will be understood that particular embodiments described herein are shown by way of illustration and not as limitations of the invention.

## Claims

1. A method for predicting an immune response in a subject to an immune therapy with an agent comprising an antigenic protein of interest comprising the steps of:
culturing isolated immune cells from the subject with a cluster of several overlapping peptides from an overlapping peptide library of the antigenic protein of interest;
analyzing simultaneously a supernatant of the subject's culture medium for multiple parameters of immune reactivity comprising the specificity and cytokine response of cytokines and chemokines, which allow identification of Type-1, Type-2 and T-Regulatory cell phenotypes to the cluster of several overlapping peptides;
determining the type of immune response based on the coordinated cytokine expression in response to the individual peptides in the culture medium as an indicator of treatment outcome.

2. The method of claim 1, wherein the therapy is immunotherapy such as vaccination, passive immunotherapy and adoptive cell transfer.

## Patentansprüche

1. Verfahren zum Vorhersagen einer Immunreaktion in einem Individuum auf eine Immuntherapie mit einem Mittel, welches ein antigenes Protein von Interesse enthält, welches folgende Schritte aufweist:
Kultivieren isolierter Immunzellen von dem Individuum mit einem Cluster aus mehreren überlappenden Peptiden des antigenen Proteins von Interesse aus einer Bibliothek überlappender Peptide;
simultanes Analysieren eines Überstandes des Kulturmediums des Individuums hinsichtlich mehrerer Parameter einer Immunreaktivität, welche die Spezifität und die Zytokinreaktion von Zytokinen und Chemokinen, welche die Identifizierung von Zellphänotypen Zellen vom Typ 1, Typ 2 und T-regulatorisch ermöglichen, gegenüber dem Cluster mehrerer überlappender Peptide umfassen;
Bestimmen des Typs der Immunreaktion auf der Grundlage der koordinierten Zytokinexpression als Reaktion auf die einzelnen Peptide in dem Kulturmedium als Indikator eines Behandlungsergebnisses.

2. Verfahren nach Anspruch 1, wobei es sich bei der Therapie um eine Immuntherapie handelt, beispielsweise um eine Impfung, eine passive Immuntherapie und adoptiven Zelltransfer.

## Revendications

1. Procédé de prédiction d'une réponse immunitaire chez un sujet à une thérapie immunitaire avec un agent comprenant une protéine antigénique d'intérêt, comprenant les étapes suivantes :
cultiver des cellules immunitaires isolées provenant du sujet avec un ensemble de plusieurs peptides chevauchants provenant d'une banque de peptides chevauchants de la protéine antigénique d'intérêt ;
analyser simultanément le surnageant du milieu de culture du sujet pour déterminer plusieurs paramètres de réactivité immunitaire comprenant la spécificité et la réponse en cytokines des cytokines et des chimiokines, qui permettent l'identification des phénotypes des lymphocytes de type 1, de type 2 et T régulateurs, pour l'ensemble de plusieurs peptides chevauchants ;
déterminer le type de réponse immunitaire sur la base de l'expression coordonnée des cytokines en réponse aux peptides individuels dans le milieu de culture en tant qu'indicateur du résultat du traitement.

2. Procédé selon la revendication 1, dans lequel la thérapie est une immunothérapie telle qu'une vaccination, une immunothérapie passive et un transfert adoptif de cellules.
